# EUROPEAN PATENT SPECIFICATION

(11) **EP 2 470 554 B1**
(45) Date of publication and mention of the grant of the patent: **28.06.2017**
(21) Application number: 10815729.8
(22) Date of filing: 24.08.2010
(51) Int. Cl.: A61K 39/295, C12N 15/62

(54) **COMPOSITIONS THAT INDUCE T CELL HELP**
ZUSAMMENSETZUNGEN ZUR INDUZIERUNG VON T-ZELLEN-HILFE
COMPOSITIONS QUI INDUISENT L'ASSISTANCE AUX LYMPHOCYTES T

(30) Priority: 06.01.2010 US 335611 P; 26.08.2009 US 237147 P
(43) Date of publication of application: 04.07.2012
(60) Divisional application: 17171181.5
(73) Proprietor: Selecta Biosciences, Inc., Watertown, MA 02472 (US)
(72) Inventor: FRASER, Christopher, Arlington MA 02476 (US); LIPFORD, Grayson, B., Watertown MA 02472 (US); LAMOTHE, Robert, Cambridge MA 02139 (US); ALTREUTER, David, H., Wayland MA 01778 (US)
(74) Representative: Sharples, Andrew John
(86) International application number: PCT/US2010/002330
(87) International publication number: WO 2011/031298

(56) References cited:
- WO-A1-95/25120
- WO-A2-98/05684
- WO-A2-99/55730
- US-A1- 2004 230 380
- US-A1- 2005 137 156
- US-A1- 2006 051 317
- US-A1- 2006 093 617
- US-A1- 2008 064 859
- FALUGI F ET AL: "Rationally designed strings of promiscuous CD4(+) T cell epitopes provide help to Haemophilus influenzae type b oligosaccharide: a model for new conjugate vaccines", EUROPEAN JOURNAL OF IMMUNOLOGY, WILEY - V C H VERLAG GMBH & CO. KGAA, DE, vol. 31, no. 12, 1 December 2001 (2001-12-01), pages 3816-3824, XP002401049, ISSN: 0014-2980, DOI: 10.1002/1521-4141(200112)31:12<3816::AID-I MMU3816>3.0.CO;2-K
- HEWITT E W ET AL: "NATURAL PROCESSING SITES FOR HUMAN CATHEPSIN E AND CATHEPSIN D IN TETANUS TOXIN//IMPLICATIONS FOR T CELL EPITOPE GENERATION", THE JOURNAL OF IMMUNOLOGY, THE AMERICAN ASSOCIATION OF IMMUNOLOGISTS, US, vol. 195, 15 November 1997 (1997-11-15), pages 4693-4699, XP001068771, ISSN: 0022-1767
- VERMA N K ET AL: "Induction of a cellular immune response to a defined T-cell epitope as an insert in the flagellin of a live vaccine strain of Salmonella", VACCINE, ELSEVIER LTD, GB, vol. 13, no. 3, 1 January 1995 (1995-01-01), pages 235-244, XP004057661, ISSN: 0264-410X, DOI: 10.1016/0264-410X(95)93308-V
- N. LUTZNER ET AL: "Quantifying Cathepsin S Activity in Antigen Presenting Cells Using a Novel Specific Substrate", JOURNAL OF BIOLOGICAL CHEMISTRY, vol. 283, no. 52, 26 December 2008 (2008-12-26), pages 36185-36194, XP055110717, ISSN: 0021-9258, DOI: 10.1074/jbc.M806500200

### SEQUENCE LISTING

<110> Selecta Biosciences, Inc. et al.
<120> COMPOSITIONS THAT INDUCE T CELL HELP
<130> S1681.70005WO00
<140> not yet assigned
   <141> 2010-08-24
<150> US 61/237147
   <151> 2009-08-26
<150> US 61/335611
   <151> 2010-01-06
<160> 129
<170> PatentIn version 3.5
<210> 1
   <211> 21
   <212> PRT
   <213> artificial sequence
<220>
   <223> synthetic polypeptide
<400> 1
   Asn Asn Phe Thr Val Ser Phe Trp Leu Arg Val Pro Lys Val Ser Ala
   1               5                   10                  15
   Ser His Leu Glu Thr
               20
<210> 2
   <211> 9
   <212> PRT
   <213> artificial sequence
<220>
   <223> synthetic polypeptide
<400> 2
   Thr Leu Leu Tyr Val Leu Phe Glu Val
   1               5
<210> 3
   <211> 15
   <212> PRT
   <213> artificial sequence
<220>
   <223> synthetic polypeptide
<400> 3
   Ile Leu Met Gln Tyr Ile Lys Ala Asn Ser Lys Phe Ile Gly Ile
   1               5                   10                  15
<210> 4
   <211> 20
   <212> PRT
   <213> artificial sequence
<220>
   <223> synthetic polypeptide
<400> 4
   Gln Ser Ile Ala Leu Ser Ser Leu Met Val Ala Gln Ala Ile Pro Leu
   1               5                   10                  15
   Val Gly Glu Leu
               20
<210> 5
   <211> 30
   <212> PRT
   <213> artificial sequence
<220>
   <223> synthetic polypeptide
<400> 5
   Thr Leu Leu Tyr Val Leu Phe Glu Val Asn Asn Phe Thr Val Ser Phe
   1               5                   10                  15
   Trp Leu Arg Val Pro Lys Val Ser Ala Ser His Leu Glu Thr
               20                  25                  30
<210> 6
   <211> 24
   <212> PRT
   <213> artificial sequence
<220>
   <223> synthetic polypeptide
<400> 6
   Thr Leu Leu Tyr Val Leu Phe Glu Val Ile Leu Met Gln Tyr Ile Lys
   1               5                   10                  15
   Ala Asn Ser Lys Phe Ile Gly Ile
               20
<210> 7
   <211> 35
   <212> PRT
   <213> artificial sequence
<220>
   <223> synthetic polypeptide
<400> 7
   Ile Leu Met Gln Tyr Ile Lys Ala Asn Ser Lys Phe Ile Gly Ile Gln
   1               5                   10                  15
   Ser Ile Ala Leu Ser Ser Leu Met Val Ala Gln Ala Ile Pro Leu Val
               20                  25                  30
   Gly Glu Leu
           35
<210> 8
   <211> 35
   <212> PRT
   <213> artificial sequence
<220>
   <223> synthetic polypeptide
<400> 8
   Gln Ser Ile Ala Leu Ser Ser Leu Met Val Ala Gln Ala Ile Pro Leu
   1               5                   10                  15
   Val Gly Glu Leu Ile Leu Met Gln Tyr Ile Lys Ala Asn Ser Lys Phe
               20                  25                  30
   Ile Gly Ile
           35
<210> 9
   <211> 27
   <212> PRT
   <213> artificial sequence
<220>
   <223> synthetic polypeptide
<400> 9
   Ile Leu Met Gln Tyr Ile Lys Ala Asn Ser Lys Phe Ile Gly Ile Gln
   1               5                   10                  15
   Ser Ile Ala Leu Ser Ser Leu Met Val Ala Gln
               20                  25
<210> 10
   <211> 29
   <212> PRT
   <213> artificial sequence
<220>
   <223> synthetic polypeptide
<400> 10
   Gln Ser Ile Ala Leu Ser Ser Leu Met Val Ala Gln Ala Ile Ile Leu
   1               5                   10                  15
   Met Gln Tyr Ile Lys Ala Asn Ser Lys Phe Ile Gly Ile
               20                  25
<210> 11
   <211> 29
   <212> PRT
   <213> artificial sequence
<220>
   <223> synthetic polypeptide
<400> 11
   Thr Leu Leu Tyr Val Leu Phe Glu Val Pro Met Gly Leu Pro Ile Leu
   1               5                   10                  15
   Met Gln Tyr Ile Lys Ala Asn Ser Lys Phe Ile Gly Ile
               20                  25
<210> 12
   <211> 29
   <212> PRT
   <213> artificial sequence
<220>
   <223> synthetic polypeptide
<400> 12
   Thr Leu Leu Tyr Val Leu Phe Glu Val Lys Val Ser Val Arg Ile Leu
   1               5                   10                  15
   Met Gln Tyr Ile Lys Ala Asn Ser Lys Phe Ile Gly Ile
               20                  25
<210> 13
   <211> 32
   <212> PRT
   <213> artificial sequence
<220>
   <223> synthetic polypeptide
<400> 13
   Ile Leu Met Gln Tyr Ile Lys Ala Asn Ser Lys Phe Ile Gly Ile Pro
   1               5                   10                  15
   Met Gly Leu Pro Gln Ser Ile Ala Leu Ser Ser Leu Met Val Ala Gln
               20                  25                  30
<210> 14
   <211> 32
   <212> PRT
   <213> artificial sequence
<220>
   <223> synthetic polypeptide
<400> 14
   Ile Leu Met Gln Tyr Ile Lys Ala Asn Ser Lys Phe Ile Gly Ile Lys
   1               5                   10                  15
   Val Ser Val Arg Gln Ser Ile Ala Leu Ser Ser Leu Met Val Ala Gln
               20                  25                  30
<210> 15
   <211> 21
   <212> PRT
   <213> artificial sequence
<220>
   <223> synthetic polypeptide
<400> 15
   Thr Leu Leu Tyr Val Leu Phe Glu Val Gln Ser Ile Ala Leu Ser Ser
   1               5                   10                  15
   Leu Met Val Ala Gln
               20
<210> 16
   <211> 26
   <212> PRT
   <213> artificial sequence
<220>
   <223> synthetic polypeptide
<400> 16
   Thr Leu Leu Tyr Val Leu Phe Glu Val Pro Met Gly Leu Pro Gln Ser
   1               5                   10                  15
   Ile Ala Leu Ser Ser Leu Met Val Ala Gln
               20                  25
<210> 17
   <211> 26
   <212> PRT
   <213> artificial sequence
<220>
   <223> synthetic polypeptide
<400> 17
   Thr Leu Leu Tyr Val Leu Phe Glu Val Lys Val Ser Val Arg Gln Ser
   1               5                   10                  15
   Ile Ala Leu Ser Ser Leu Met Val Ala Gln
               20                  25
<210> 18
   <211> 35
   <212> PRT
   <213> artificial sequence
<220>
   <223> synthetic polypeptide
<400> 18
   Thr Leu Leu Tyr Val Leu Phe Glu Val Pro Met Gly Leu Pro Asn Asn
   1               5                   10                  15
   Phe Thr Val Ser Phe Trp Leu Arg Val Pro Lys Val Ser Ala Ser His
               20                  25                  30
   Leu Glu Thr
           35
<210> 19
   <211> 35
   <212> PRT
   <213> artificial sequence
<220>
   <223> synthetic polypeptide
<400> 19
   Thr Leu Leu Tyr Val Leu Phe Glu Val Lys Val Ser Val Arg Asn Asn
   1               5                   10                  15
   Phe Thr Val Ser Phe Trp Leu Arg Val Pro Lys Val Ser Ala Ser His
               20                  25                  30
   Leu Glu Thr
           35
<210> 20
   <211> 36
   <212> PRT
   <213> artificial sequence
<220>
   <223> synthetic polypeptide
<400> 20
   Ile Leu Met Gln Tyr Ile Lys Ala Asn Ser Lys Phe Ile Gly Ile Gln
   1               5                   10                  15
   Ser Ile Ala Leu Ser Ser Leu Met Val Ala Gln Thr Leu Leu Tyr Val
               20                  25                  30
   Leu Phe Glu Val
           35
<210> 21
   <211> 36
   <212> PRT
   <213> artificial sequence
<220>
   <223> synthetic polypeptide
<400> 21
   Thr Leu Leu Tyr Val Leu Phe Glu Val Ile Leu Met Gln Tyr Ile Lys
   1               5                   10                  15
   Ala Asn Ser Lys Phe Ile Gly Ile Gln Ser Ile Ala Leu Ser Ser Leu
               20                  25                  30
   Met Val Ala Gln
           35
<210> 22
   <211> 17
   <212> PRT
   <213> artificial sequence
<220>
   <223> synthetic polypeptide
<400> 22
   Gln Ser Ile Ala Leu Ser Ser Leu Met Val Ala Gln Ala Ile Pro Leu
   1               5                   10                  15
   Val
<210> 23
   <211> 20
   <212> PRT
   <213> artificial sequence
<220>
   <223> synthetic polypeptide
<400> 23
   Ile Asp Lys Ile Ser Asp Val Ser Thr Ile Val Pro Tyr Ile Gly Pro
   1               5                   10                  15
   Ala Leu Asn Ile
               20
<210> 24
   <211> 37
   <212> PRT
   <213> artificial sequence
<220>
   <223> synthetic polypeptide
<400> 24
   Gln Ser Ile Ala Leu Ser Ser Leu Met Val Ala Gln Ala Ile Pro Leu
   1               5                   10                  15
   Val Ile Asp Lys Ile Ser Asp Val Ser Thr Ile Val Pro Tyr Ile Gly
               20                  25                  30
   Pro Ala Leu Asn Ile
           35
<210> 25
   <211> 37
   <212> PRT
   <213> artificial sequence
<220>
   <223> synthetic polypeptide
<400> 25
   Ile Asp Lys Ile Ser Asp Val Ser Thr Ile Val Pro Tyr Ile Gly Pro
   1               5                   10                  15
   Ala Leu Asn Ile Gln Ser Ile Ala Leu Ser Ser Leu Met Val Ala Gln
                   20                  25                  30
   Ala Ile Pro Leu Val
           35
<210> 26
   <211> 42
   <212> PRT
   <213> artificial sequence
<220>
   <223> synthetic polypeptide
<400> 26
   Gln Ser Ile Ala Leu Ser Ser Leu Met Val Ala Gln Ala Ile Pro Leu
   1               5                   10                  15
   Val Pro Met Gly Leu Pro Ile Asp Lys Ile Ser Asp Val Ser Thr Ile
               20                  25                  30
   Val Pro Tyr Ile Gly Pro Ala Leu Asn Ile
           35                  40
<210> 27
   <211> 42
   <212> PRT
   <213> artificial sequence
<220>
   <223> synthetic polypeptide
<400> 27
   Ile Asp Lys Ile Ser Asp Val Ser Thr Ile Val Pro Tyr Ile Gly Pro
   1               5                   10                  15
   Ala Leu Asn Ile Pro Met Gly Leu Pro Gln Ser Ile Ala Leu Ser Ser
               20                  25                  30
   Leu Met Val Ala Gln Ala Ile Pro Leu Val
           35                  40
<210> 28
   <211> 11
   <212> PRT
   <213> artificial sequence
<220>
   <223> synthetic polypeptide
<400> 28
   Tyr Val Lys Gln Asn Thr Leu Lys Leu Ala Thr
   1               5                   10
<210> 29
   <211> 19
   <212> PRT
   <213> artificial sequence
<220>
   <223> synthetic polypeptide
<400> 29
   Cys Tyr Pro Tyr Asp Val Pro Asp Tyr Ala Ser Leu Arg Ser Leu Val
   1               5                   10                  15
   Ala Ser Ser
<210> 30
   <211> 14
   <212> PRT
   <213> artificial sequence
<220>
   <223> synthetic polypeptide
<400> 30
   Asn Ala Glu Leu Leu Val Ala Leu Glu Asn Gln His Thr Ile
   1               5                   10
<210> 31
   <211> 16
   <212> PRT
   <213> artificial sequence
<220>
   <223> synthetic polypeptide
<400> 31
   Thr Ser Leu Tyr Val Arg Ala Ser Gly Arg Val Thr Val Ser Thr Lys
   1               5                   10                  15
<210> 32
   <211> 20
   <212> PRT
   <213> artificial sequence
<220>
   <223> synthetic polypeptide
<400> 32
   Glu Lys Ile Val Leu Leu Phe Ala Ile Val Ser Leu Val Lys Ser Asp
     1               5                   10                  15
   Gln Ile Cys Ile
               20
<210> 33
   <211> 20
   <212> PRT
   <213> artificial sequence
<220>
   <223> synthetic polypeptide
<400> 33
   Gln Ile Leu Ser Ile Tyr Ser Thr Val Ala Ser Ser Leu Ala Leu Ala
   1               5                   10                  15
   Ile Met Val Ala
               20
<210> 34
   <211> 24
   <212> PRT
   <213> artificial sequence
<220>
   <223> synthetic polypeptide
<400> 34
   Met Val Thr Gly Ile Val Ser Leu Met Leu Gln Ile Gly Asn Met Ile
   1 5 10 15
   Ser Ile Trp Val Ser His Ser Ile
   20
<210> 35
   <211> 17
   <212> PRT
   <213> artificial sequence
<220>
   <223> synthetic polypeptide
<400> 35
   Glu Asp Leu Ile Phe Leu Ala Arg Ser Ala Leu Ile Leu Arg Gly Ser
   1 5 10 15
   Val
<210> 36
   <211> 19
   <212> PRT
   <213> artificial sequence
<220>
   <223> synthetic polypeptide
<400> 36
   Cys Ser Gln Arg Ser Lys Phe Leu Leu Met Asp Ala Leu Lys Leu Ser
   1 5 10 15
   Ile Glu Asp
<210> 37
   <211> 17
   <212> PRT
   <213> artificial sequence
<220>
   <223> synthetic polypeptide
<400> 37
   Ile Arg Gly Phe Val Tyr Phe Val Glu Thr Leu Ala Arg Ser Ile Cys
   1 5 10 15
   Glu
<210> 38
   <211> 21
   <212> PRT
   <213> artificial sequence
<220>
   <223> synthetic polypeptide
<400> 38
   Thr Phe Glu Phe Thr Ser Phe Phe Tyr Arg Tyr Gly Phe Val Ala Asn
   1               5                   10                  15
   Phe Ser Met Glu Leu
               20
<210> 39
   <211> 31
   <212> PRT
   <213> artificial sequence
<220>
   <223> synthetic polypeptide
<400> 39
   Leu Ile Phe Leu Ala Arg Ser Ala Leu Ile Leu Arg Lys Val Ser Val
   1               5                   10                  15
   Arg Asn Ala Glu Leu Leu Val Ala Leu Glu Asn Gln His Thr Ile
               20                  25                  30
<210> 40
   <211> 31
   <212> PRT
   <213> artificial sequence
<220>
   <223> synthetic polypeptide
<400> 40
   Asn Ala Glu Leu Leu Val Ala Leu Glu Asn Gln His Thr Ile Lys Val
   1               5                   10                  15
   Ser Val Arg Leu Ile Phe Leu Ala Arg Ser Ala Leu Ile Leu Arg
               20                  25                  30
<210> 41
   <211> 33
   <212> PRT
   <213> artificial sequence
<220>
   <223> synthetic polypeptide
<400> 41
   Ile Leu Ser Ile Tyr Ser Thr Val Ala Ser Ser Leu Ala Leu Ala Ile
   1               5                   10                  15
   Lys Val Ser Val Arg Leu Ile Phe Leu Ala Arg Ser Ala Leu Ile Leu
               20                  25                  30
   Arg
<210> 42
   <211> 33
   <212> PRT
   <213> artificial sequence
<220>
   <223> synthetic polypeptide
<400> 42
   Leu Ile Phe Leu Ala Arg Ser Ala Leu Ile Leu Arg Lys Val Ser Val
   1               5                   10                  15
   Arg Ile Leu Ser Ile Tyr Ser Thr Val Ala Ser Ser Leu Ala Leu Ala
               20                  25                  30
   Ile
<210> 43
   <211> 32
   <212> PRT
   <213> artificial sequence
<220>
   <223> synthetic polypeptide
<400> 43
   Leu Ile Phe Leu Ala Arg Ser Ala Leu Ile Leu Arg Lys Val Ser Val
   1               5                   10                  15
   Arg Cys Ser Gln Arg Ser Lys Phe Leu Leu Met Asp Ala Leu Lys Leu
               20                  25                  30
<210> 44
   <211> 32
   <212> PRT
   <213> artificial sequence
<220>
   <223> synthetic polypeptide
<400> 44
   Cys Ser Gln Arg Ser Lys Phe Leu Leu Met Asp Ala Leu Lys Leu Lys
   1 5 10 15
   Val Ser Val Arg Leu Ile Phe Leu Ala Arg Ser Ala Leu Ile Leu Arg
   20 25 30
<210> 45
   <211> 38
   <212> PRT
   <213> artificial sequence
<220>
   <223> synthetic polypeptide
<400> 45
   Thr Phe Glu Phe Thr Ser Phe Phe Tyr Arg Tyr Gly Phe Val Ala Asn
   1 5 10 15
   Phe Ser Met Glu Leu Ile Arg Gly Phe Val Tyr Phe Val Glu Thr Leu
   20 25 30
   Ala Arg Ser Ile Cys Glu
   35
<210> 46
   <211> 38
   <212> PRT
   <213> artificial sequence
<220>
   <223> synthetic polypeptide
<400> 46
   Ile Arg Gly Phe Val Tyr Phe Val Glu Thr Leu Ala Arg Ser Ile Cys
   1 5 10 15
   Glu Thr Phe Glu Phe Thr Ser Phe Phe Tyr Arg Tyr Gly Phe Val Ala
   20 25 30
   Asn Phe Ser Met Glu Leu
   35
<210> 47
   <211> 60
   <212> DNA
   <213> artificial sequence
<220>
   <223> synthetic oligonucleotide
<400> 47
   aataatttta ccgttagctt ttggttgagg gttcctaaag tatctgctag tcatttagaa 60
<210> 48
   <211> 63
   <212> DNA
   <213> artificial sequence
<220>
   <223> synthetic oligonucleotide
<400> 48
   aacaacttca ccgtgagctt ctggctgaga gtgcccaagg tgagcgccag ccacctggag 60
   acc 63
<210> 49
   <211> 26
   <212> DNA
   <213> artificial sequence
<220>
   <223> synthetic oligonucleotide
<400> 49
   acgcttctct atgttctgtt cgaagt 26
<210> 50
   <211> 27
   <212> DNA
   <213> artificial sequence
<220>
   <223> synthetic oligonucleotide
<400> 50
   accctgctgt acgtgctgtt cgaggtg 27
<210> 51
   <211> 45
   <212> DNA
   <213> artificial sequence
<220>
   <223> synthetic oligonucleotide
<400> 51
   attttaatgc agtatataaa agcaaattct aaatttatag gtata 45
<210> 52
   <211> 45
   <212> DNA
   <213> artificial sequence
<220>
   <223> synthetic oligonucleotide
<400> 52
   atcctgatgc agtacatcaa ggccaacagc aagttcatcg gcatc 45
<210> 53
   <211> 60
   <212> DNA
   <213> artificial sequence
<220>
   <223> synthetic oligonucleotide
<400> 53
   caatcgatag ctttatcgtc tttaatggtt gctcaagcta taccattggt aggagagcta 60
<210> 54
   <211> 60
   <212> DNA
   <213> artificial sequence
<220>
   <223> synthetic oligonucleotide
<400> 54
   cagagcatcg ccctgagcag cctgatggtg gcccaggcca tccccctggt gggcgagctg 60
<210> 55
   <211> 90
   <212> DNA
   <213> artificial sequence
<220>
   <223> synthetic oligonucleotide
<400> 55
   accctgctgt acgtgctgtt cgaggtgaac aacttcaccg tgagcttctg gctgagagtg 60
   cccaaggtga gcgccagcca cctggagacc 90
<210> 56
   <211> 72
   <212> DNA
   <213> artificial sequence
<220>
   <223> synthetic oligonucleotide
<400> 56
   accctgctgt acgtgctgtt cgaggtgatc ctgatgcagt acatcaaggc caacagcaag 60
   ttcatcggca tc 72
<210> 57
   <211> 105
   <212> DNA
   <213> artificial sequence
<220>
   <223> synthetic oligonucleotide
<400> 57
   atcctgatgc agtacatcaa ggccaacagc aagttcatcg gcatccagag catcgccctg        60
   agcagcctga tggtggccca ggccatcccc ctggtgggcg agctg                       105
<210> 58
   <211> 105
   <212> DNA
   <213> artificial sequence
<220>
   <223> synthetic oligonucleotide
<400> 58
   cagagcatcg ccctgagcag cctgatggtg gcccaggcca tccccctggt gggcgagctg      60
   atcctgatgc agtacatcaa ggccaacagc aagttcatcg gcatc                       105
<210> 59
   <211> 81
   <212> DNA
   <213> artificial sequence
<220>
   <223> synthetic oligonucleotide
<400> 59
   atcctgatgc agtacatcaa ggccaacagc aagttcatcg gcatccagag catcgccctg        60
   agcagcctga tggtggccca g                                                  81
<210> 60
   <211> 87
   <212> DNA
   <213> artificial sequence
<220>
   <223> synthetic oligonucleotide
<400> 60
   cagagcatcg ccctgagcag cctgatggtg gcccaggcca tcatcctgat gcagtacatc        60
   aaggccaaca gcaagttcat cggcatc                                            87
<210> 61
   <211> 87
   <212> DNA
   <213> artificial sequence
<220>
   <223> synthetic oligonucleotide
<400> 61
   accctgctgt atgtgctgtt tgaagtgccg atgggcctgc cgattctgat gcagtatatt 60
   aaagcgaaca gcaaatttat tggcatt 87
<210> 62
   <211> 87
   <212> DNA
   <213> artificial sequence
<220>
   <223> synthetic oligonucleotide
<400> 62
   accctgctgt acgtgctgtt cgaggtgccc atgggcctgc ccatcctgat gcagtacatc 60
   aaggccaaca gcaagttcat cggcatc 87
<210> 63
   <211> 87
   <212> DNA
   <213> artificial sequence
<220>
   <223> synthetic oligonucleotide
<400> 63
   accctgctgt atgtgctgtt tgaagtgaaa gtgagcgtgc gcattctgat gcagtatatt 60
   aaagcgaaca gcaaatttat tggcatt 87
<210> 64
   <211> 87
   <212> DNA
   <213> artificial sequence
<220>
   <223> synthetic oligonucleotide
<400> 64
   accctgctgt acgtgctgtt cgaggtgaag gtgagcgtga gaatcctgat gcagtacatc 60
   aaggccaaca gcaagttcat cggcatc 87
<210> 65
   <211> 96
   <212> DNA
   <213> artificial sequence
<220>
   <223> synthetic oligonucleotide
<400> 65
   attctgatgc agtatattaa agcgaacagc aaatttattg gcattccgat gggcctgccg 60
   cagagcattg cgctgagcag cctgatggtg gcgcag 96
<210> 66
   <211> 96
   <212> DNA
   <213> artificial sequence
<220>
   <223> synthetic oligonucleotide
<400> 66
   atcctgatgc agtacatcaa ggccaacagc aagttcatcg gcatccccat gggcctgccc 60
   cagagcatcg ccctgagcag cctgatggtg gcccag 96
<210> 67
   <211> 96
   <212> DNA
   <213> artificial sequence
<220>
   <223> synthetic oligonucleotide
<400> 67
   attctgatgc agtatattaa agcgaacagc aaatttattg gcattaaagt gagcgtgcgc 60
   cagagcattg cgctgagcag cctgatggtg gcgcag 96
<210> 68
   <211> 96
   <212> DNA
   <213> artificial sequence
<220>
   <223> synthetic oligonucleotide
<400> 68
   atcctgatgc agtacatcaa ggccaacagc aagttcatcg gcatcaaggt gagcgtgaga 60
   cagagcatcg ccctgagcag cctgatggtg gcccag 96
<210> 69
   <211> 15
   <212> DNA
   <213> artificial sequence
<220>
   <223> synthetic oligonucleotide
<400> 69
   ccgatgggcc tacca 15
<210> 70
   <211> 17
   <212> DNA
   <213> artificial sequence
<220>
   <223> synthetic oligonucleotide
<400> 70
   aaggtctcag tgagaac 17
<210> 71
   <211> 30
   <212> PRT
   <213> artificial sequence
<220>
   <223> synthetic polypeptide
<400> 71
   Glu Glu Ser Thr Leu Leu Tyr Val Leu Phe Glu Val Lys Val Ser Val
   1               5                   10                  15
   Arg Gln Ser Ile Ala Leu Ser Ser Leu Met Val Ala Gln Lys
               20                  25                  30
<210> 72
   <211> 30
   <212> PRT
   <213> artificial sequence
<220>
   <223> synthetic polypeptide
<400> 72
   Glu Ser Thr Leu Leu Tyr Val Leu Phe Glu Val Lys Val Ser Val Arg
   1               5                   10                  15
   Gln Ser Ile Ala Leu Ser Ser Leu Met Val Ala Gln Lys Glu
               20                  25                  30
<210> 73
   <211> 30
   <212> PRT
   <213> artificial sequence
<220>
   <223> synthetic polypeptide
<400> 73
   Lys Glu Ser Thr Leu Leu Tyr Val Leu Phe Glu Val Lys Val Ser Val
   1 5 10 15
   Arg Gln Ser Ile Ala Leu Ser Ser Leu Met Val Ala Gln Glu
   20 25 30
<210> 74
   <211> 11
   <212> PRT
   <213> artificial sequence
<220>
   <223> synthetic polypeptide
<400> 74
   Tyr Val Lys Gln Asn Thr Leu Lys Leu Ala Thr
   1 5 10
<210> 75
   <211> 19
   <212> PRT
   <213> artificial sequence
<220>
   <223> synthetic polypeptide
<400> 75
   Cys Tyr Pro Tyr Asp Val Pro Asp Tyr Ala Ser Leu Arg Ser Leu Val
   1 5 10 15
   Ala Ser Ser
<210> 76
   <211> 14
   <212> PRT
   <213> artificial sequence
<220>
   <223> synthetic polypeptide
<400> 76
   Asn Ala Glu Leu Leu Val Ala Leu Glu Asn Gln His Thr Ile
   1 5 10
<210> 77
   <211> 16
   <212> PRT
   <213> artificial sequence
<220>
   <223> synthetic polypeptide
<400> 77
   Thr Ser Leu Tyr Val Arg Ala Ser Gly Arg Val Thr Val Ser Thr Lys
   1               5                   10                  15
<210> 78
   <211> 20
   <212> PRT
   <213> artificial sequence
<220>
   <223> synthetic polypeptide
<400> 78
   Glu Lys Ile Val Leu Leu Phe Ala Ile Val Ser Leu Val Lys Ser Asp
   1               5                   10                  15
   Gln Ile Cys Ile
               20
<210> 79
   <211> 20
   <212> PRT
   <213> artificial sequence
<220>
   <223> synthetic polypeptide
<400> 79
   Gln Ile Leu Ser Ile Tyr Ser Thr Val Ala Ser Ser Leu Ala Leu Ala
   1               5                   10                  15
   Ile Met Val Ala
               20
<210> 80
   <211> 24
   <212> PRT
   <213> artificial sequence
<220>
   <223> synthetic polypeptide
<400> 80
   Met Val Thr Gly Ile Val Ser Leu Met Leu Gln Ile Gly Asn Met Ile
   1               5                   10                  15
   Ser Ile Trp Val Ser His Ser Ile
               20
<210> 81
   <211> 17
   <212> PRT
   <213> artificial sequence
<220>
   <223> synthetic polypeptide
<400> 81
   Glu Asp Leu Ile Phe Leu Ala Arg Ser Ala Leu Ile Leu Arg Gly Ser
   1               5                   10                  15
   Val
<210> 82
   <211> 19
   <212> PRT
   <213> artificial sequence
<220>
   <223> synthetic polypeptide
<400> 82 Ile Glu Asp
<210> 83
   <211> 17
   <212> PRT
   <213> artificial sequence
<220>
   <223> synthetic polypeptide
<400> 83
   Ile Arg Gly Phe Val Tyr Phe Val Glu Thr Leu Ala Arg Ser Ile Cys
   1               5                   10                  15
   Glu
<210> 84
   <211> 21
   <212> PRT
   <213> artificial sequence
<220>
   <223> synthetic polypeptide
<400> 84
   Thr Phe Glu Phe Thr Ser Phe Phe Tyr Arg Tyr Gly Phe Val Ala Asn
   1               5                   10                  15
   Phe Ser Met Glu Leu
               20
<210> 85
   <211> 15
   <212> PRT
   <213> artificial sequence
<220>
   <223> synthetic polypeptide
<400> 85
   Met Met Met Gly Met Phe Asn Met Leu Ser Thr Val Leu Gly Val
   1               5                   10                  15
<210> 86
   <211> 31
   <212> PRT
   <213> artificial sequence
<220>
   <223> synthetic polypeptide
<400> 86
   Leu Ile Phe Leu Ala Arg Ser Ala Leu Ile Leu Arg Lys Val Ser Val
   1               5                   10                  15
   Arg Asn Ala Glu Leu Leu Val Ala Leu Glu Asn Gln His Thr Ile
               20                  25                  30
<210> 87
   <211> 31
   <212> PRT
   <213> artificial sequence
<220>
   <223> synthetic polypeptide
<400> 87
   Asn Ala Glu Leu Leu Val Ala Leu Glu Asn Gln His Thr Ile Lys Val
   1               5                   10                  15
   Ser Val Arg Leu Ile Phe Leu Ala Arg Ser Ala Leu Ile Leu Arg
               20                  25                  30
<210> 88
   <211> 33
   <212> PRT
   <213> artificial sequence
<220>
   <223> synthetic polypeptide
<400> 88
   Ile Leu Ser Ile Tyr Ser Thr Val Ala Ser Ser Leu Ala Leu Ala Ile
   1 5 10 15
   Lys Val Ser Val Arg Leu Ile Phe Leu Ala Arg Ser Ala Leu Ile Leu
   20 25 30
   Arg
<210> 89
   <211> 33
   <212> PRT
   <213> artificial sequence
<220>
   <223> synthetic polypeptide
<400> 89
   Leu Ile Phe Leu Ala Arg Ser Ala Leu Ile Leu Arg Lys Val Ser Val
   1 5 10 15
   Arg Ile Leu Ser Ile Tyr Ser Thr Val Ala Ser Ser Leu Ala Leu Ala
   20 25 30
   Ile
<210> 90
   <211> 32
   <212> PRT
   <213> artificial sequence
<220>
   <223> synthetic polypeptide
<400> 90
   Leu Ile Phe Leu Ala Arg Ser Ala Leu Ile Leu Arg Lys Val Ser Val
   1 5 10 15
   Arg Cys Ser Gln Arg Ser Lys Phe Leu Leu Met Asp Ala Leu Lys Leu
   20 25 30
<210> 91
   <211> 32
   <212> PRT
   <213> artificial sequence
<220>
   <223> synthetic polypeptide
<400> 91
   Cys Ser Gln Arg Ser Lys Phe Leu Leu Met Asp Ala Leu Lys Leu Lys
   1 5 10 15
   Val Ser Val Arg Leu Ile Phe Leu Ala Arg Ser Ala Leu Ile Leu Arg
   20 25 30
<210> 92
   <211> 20
   <212> PRT
   <213> artificial sequence
<220>
   <223> synthetic polypeptide
<400> 92
   Glu Lys Ile Val Leu Leu Phe Ala Ile Val Ser Leu Val Lys Ser Asp
   1 5 10 15
   Gln Ile Cys Ile
   20
<210> 93
   <211> 36
   <212> PRT
   <213> artificial sequence
<220>
   <223> synthetic polypeptide
<400> 93
   Met Met Met Gly Met Phe Asn Met Leu Ser Thr Val Leu Gly Val Thr
   1 5 10 15
   Phe Glu Phe Thr Ser Phe Phe Tyr Arg Tyr Gly Phe Val Ala Asn Phe
   20 25 30
   Ser Met Glu Leu
   35
<210> 94
   <211> 36
   <212> PRT
   <213> artificial sequence
<220>
   <223> synthetic polypeptide
<400> 94
   Thr Phe Glu Phe Thr Ser Phe Phe Tyr Arg Tyr Gly Phe Val Ala Asn
   1               5                   10                  15
   Phe Ser Met Glu Leu Met Met Met Gly Met Phe Asn Met Leu Ser Thr
               20                  25                  30
   Val Leu Gly Val
           35
<210> 95
   <211> 38
   <212> PRT
   <213> artificial sequence
<220>
   <223> synthetic polypeptide
<400> 95
   Thr Phe Glu Phe Thr Ser Phe Phe Tyr Arg Tyr Gly Phe Val Ala Asn
   1               5                   10                  15
   Phe Ser Met Glu Leu Ile Arg Gly Phe Val Tyr Phe Val Glu Thr Leu
               20                  25                  30
   Ala Arg Ser Ile Cys Glu
           35
<210> 96
   <211> 38
   <212> PRT
   <213> artificial sequence
<220>
   <223> synthetic polypeptide
<400> 96
   Ile Arg Gly Phe Val Tyr Phe Val Glu Thr Leu Ala Arg Ser Ile Cys
   1               5                   10                  15
   Glu Thr Phe Glu Phe Thr Ser Phe Phe Tyr Arg Tyr Gly Phe Val Ala
               20                  25                  30
   Asn Phe Ser Met Glu Leu
           35
<210> 97
   <211> 37
   <212> PRT
   <213> artificial sequence
<220>
   <223> synthetic polypeptide
<400> 97
   Met Met Met Gly Met Phe Asn Met Leu Ser Thr Val Leu Gly Val Lys
   1               5                   10                  15
   Val Ser Val Arg Ile Arg Gly Phe Val Tyr Phe Val Glu Thr Leu Ala
               20                  25                  30
   Arg Ser Ile Cys Glu
           35
<210> 98
   <211> 37
   <212> PRT
   <213> artificial sequence
<220>
   <223> synthetic polypeptide
<400> 98
   Ile Arg Gly Phe Val Tyr Phe Val Glu Thr Leu Ala Arg Ser Ile Cys
   1               5                   10                  15
   Glu Lys Val Ser Val Arg Met Met Met Gly Met Phe Asn Met Leu Ser
               20                  25                  30
   Thr Val Leu Gly Val
           35
<210> 99
   <211> 5
   <212> PRT
   <213> artificial sequence
<220>
   <223> synthetic polypeptide
<400> 99
   Pro Met Gly Leu Pro
   1               5
<210> 100
   <211> 5
   <212> PRT
   <213> artificial sequence
<220>
   <223> synthetic polypeptide
<400> 100
   Lys Val Ser Val Arg
   1               5
<210> 101
   <211> 6
   <212> PRT
   <213> artificial sequence
<220>
   <223> synthetic polypeptide
<400> 101
   Met Met Met Thr Phe Glu
   1               5
<210> 102
   <211> 6
   <212> PRT
   <213> artificial sequence
<220>
   <223> synthetic polypeptide
<400> 102
   Thr Phe Glu Met Met Met
   1               5
<210> 103
   <211> 6
   <212> PRT
   <213> artificial sequence
<220>
   <223> synthetic polypeptide
<400> 103
   Thr Phe Glu Ile Arg Gly
   1               5
<210> 104
   <211> 6
   <212> PRT
   <213> artificial sequence
<220>
   <223> synthetic polypeptide
<400> 104
   Ile Arg Gly Thr Phe Glu
   1               5
<210> 105
   <211> 7
   <212> PRT
   <213> artificial sequence
<220>
   <223> synthetic polypeptide
<400> 105
   Met Met Met Lys Ile Arg Gly
   1               5
<210> 106
   <211> 7
   <212> PRT
   <213> artificial sequence
<220>
   <223> synthetic polypeptide
<400> 106
   Ile Arg Gly Lys Met Met Met
   1               5
<210> 107
   <211> 32
   <212> PRT
   <213> artificial sequence
<220>
   <223> synthetic polypeptide
<400> 107
   Thr Leu Leu Tyr Val Leu Phe Glu Val Asn Asn Phe Thr Val Ser Phe
   1               5                   10                  15
   Trp Leu Arg Val Pro Lys Val Ser Ala Ser His Leu Glu Thr Gly Cys
               20                  25                  30
<210> 108
   <211> 36
   <212> PRT
   <213> artificial sequence
<220>
   <223> synthetic polypeptide
<400> 108
   Ser Lys Asn Ile Leu Met Gln Tyr Ile Lys Ala Asn Ser Lys Phe Ile
   1               5                   10                  15
   Gly Ile Pro Met Gly Leu Pro Gln Ser Ile Ala Leu Ser Ser Leu Met
               20                  25                  30
   Val Ala Gln Lys
           35
<210> 109
   <211> 37
   <212> PRT
   <213> artificial sequence
<220>
   <223> synthetic polypeptide
<400> 109
   Ser Lys Asn Ile Leu Met Gln Tyr Ile Lys Ala Asn Ser Lys Phe Ile
   1               5                   10                  15
   Gly Ile Pro Met Gly Leu Pro Gln Ser Ile Ala Leu Ser Ser Leu Met
               20                  25                  30
   Val Ala Gln Lys Glu
           35
<210> 110
   <211> 35
   <212> PRT
   <213> artificial sequence
<220>
   <223> synthetic polypeptide
<400> 110
   Lys Asn Ile Leu Met Gln Tyr Ile Lys Ala Asn Ser Lys Phe Ile Gly
   1               5                   10                  15
   Ile Pro Met Gly Leu Pro Gln Ser Ile Ala Leu Ser Ser Leu Met Val
               20                  25                  30
   Ala Gln Lys
           35
<210> 111
   <211> 36
   <212> PRT
   <213> artificial sequence
<220>
   <223> synthetic polypeptide
<400> 111
   Lys Asn Ile Leu Met Gln Tyr Ile Lys Ala Asn Ser Lys Phe Ile Gly
   1               5                   10                  15
   Ile Pro Met Gly Leu Pro Gln Ser Ile Ala Leu Ser Ser Leu Met Val
               20                  25                  30
   Ala Gln Lys Glu
           35
<210> 112
   <211> 32
   <212> PRT
   <213> artificial sequence
<220>
   <223> synthetic polypeptide
<220>
   <221> MISC_FEATURE
   <222> (32)..(32)
   <223> PEGylation
<400> 112
   Ile Leu Met Gln Tyr Ile Lys Ala Asn Ser Lys Phe Ile Gly Ile Thr
   1               5                   10                  15
   Ser Gly Thr Ser Gln Ser Ile Ala Leu Ser Ser Leu Met Val Ala Gln
               20                  25                  30
<210> 113
   <211> 32
   <212> PRT
   <213> artificial sequence
<220>
   <223> synthetic polypeptide
<400> 113
   Ile Leu Met Gln Tyr Ile Lys Ala Asn Ser Lys Phe Ile Gly Ile Gly
   1               5                   10                  15
   Glu Gly Asp Asp Gln Ser Ile Ala Leu Ser Ser Leu Met Val Ala Gln
               20                  25                  30
<210> 114
   <211> 28
   <212> PRT
   <213> artificial sequence
<220>
   <223> synthetic polypeptide
<400> 114
   Ser Gln Tyr Ile Lys Ala Asn Ser Lys Phe Ile Gly Ile Thr Glu Leu
   1               5                   10                  15
   Gln Ser Ile Ala Leu Ser Ser Leu Met Val Ala Gln
               20                  25
<210> 115
   <211> 29
   <212> PRT
   <213> artificial sequence
<220>
   <223> synthetic polypeptide
<400> 115
   Ile Leu Met Gln Tyr Ile Lys Ala Asn Ser Lys Phe Ile Gly Ile Lys
   1               5                   10                  15
   Lys Gln Ser Ile Ala Leu Ser Ser Leu Met Val Ala Gln
               20                  25
<210> 116
   <211> 33
   <212> PRT
   <213> artificial sequence
<220>
   <223> synthetic polypeptide
<400> 116
   Lys Asn Ile Leu Met Gln Tyr Ile Lys Ala Asn Ser Lys Phe Ile Gly
   1               5                   10                  15
   Ile Lys Lys Gln Ser Ile Ala Leu Ser Ser Leu Met Val Ala Gln Lys
               20                  25                  30
   Glu
<210> 117
   <211> 29
   <212> PRT
   <213> artificial sequence
<220>
   <223> synthetic polypeptide
<220>
   <221> MISC_FEATURE
   <222> (16) .. (17)
   <223> disulfide linker
<400> 117
   Ile Leu Met Gln Tyr Ile Lys Ala Asn Ser Lys Phe Ile Gly Ile Cys
   1               5                   10                  15
   Cys Gln Ser Ile Ala Leu Ser Ser Leu Met Val Ala Gln
               20                  25
<210> 118
   <211> 27
   <212> PRT
   <213> artificial sequence
<220>
   <223> synthetic polypeptide
<220>
   <221> MISC_FEATURE
   <222> (15) .. (16)
   <223> hydrazone linker (acidic sensitive cleavage)
<400> 118
   Ile Leu Met Gln Tyr Ile Lys Ala Asn Ser Lys Phe Ile Gly Ile Gln
   1               5                   10                  15
   Ser Ile Ala Leu Ser Ser Leu Met Val Ala Gln
               20                  25
<210> 119
   <211> 6
   <212> PRT
   <213> artificial sequence
<220>
   <223> synthetic polypeptide
<400> 119
   Ser Lys Val Ser Val Arg
   1               5
<210> 120
   <211> 9
   <212> PRT
   <213> artificial sequence
<220>
   <223> synthetic polypeptide
<400> 120
   Thr Leu Leu Tyr Val Leu Phe Glu Leu
   1 5
<210> 121
   <211> 9
   <212> PRT
   <213> artificial sequence
<220>
   <223> synthetic polypeptide
<400> 121
   Thr Leu Leu Tyr Leu Leu Phe Glu Leu
   1 5
<210> 122
   <211> 9
   <212> PRT
   <213> artificial sequence
<220>
   <223> synthetic polypeptide
<400> 122
   Thr Leu Leu Phe Leu Leu Phe Glu Leu
   1 5
<210> 123
   <211> 15
   <212> PRT
   <213> artificial sequence
<220>
   <223> synthetic polypeptide
<400> 123
   Ile Leu Met Gln Tyr Ile Lys Ala Asn Ser Lys Phe Leu Gly Leu
   1 5 10 15
<210> 124
   <211> 15
   <212> PRT
   <213> artificial sequence
<220>
   <223> synthetic polypeptide
<400> 124
   Ile Ile Met Gln Tyr Ile Lys Ala Asn Ser Lys Phe Leu Gly Leu
   1 5 10 15
<210> 125
   <211> 15
   <212> PRT
   <213> artificial sequence
<220>
   <223> synthetic polypeptide
<400> 125
   Ile Ile Met Gln Tyr Ile Arg Ala Asn Ser Arg Phe Leu Gly Leu
   1               5                   10                  15
<210> 126
   <211> 32
   <212> PRT
   <213> artificial sequence
<220>
   <223> synthetic polypeptide
<400> 126
   Ile Leu Met Gln Tyr Ile Lys Ala Asn Ser Lys Phe Ile Gly Ile Gly
   1               5                   10                  15
   Glu Gly Asp Asp Gln Ser Ile Ala Leu Ser Ser Leu Met Val Ala Gln
               20                  25                  30
<210> 127
   <211> 30
   <212> PRT
   <213> artificial sequence
<220>
   <223> synthetic polypeptide
<400> 127
   Glu Glu His Thr Leu Leu Tyr Val Leu Phe Glu Val Lys Val Ser Val
   1               5                   10                  15
   Arg Gln Ser Ile Ala Leu Ser Ser Leu Met Val Ala Gln Lys
               20                  25                  30
<210> 128
   <211> 30
   <212> PRT
   <213> artificial sequence
<220>
   <223> synthetic polypeptide
<400> 128
   Glu His Thr Leu Leu Tyr Val Leu Phe Glu Val Lys Val Ser Val Arg
   1               5                   10                  15
   Gln Ser Ile Ala Leu Ser Ser Leu Met Val Ala Gln Lys Glu
               20                  25                  30
<210> 129
   <211> 30
   <212> PRT
   <213> artificial sequence
<220>
   <223> synthetic polypeptide
<400> 129
   Lys Glu His Thr Leu Leu Tyr Val Leu Phe Glu Val Lys Val Ser Val
   1               5                   10                  15
   Arg Gln Ser Ile Ala Leu Ser Ser Leu Met Val Ala Gln Glu
               20                  25                  30

## Description

### BACKGROUND OF THE INVENTION

The activity of certain vaccines can be enhanced by the concomitant provision of T cell help. T cell help can be induced through presentation of certain peptide antigens that can form complexes with MHC II. What is needed are compositions and methods that can induce improved T cell help for a vaccine response. Polyepitope proteins comprising MHC II binding peptides linked by Lys-Gly linkers are disclosed in WO99/55730 and in Falugi et al., Eur. J. Immunol. (2001) 31:3816-3824.

### SUMMARY OF THE INVENTION

In an aspect, the invention relates to a composition comprising synthetic nanocarriers comprising: A - x - B; and a pharmaceutically acceptable excipient; wherein x comprises a cathepsin cleavage site comprising KVSVR; wherein A comprises a first MHC II binding peptide, and the first MHC II binding peptide comprising a peptide having at least 70% identity to a natural HLA-DP binding peptide, a peptide having at least 70% identity to a natural HLA-DQ binding peptide, or a peptide having at least 70% identity to a natural HLA-DR binding peptide; wherein B comprises a second MHC II binding peptide, and the second MHC II binding peptide comprising a peptide having at least 70% identity to a natural HLA-DP binding peptide, a peptide having at least 70% identity to a natural HLA-DQ binding peptide, or a peptide having at least 70% identity to a natural HLA-DR binding peptide, and wherein A and B do not have 100% identity to one another.

In an aspect, the invention relates to composition comprising: one or more isolated nucleic acids that encode a composition comprising A - x - B as defined above, wherein when there is more than one isolated nucleic acid, the isolated nucleic acids together encode the composition comprising A - x - B. In an aspect, the invention relates to a composition comprising A-x-B, wherein x comprises a cathepsin cleavage site comprising KVSVR and wherein A-x-B comprises an amino acid sequence that has at least 75% identity to any one of the amino acid sequences set forth as SEQ ID NOs: 12, 14, 17, 19, and 39-44.

### BRIEF DESCRIPTION OF THE FIGURES

Figure 1 shows representative example of flow cytometry data showing IFN-γ expression in peptide stimulated CD4+/ CD45RAlow/ CD62Lhigh central memory T-cells.

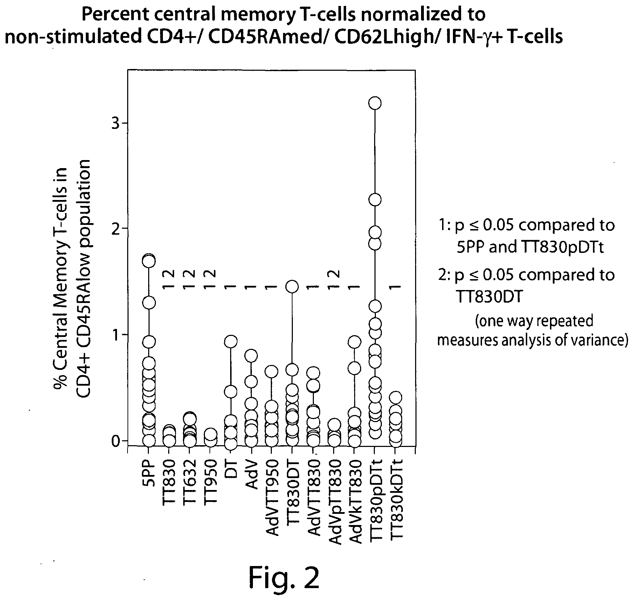

Figure 2 shows the percent central memory T-cells normalized to non-stimulated CD4+/ CD45RAmed/ CD62Lhigh/ IFN-γ+ T-cells. Class II peptide chimeras give a robust CD4 memory T-cell recall response. Peptides were added at a final concentration of 4µM. Negative and positive PBMC controls were non-stimulated or stimulated with a pool of 5 peptides (5PP), respectively. Prior to flow cytometric analysis the cells were stained with CD4-FITC, CD45RA-PE and CD62LCy7PE. The cells were then permeablized, fixed and stained with IFN-γ. Central memory T-cells are CD4+ / CD45RAmedium / CD62Lhigh / IFN-γ+. The values shown are the percent of CD62L+/IFN-γ+ cells found in a CD4+/CD62L gate. The values were normalized by subtracting the values for a non-stimulated control for each donor.
Figure 3 shows the number (out of 20) donors positive for memory T-cells responding to peptide. Donors were considered positive if values were greater than 0.08% responding central memory T-cells in the CD4+CD45RAlow population.

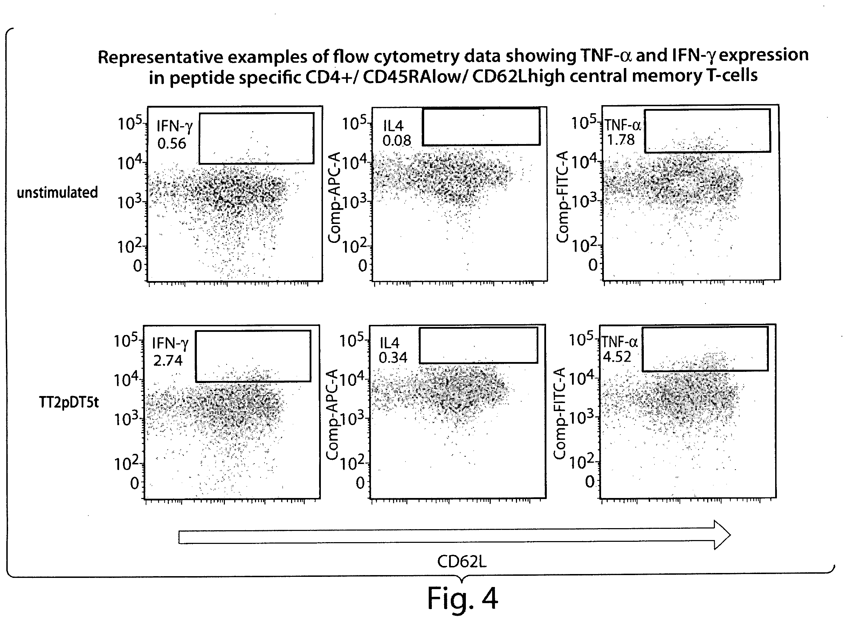

Figure 4 shows representative examples of flow cytometry data showing TNF-α and IFN-γ expression in peptide specific CD4+/ CD45RAlow/CD62Lhigh central memory T-cells. Class II Peptide chimeras give a robust Dendritic cell / CD4 central memory T-cell recall response. Monocytes were isolated from PBMCs by magnetic bead negative selection and grown in IL-4 and GM-CSF for one week to induce dendritic cell (DC) differentiation. Autologous CD4+ cells were isolated from cryopreserved PBMC and cultured together with the DCs in the presence or absence of peptide. Detection of TNF-α and IFN-γ expression in central memory T-cells was as described previously. Immature central memory T-cells express IFN-γ / TNF-α and IL-2; committed effector memory t-cells express IL-4 or IFN-γ only.
Figure 5 shows the percent IL-4, TNF-α, or IFN-γ expression in peptide specific CD4+/ CD45RAlow/ CD62Lhigh central memory T-cells. Cytokine expression in dendritic cell / autologous CD4 T-cell co-culture in the presence or absence of peptide is shown. The number of cytokine positive memory T-cells per 75000 events collected by flow cytometry (normalized to non-stimulated) are shown.
Figure 6 shows the percent TNF-α plus IFN-γ or TNF-α plus IL-4 co-expression in peptide specific CD4+/ CD45RAlow/ CD62Lhigh central memory T-cells. Cytokine co-expression in dendritic cell / autologous CD4 T-cell co-culture in the presence or absence of peptide is shown.
Figure 7 shows TT830pDTt variants (SEQ ID NOs: 13, 108-113, 126, 114-118, respectively.)
Figure 8 shows the percent CD62L+/ IFN-γ+ central memory T-cells in CD4+/CD45RAlow (4 Donors). Class II Peptide chimeras give a robust CD4 memory T-cell recall response. Central memory T-cells are CD4+/CD45RAlow/CD62L+/IFN-γ+. The values shown are the percent of CD62L+/IFN-γ+ cells found in a CD4+/CD62L gate.

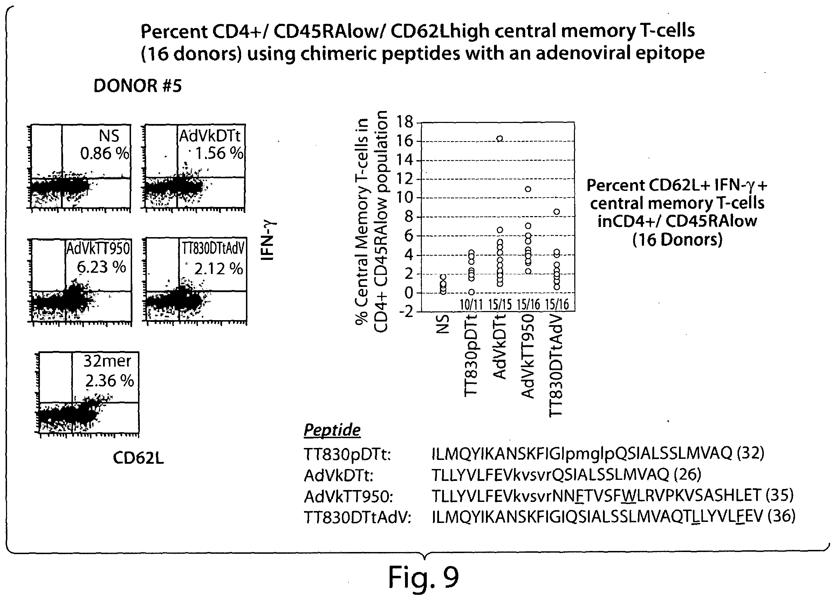

Figure 9 shows the percent CD4+/ CD45RAlow/ CD62Lhigh central memory T-cells (16 donors) using chimeric peptides with an adenoviral epitope. Class II peptide chimeras give a robust CD4 memory T-cell recall response. Central memory T-cells are CD4+/CD45RAlow/CD62L+/IFN-γ+. The values shown are the percent of CD62L+/IFN-γ+ cells found in a CD4+/CD62L gate. SEQ ID NOs: 13, 17, 19 and 20 are shown, respectively.
Figure 10 shows the percent CD4+/ CD45RAlow/ CD62Lhigh central memory T-cells (16 donors) in adenoviral AdVkDTt variants. Modified AdVkDTt peptide chimeras give a robust CD4 memory T-cell recall response. Central memory T-cells are CD4+/CD45RAlow/CD62L+/IFN-γ+. The values shown are the percent of CD62L+/IFN-γ+ cells found in a CD4+/CD62L gate. SEQ ID NOs: 71-73 and 127-129 are shown, respectively.
Figure 11 shows chimeric epitopes for influenza, selected for highly conserved pan HLA-DR profiles. SEQ ID NOs: 39-44, 32 and 93-98 are shown, respectively.
Figure 12 shows the percent CD4+/ CD45RAlow/ CD62Lhigh central memory T-cells (5 donors) in chimeric conserved influenza epitopes. Modified highly conserved Influenza peptide chimeras give a robust CD4 memory T-cell recall response. Central memory T-cells are CD4+/CD45RAlow/CD62L+/IFN-γ+. The values shown are the percent of CD62L+/IFN-γ+ cells found in a CD4+/CD62L gate. SEQ ID NOs: 101-106 are shown, respectively.
Figure 13 shows anti-nicotine titers generated using inventive compositions and synthetic nanocarriers.
Figure 14 shows anti-nicotine titers generated using inventive compositions and synthetic nanocarriers.

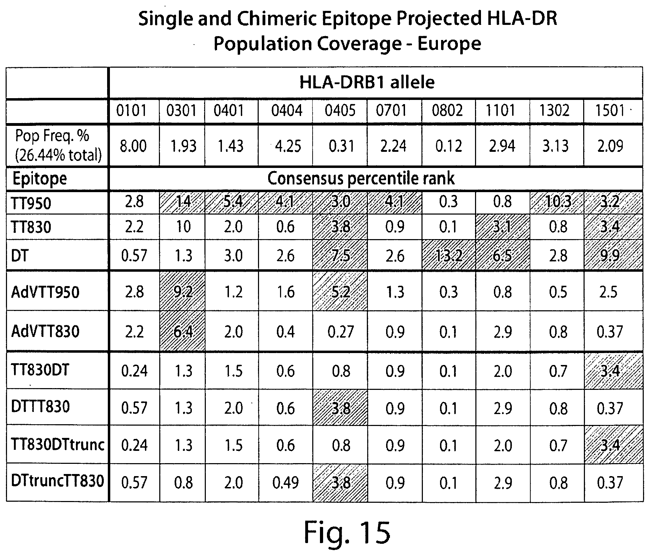

Figure 15 shows chimeric epitope selection using the Immune Epitope Database* (IEDB) T cell epitope prediction program. For each peptide, a percentile rank using each of three methods (ARB, SMM_align and Sturniolo) were generated by comparing the peptide's score against the scores of five million random 15 mers selected from SWISSPROT database. The percentile ranks for the three methods were then used to generate the rank for consensus method. A small numbered percentile rank indicates high affinity. Predicted high affinity binding (<3 top percentile) are in Bold. Allele distribution is given for European populations (Bulgarian, Croatian, Cuban (Eu), Czech, Finn, Georgian, Irish, North America (Eu), Slovenian.)
Figure 16 shows single and chimeric epitope projected HLA-DR population coverage - Europe.
Figure 17 shows a predicted binding analysis of individual Class II epitopes for Influenza A. SEQ ID NOs: 78-82 are depicted in the first column of the table.
Figure 18 shows a predicted binding analysis of chimeric epitopes for Influenza A.
Figure 19 shows conserved pan-Class II PB1 chimeric peptides for Influenza A+B. SEQ ID NOs: 101-106 are depicted in the first column of the table.
Figure 20 shows an amino acid substitution without loss of predicted binding affinity to Class II. SEQ ID NOs: 2, 120-122, 3 and 123-125 are shown, respectively.

### DETAILED DESCRIPTION OF THE INVENTION

Before describing the present invention in detail, it is to be understood that this invention is not limited to particularly exemplified materials or process parameters as such may, of course, vary. It is also to be understood that the terminology used herein is for the purpose of describing particular embodiments of the invention only, and is not intended to be limiting of the use of alternative terminology to describe the present invention.

As used in this specification and the appended claims, the singular forms "a," "an" and "the" include plural referents unless the content clearly dictates otherwise. For example, reference to "a polymer" includes a mixture of two or more such molecules, reference to "a solvent" includes a mixture of two or more such solvents, reference to "an adhesive" includes mixtures of two or more such materials, and the like.

### A. INTRODUCTION

Immune responses to vaccines can be beneficially enhanced to give a more robust antibody response by including a Class II binding memory epitope in the vaccine. However, Class II is made up of three different sets of genes, (HLA-DR, DP and DQ), each with different epitope binding affinities. In addition, each of the genes has several alleles that can be found in a population, which produce proteins with variable epitope binding ability, so that individual T cell epitopes are Class II allele restricted. Class II restriction of epitopes therefore causes a problem in that the epitope has limited population coverage. In order to get broad population coverage a peptide would have to be designed to be a promiscuous and non-selective for DP, DQ, and DR. This problem may be overcome by designing peptides to be specific for antigens that most of the population has been exposed to, and have broad activity across HLA class II alleles. Individual epitopes that have broad, but limited activity include for example, epitopes found in common vaccines such as tetanus toxin (TT) and diphtheria toxin (DT). In addition epitopes found in naturally occurring viruses such as adenovirus (AdV) to which most of the population has been exposed and have active antibody titres to, may have broad population coverage. Ideally, designed peptides will have a high affinity epitope for the dominant DP4 allele (DPA1*01/DPB1*401, and DPA1*0103/DPB1*0402) and /or high affinity epitopes for HLA-DR or HLA-DQ alleles with broad reactivity in a population. In order to identify broad coverage Class II peptides, the inventors designed and tested chimeric epitopes based on predicted HLA Class II affinities.

As shown in the Examples, the inventive peptides that were designed based on predicted HLA Class II affinities give broad coverage across multiple HLA class II DP, DQ, and DR alleles in humans, and give robust memory T-cell activation. These new peptides show a broad coverage across several Class II alleles, and a significant improvement in generating a CD4+ memory T-cell recall response.

Examples 1-4 illustrate the general inventive approach. Examples 5 and 6 illustrate peptide physical property modifications and inventive compositions obtained or derived from influenza virus. Examples 7-13 illustrate various applications of the inventive compositions.

The present invention will now be described in more detail.

### B. DEFINITION

"Adjuvant" means an agent that does not constitute a specific antigen, but boosts the strength and longevity of immune response to a co-administered antigen. Such adjuvants may include, but are not limited to stimulators of pattern recognition receptors, such as Toll-like receptors, RIG-1 and NOD-like receptors (NLR), mineral salts, such as alum, alum combined with monphosphoryl lipid (MPL) A of Enterobacteria, such as *Escherihia coli, Salmonella minnesota*, *Salmonella typhimurium*, or *Shigella flexneri* or specifically with MPL® (AS04), MPL A of above-mentioned bacteria separately, saponins, such as QS-21,Quil-A, ISCOMs, ISCOMATRIX™, emulsions such as MF59™, Montanide® ISA 51 and ISA 720, AS02 (QS21+squalene+ MPL®) , liposomes and liposomal formulations such as AS01, synthesized or specifically prepared microparticles and microcarriers such as bacteria-derived outer membrane vesicles (OMV) of *N. gonorrheae, Chlamydia trachomatis* and others, or chitosan particles, depot-forming agents, such as Pluronic® block co-polymers, specifically modified or prepared peptides, such as muramyl dipeptide, aminoalkyl glucosaminide 4-phosphates, such as RC529, or proteins, such as bacterial toxoids or toxin fragments.

In embodiments, adjuvants comprise agonists for pattern recognition receptors (PRR), including, but not limited to Toll-Like Receptors (TLRs), specifically TLRs 2, 3, 4, 5, 7, 8, 9 and/or combinations thereof. In other embodiments, adjuvants comprise agonists for Toll-Like Receptors 3, agonists for Toll-Like Receptors 7 and 8, or agonists for Toll-Like Receptor 9; preferably the recited adjuvants comprise imidazoquinolines; such as R848; adenine derivatives, such as those disclosed in US patent 6,329,381 (Sumitomo Pharmaceutical Company); immunostimulatory DNA; or immunostimulatory RNA. In specific embodiments, synthetic nanocarriers incorporate as adjuvants compounds that are agonists for toll-like receptors (TLRs) 7 & 8 ("TLR 7/8 agonists"). Of utility are the TLR 7/8 agonist compounds disclosed in US Patent 6,696,076 to Tomai et al., including but not limited to imidazoquinoline amines, imidazopyridine amines, 6,7-fused cycloalkylimidazopyridine amines, and 1,2-bridged imidazoquinoline amines. Preferred adjuvants comprise imiquimod and resiquimod (also known as R848). In specific embodiments, an adjuvant may be an agonist for the DC surface molecule CD40. In certain embodiments, to stimulate immunity rather than tolerance, a synthetic nanocarrier incorporates an adjuvant that promotes DC maturation (needed for priming of naive T cells) and the production of cytokines, such as type I interferons, which promote antibody immune responses. In embodiments, adjuvants also may comprise immunostimulatory RNA molecules, such as but not limited to dsRNA or poly I:C (a TLR3 stimulant), and/or those disclosed in F. Heil et al., "Species-Specific Recognition of Single-Stranded RNA via Toll-like Receptor 7 and 8" Science 303(5663), 1526-1529 (2004); J. Vollmer et al., "Immune modulation by chemically modified ribonucleosides and oligoribonucleotides" WO 2008033432 A2; A. Forsbach et al., "Immunostimulatory oligoribonucleotides containing specific sequence motif(s) and targeting the Toll-like receptor 8 pathway" WO 2007062107 A2; E. Uhlmann et al., "Modified oligoribonucleotide analogs with enhanced immunostimulatory activity" U.S. Pat. Appl. Publ. US 2006241076; G. Lipford et al., "Immunostimulatory viral RNA oligonucleotides and use for treating cancer and infections" WO 2005097993 A2; G. Lipford et al., "Immunostimulatory G,U-containing oligoribonucleotides, compositions, and screening methods" WO 2003086280 A2. In some embodiments, an adjuvant may be a TLR-4 agonist, such as bacterial lipopolysacccharide (LPS), VSV-G, and/or HMGB-1. In some embodiments, adjuvants may comprise TLR-5 agonists, such as flagellin, or portions or derivatives thereof, including but not limited to those disclosed in US Patents 6,130,082, 6,585,980, and 7,192,725. In specific embodiments, synthetic nanocarriers incorporate a ligand for Toll-like receptor (TLR)-9, such as immunostimulatory DNA molecules comprising CpGs, which induce type I interferon secretion, and stimulate T and B cell activation leading to increased antibody production and cytotoxic T cell responses (Krieg et al., CpG motifs in bacterial DNA trigger direct B cell activation. Nature. 1995. 374:546-549; Chu et al. CpG oligodeoxynucleotides act as adjuvants that switch on T helper 1 (Th1) immunity. J. Exp. Med. 1997. 186:1623-1631; Lipford et al. CpG-containing synthetic oligonucleotides promote B and cytotoxic T cell responses to protein antigen: a new class of vaccine adjuvants. Eur. J. Immunol. 1997. 27:2340-2344; Roman et al. Immunostimulatory DNA sequences function as T helper-1-promoting adjuvants.. Nat. Med. 1997. 3:849-854; Davis et al. CpG DNA is a potent enhancer of specific immunity in mice immunized with recombinant hepatitis B surface antigen. J. Immunol. 1998. 160:870-876; Lipford et al., Bacterial DNA as immune cell activator. Trends Microbiol. 1998. 6:496-500; US Patent 6,207,646 to Krieg et al.; US Patent 7,223,398 to Tuck et al.; US Patent 7,250,403 to Van Nest et al.; or US Patent 7,566,703 to Krieg et al.

In some embodiments, adjuvants may be proinflammatory stimuli released from necrotic cells (e.g., urate crystals). In some embodiments, adjuvants may be activated components of the complement cascade (e.g., CD21, CD35, etc.). In some embodiments, adjuvants may be activated components of immune complexes. The adjuvants also include complement receptor agonists, such as a molecule that binds to CD21 or CD35. In some embodiments, the complement receptor agonist induces endogenous complement opsonization of the synthetic nanocarrier. In some embodiments, adjuvants are cytokines, which are small proteins or biological factors (in the range of 5 kD - 20 kD) that are released by cells and have specific effects on cell-cell interaction, communication and behavior of other cells. In some embodiments, the cytokine receptor agonist is a small molecule, antibody, fusion protein, or aptamer.

In embodiments, at least a portion of the dose of adjuvant may be coupled to synthetic nanocarriers, preferably, all of the dose of adjuvant is coupled to synthetic nanocarriers. In other embodiments, at least a portion of the dose of the adjuvant is not coupled to the synthetic nanocarriers. In embodiments, the dose of adjuvant comprises two or more types of adjuvants. For instance, and without limitation, adjuvants that act on different TLR receptors may be combined. As an example, in an embodiment a TLR 7/8 agonist may be combined with a TLR 9 agonist. In another embodiment, a TLR 7/8 agonist may be combined with a TLR 4 agonist. In yet another embodiment, a TLR 9 agonist may be combined with a TLR 3 agonist.

"Administering" or "administration" means providing a drug to a subject in a manner that is pharmacologically useful.

"Antigen" means a B cell antigen or T cell antigen.

"B cell antigen" means any antigen that is or recognized by and triggers an immune response in a B cell (e.g., an antigen that is specifically recognized by a B cell receptor on a B cell). In some embodiments, an antigen that is a T cell antigen is also a B cell antigen. In other embodiments, the T cell antigen is not also a B cell antigen. B cell antigens include, but are not limited to proteins, peptides, small molecules, and carbohydrates. In some embodiments, the B cell antigen is a non-protein antigen (i.e., not a protein or peptide antigen). In some embodiments, the B cell antigen is a carbohydrate associated with an infectious agent. In some embodiments, the B cell antigen is a glycoprotein or glycopeptide associated with an infectious agent. The infectious agent can be a bacterium, virus, fungus, protozoan, or parasite. In some embodiments, the B cell antigen is a poorly immunogenic antigen. In some embodiments, the B cell antigen is an abused substance or a portion thereof. In some embodiments, the B cell antigen is an addictive substance or a portion thereof. Addictive substances include, but are not limited to, nicotine, a narcotic, a cough suppressant, a tranquilizer, and a sedative. In some embodiments, the B cell antigen is a toxin, such as a toxin from a chemical weapon or natural sources. The B cell antigen may also be a hazardous environmental agent. In some embodiments, the B cell antigen is a self antigen. In other embodiments, the B cell antigen is an alloantigen, an allergen, a contact sensitizer, a degenerative disease antigen, a hapten, an infectious disease antigen, a cancer antigen, an atopic disease antigen, an autoimmune disease antigen, an addictive substance, a xenoantigen, or a metabolic disease enzyme or enzymatic product thereof.

"Couple" or "Coupled" or "Couples" (and the like) means to chemically associate one entity (for example a moiety) with another. In some embodiments, the coupling is covalent. In non-covalent embodiments, the non-covalent coupling is mediated by non-covalent interactions including but not limited to charge interactions, affinity interactions, metal coordination, physical adsorption, host-guest interactions, hydrophobic interactions, TT stacking interactions, hydrogen bonding interactions, van der Waals interactions, magnetic interactions, electrostatic interactions, dipole-dipole interactions, and/or combinations thereof.

"Derived" means taken from a source and subjected to substantial modification. For instance, a peptide or nucleic acid with a sequence with only 50% identity to a natural peptide or nucleic acid, preferably a natural consensus peptide or nucleic acid, would be said to be derived from the natural peptide or nucleic acid. Nucleic acids that are derived, however, are not intended to include nucleic acids with sequences that are non-identical to a natural nucleic acid sequence, preferably a natural consensus nucleic acid sequence, solely due to degeneracy of the genetic code. Substantial modification is modification that significantly affects the chemical or immunological properties of the material in question. Derived peptides and nucleic acids can also include those with a sequence with greater than 50% identity to a natural peptide or nucleic acid sequence if said derived peptides and nucleic acids have altered chemical or immunological properties as compared to the natural peptide or nucleic acid. These chemical or immunological properties comprise hydrophilicity, stability, binding affinity to MHC II, and ability to couple with a carrier such as a synthetic nanocarrier.

"Dosage form" means a drug in a medium, carrier, vehicle, or device suitable for administration to a subject.

"Encapsulate" or "encapsulated" means to enclose within a synthetic nanocarrier, preferably enclose completely within a synthetic nanocarrier. Most or all of a substance that is encapsulated is not exposed to the local environment external to the synthetic nanocarrier. Encapsulation is distinct from the presence of at least a portion of a substance on a surface of a synthetic nanocarrier, which leaves the substance exposed to the local environment external to the synthetic nanocarrier. In an embodiment, an example of a process that results in at least a portion of a substance being present on a surface of the synthetic nanocarrier is adsorption.

"MHC II binding peptide" means a peptide that binds to the Major Histocompatability Complex Class II at sufficient affinity to allow the peptide/MHC complex to interact with a T-cell receptor on T-cells. The interaction of the peptide/MHC complex with T-cell receptor on T-cells can be established through measurement of cytokine production and/or T-cell proliferation using conventional techniques. In embodiments, MHC II binding peptides have an affinity IC50 value of 5000 nM or less, preferably 500 nM or less, and more preferably 50 nM or less for binding to an MHC II molecule. In embodiments, MHC II binding peptides according to the invention (expressly including first, second, and third MHC II binding peptides) have lengths equal to or greater than 5-mer, and can be as large as a protein. In other embodiments, MHC II binding peptides according to the invention (expressly including first, second, and third MHC II binding peptides) have lengths ranging from 5-mer to 50-mer, preferably ranging from 5-mer to 40-mer, more preferably ranging from 5-mer to 30-mer, and still more preferably from 6-mer to 25-mer.

"Identity" means the percentage of amino acid or residues or nucleic acid bases that are identically positioned in a one-dimensional sequence alignment. Identity is a measure of how closely the sequences being compared are related. In an embodiment, identity between two sequences can be determined using the BESTFIT program. In embodiments, the recited MHC II binding peptides (such as A, B, or C) may have at least 70%, preferably at least 80%, more preferably at least 90%, even more preferably at least 95%, even more preferably at least 97%, or even more preferably at least 99% identity to a natural HLA-DP binding peptide, a natural HLA-DQ binding peptide, and/or a natural HLA-DR binding peptide. In embodiments, A, B, and C are not 100% identical to one another; and in embodiments A and B are not 100% identical to one another. In embodiments, the recited nucleic acids may have at least 60%, preferably at least 70%, more preferably at least 80%, even more preferably at least 90%, even more preferably at least 95%, even more preferably at least 97%, or even more preferably at least 99% identity to a nucleic acid sequence that encodes, or is complementary to one that encodes, a natural HLA-DP binding peptide, a natural HLA-DQ binding peptide, and/or a natural HLA-DR binding peptide.

"Isolated nucleic acid" means a nucleic acid that is separated from its native environment and present in sufficient quantity to permit its identification or use. An isolated nucleic acid may be one that is (i) amplified *in vitro* by, for example, polymerase chain reaction (PCR); (ii) recombinantly produced by cloning; (iii) purified, as by cleavage and gel separation; or (iv) synthesized by, for example, chemical synthesis. An isolated nucleic acid is one which is readily manipulable by recombinant DNA techniques well known in the art. Thus, a nucleotide sequence contained in a vector in which 5' and 3' restriction sites are known or for which polymerase chain reaction (PCR) primer sequences have been disclosed is considered isolated but a nucleic acid sequence existing in its native state in its natural host is not. An isolated nucleic acid may be substantially purified, but need not be. For example, a nucleic acid that is isolated within a cloning or expression vector is not pure in that it may comprise only a tiny percentage of the material in the cell in which it resides. Such a nucleic acid is isolated, however, as the term is used herein because it is readily manipulable by standard techniques known to those of ordinary skill in the art. Any of the nucleic acids provided herein may be isolated.

"Isolated polypeptide" means the polypeptide is separated from its native environment and present in sufficient quantity to permit its identification or use. This means, for example, the polypeptide may be (i) selectively produced by expression cloning or (ii) purified as by chromatography or electrophoresis. Isolated proteins or polypeptides may be, but need not be, substantially pure. Because an isolated polypeptide may be admixed with a pharmaceutically acceptable carrier in a pharmaceutical preparation, the polypeptide may comprise only a small percentage by weight of the preparation. The polypeptide is nonetheless isolated in that it has been separated from the substances with which it may be associated in living systems, e.g., isolated from other proteins. Any of the peptides or polypeptides provided herein may be isolated.

"Linker" means a moiety that connects two chemical components together through either a single covalent bond or multiple covalent bonds.

"Maximum dimension of a synthetic nanocarrier" means the largest dimension of a nanocarrier measured along any axis of the synthetic nanocarrier. "Minimum dimension of a synthetic nanocarrier" means the smallest dimension of a synthetic nanocarrier measured along any axis of the synthetic nanocarrier. For example, for a spheroidal synthetic nanocarrier, the maximum and minimum dimension of a synthetic nanocarrier would be substantially identical, and would be the size of its diameter. Similarly, for a cubiodal synthetic nanocarrier, the minimum dimension of a synthetic nanocarrier would be the smallest of its height, width or length, while the maximum dimension of a synthetic nanocarrier would be the largest of its height, width or length. In an embodiment, a minimum dimension of at least 75%, preferably at least 80%, more preferably at least 90%, of the synthetic nanocarriers in a sample, based on the total number of synthetic nanocarriers in the sample, is greater than 100 nm. In a embodiment, a maximum dimension of at least 75%, preferably at least 80%, more preferably at least 90%, of the synthetic nanocarriers in a sample, based on the total number of synthetic nanocarriers in the sample, is equal to or less than 5 µm. Preferably, a minimum dimension of at least 75%, preferably at least 80%, more preferably at least 90%, of the synthetic nanocarriers in a sample, based on the total number of synthetic nanocarriers in the sample, is equal to or greater than 110 nm, more preferably equal to or greater than 120 nm, more preferably equal to or greater than 130 nm, and more preferably still equal to or greater than 150 nm. Preferably, a maximum dimension of at least 75%, preferably at least 80%, more preferably at least 90%, of the synthetic nanocarriers in a sample, based on the total number of synthetic nanocarriers in the sample is equal to or less than 3 µm, more preferably equal to or less than 2 µm, more preferably equal to or less than 1 µm, more preferably equal to or less than 800 nm, more preferably equal to or less than 600 nm, and more preferably still equal to or less than 500 nm. In preferred embodiments, a maximum dimension of at least 75%, preferably at least 80%, more preferably at least 90%, of the synthetic nanocarriers in a sample, based on the total number of synthetic nanocarriers in the sample, is equal to or greater than 100nm, more preferably equal to or greater than 120 nm, more preferably equal to or greater than 130 nm, more preferably equal to or greater than 140 nm, and more preferably still equal to or greater than 150 nm. Measurement of synthetic nanocarrier sizes is obtained by suspending the synthetic nanocarriers in a liquid (usually aqueous) media and using dynamic light scattering (e.g. using a Brookhaven ZetaPALS instrument).

"Natural HLA-DP binding peptide" means a peptide obtained or derived from nature that binds specifically to an MHC Class II Human Leukocyte Antigen DP at sufficient affinity to allow the peptide/HLA-DP complex to interact with the T-cell receptor on T-cells. In embodiments, natural HLA-DP binding peptides have an affinity IC50 value of 5000 nM or less, preferably 500 nM or less, and more preferably 50 nM or less for an MHC Class II Human Leukocyte Antigen DP. In embodiments, the natural HLA-DP binding peptide comprises a peptide sequence obtained or derived from viruses, bacteria or yeast, including but not limited to: Clostridium tetani, Hepatitis B virus, Human herpes virus, Influenza virus, Vaccinia virus, Epstein-Barr virus (EBV), Chicken pox virus, Measles virus, Rous sarcoma virus (RSV), Cytomegalovirus (CMV), Varicella zoster virus (VZV), Mumps virus, Corynebacterium diphtheria, Human adenoviridae, and/or Smallpox virus. Class II epitope prediction was done using the Immune Epitope Database* (IEDB) (http://www.immuneepitope.org/) T cell epitope prediction tools. For each peptide, a percentile rank for each of three methods (ARB, SMM_align and Sturniolo) was generated by comparing the peptide's score against the scores of five million random 15 mers selected from SWISSPROT database. The percentile ranks for the three methods were then used to generate the rank for consensus method.

"Natural HLA-DQ binding peptide" means a peptide obtained or derived from nature that binds specifically to an MHC Class II Human Leukocyte Antigen DQ at sufficient affinity to allow the peptide/HLA-DQ complex to interact with the T-cell receptor on T-cells. In embodiments, natural HLA-DQ binding peptides have an affinity IC50 value of 5000 nM or less, preferably 500 nM or less, and more preferably 50 nM or less for an MHC Class II Human Leukocyte Antigen DQ. In embodiments, the natural HLA-DQ binding peptide comprises a peptide sequence obtained or derived from viruses, bacteria or yeast, including but not limited to: Clostridium tetani, Hepatitis B virus, Human herpes virus, Influenza virus, Vaccinia virus, Epstein-Barr virus (EBV), Chicken pox virus, Measles virus, Rous sarcoma virus (RSV), Cytomegalovirus (CMV), Varicella zoster virus (VZV), Mumps virus, Corynebacterium diphtheria, Human adenoviridae, and/or Smallpox virus. Class II epitope prediction was done using the Immune Epitope Database* (IEDB) (http://www.immuneepitope.org/) T cell epitope prediction tools. For each peptide, a percentile rank for each of three methods (ARB, SMM_align and Sturniolo) was generated by comparing the peptide's score against the scores of five million random 15 mers selected from SWISSPROT database. The percentile ranks for the three methods were then used to generate the rank for consensus method.

"Natural HLA-DR binding peptide" means a peptide obtained or derived from nature that binds specifically to an MHC Class II Human Leukocyte Antigen DR at sufficient affinity to allow the peptide/HLA-DR complex to interact with the T-cell receptor on T-cells. In embodiments, natural HLA-DR binding peptides have an affinity IC50 value of 5000 nM or less, preferably 500 nM or less, and more preferably 50 nM or less for an MHC Class II Human Leukocyte Antigen DR. In embodiments, the natural HLA-DR binding peptide comprises a peptide sequence obtained or derived from viruses, bacteria or yeast, including but not limited to: Clostridium tetani, Hepatitis B virus, Human herpes virus, Influenza virus, Vaccinia virus, Epstein-Barr virus (EBV), Chicken pox virus, Measles virus, Rous sarcoma virus (RSV), Cytomegalovirus (CMV), Varicella zoster virus (VZV), Mumps virus, Corynebacterium diphtheria, Human adenoviridae, and/or Smallpox virus. Class II epitope prediction was done using the Immune Epitope Database* (IEDB) (http://www.immuneepitope.org/) T cell epitope prediction tools. For each peptide, a percentile rank for each of three methods (ARB, SMM_align and Sturniolo) was generated by comparing the peptide's score against the scores of five million random 15 mers selected from SWISSPROT database. The percentile ranks for the three methods were then used to generate the rank for consensus method.

"Obtained" means taken from a source without substantial modification. Substantial modification is modification that significantly affects the chemical or immunological properties of the material in question. For example, as a non-limiting example, a peptide or nucleic acid with a sequence with greater than 90%, preferably greater than 95%, preferably greater than 97%, preferably greater than 98%, preferably greater than 99%, preferably 100%, identity to a natural peptide or nucleotide sequence, preferably a natural consensus peptide or nucleotide sequence, and chemical and/or immunological properties that are not significantly different from the natural peptide or nucleic acid would be said to be obtained from the natural peptide or nucleotide sequence. Nucleic acids that are obtained are intended to include nucleic acids with sequences that are non-identical to a natural consensus nucleotide sequence solely due to degeneracy of the genetic code. Such nucleic acids may even have a sequence with less than 90% identity to a natural nucleotide sequence, preferably a natural consensus nucleotide sequence. These chemical or immunological properties comprise hydrophilicity, stability, binding affinity to MHC II, and ability to couple with a carrier such as a synthetic nanocarrier.

"Pharmaceutically acceptable excipient" means a pharmacologically inactive material used together with the recited peptides in formulating embodiments of the inventive compositions, dosage forms, vaccines, and the like. Pharmaceutically acceptable excipients comprise a variety of materials known in the art, including but not limited to saccharides (such as glucose, lactose, and the like), preservatives such as antimicrobial agents, reconstitution aids, colorants, saline (such as phosphate buffered saline), buffers, dispersants, stabilizers, other excipients noted herein, and other such materials that are conventionally known.

"Subject" means animals, including warm blooded mammals such as humans and primates; avians; domestic household or farm animals such as cats, dogs, sheep, goats, cattle, horses and pigs; laboratory animals such as mice, rats and guinea pigs; fish; reptiles; zoo and wild animals; and the like.

"Synthetic nanocarrier(s)" means a discrete object that is not found in nature, and that possesses at least one dimension that is less than or equal to 5 microns in size. Albumin nanoparticles are generally included as synthetic nanocarriers, however in certain embodiments the synthetic nanocarriers do not comprise albumin nanoparticles. In embodiments, the synthetic nanocarriers do not comprise chitosan.

A synthetic nanocarrier can be, but is not limited to, one or a plurality of lipid-based nanoparticles, polymeric nanoparticles, metallic nanoparticles, surfactant-based emulsions, dendrimers, buckyballs, nanowires, virus-like particles, peptide or protein-based particles (such as albumin nanoparticles) and/or nanoparticles that are developed using a combination of nanomaterials such as lipid-polymer nanoparticles. Synthetic nanocarriers may be a variety of different shapes, including but not limited to spheroidal, cuboidal, pyramidal, oblong, cylindrical, toroidal, and the like. Synthetic nanocarriers according to the invention comprise one or more surfaces. Exemplary synthetic nanocarriers that can be adapted for use in the practice of the present invention comprise: (1) the biodegradable nanoparticles disclosed in US Patent 5,543,158 to Gref et al., (2) the polymeric nanoparticles of Published US Patent Application 20060002852 to Saltzman et al., (3) the lithographically constructed nanoparticles of Published US Patent Application 20090028910 to DeSimone et al., (4) the disclosure of WO 2009/051837 to von Andrian et al., or (5) the nanoparticles disclosed in Published US Patent Application 2008/0145441 to Penades et al. In embodiments, synthetic nanocarriers may possess an aspect ratio greater than 1:1, 1:1.2, 1:1.5, 1:2, 1:3, 1:5, 1:7, or greater than 1:10.

Synthetic nanocarriers according to the invention that have a minimum dimension of equal to or less than about 100 nm, preferably equal to or less than 100 nm, do not comprise a surface with hydroxyl groups that activate complement or alternatively comprise a surface that consists essentially of moieties that are not hydroxyl groups that activate complement. In a preferred embodiment, synthetic nanocarriers according to the invention that have a minimum dimension of equal to or less than about 100 nm, preferably equal to or less than 100 nm, do not comprise a surface that substantially activates complement or alternatively comprise a surface that consists essentially of moieties that do not substantially activate complement. In a more preferred embodiment, synthetic nanocarriers according to the invention that have a minimum dimension of equal to or less than about 100 nm, preferably equal to or less than 100 nm, do not comprise a surface that activates complement or alternatively comprise a surface that consists essentially of moieties that do not activate complement. In an embodiment, synthetic nanocarriers according to the invention exclude virus-like particles.

"T cell antigen" means a CD4+ T-cell antigen or a CD8+ cell antigen. "CD4+ T-cell antigen" means any antigen that is recognized by and triggers an immune response in a CD4+ T-cell e.g., an antigen that is specifically recognized by a T-cell receptor on a CD4+T cell via presentation of the antigen or portion thereof bound to a Class II major histocompatability complex molecule (MHC). "CD8+ T cell antigen" means any antigen that is recognized by and triggers an immune response in a CD8+ T-cell e.g., an antigen that is specifically recognized by a T-cell receptor on a CD8+T cell via presentation of the antigen or portion thereof bound to a Class I major histocompatability complex molecule (MHC). In some embodiments, an antigen that is a T cell antigen is also a B cell antigen. In other embodiments, the T cell antigen is not also a B cell antigen. T cell antigens generally are proteins or peptides, but may be other molecules such as lipids and glycolipids. T cell antigens are antigens that stimulate a CD4+ T cell response or a CD8+ T cell response.

"Vaccine" means a composition of matter that improves the immune response to a particular pathogen or disease. A vaccine typically contains factors that stimulate a subject's immune system to recognize a specific antigen as foreign and eliminate it from the subject's body. A vaccine also establishes an immunologic 'memory' so the antigen will be quickly recognized and responded to if a person is re-challenged. Vaccines can be prophylactic (for example to prevent future infection by any pathogen), or therapeutic (for example a vaccine against a tumor specific antigen for the treatment of cancer). Vaccines according to the invention may comprise one or more MHC II binding peptides, or one or more nucleic acids that encode, or is complementary to the one or more nucleic acids that encode, the one or more MHC II binding peptides.

### C. INVENTIVE PEPTIDES & METHODS OF MAKING AND USING THEM

In embodiments, the inventive compositions and related methods comprise A - x - B, wherein x comprises a cathepsin cleavage site comprising KVSVR, A comprises a first MHC II binding peptide, and B comprises a second MHC II binding peptide. Additionally, in embodiments the inventive compositions and related methods comprise A - x - B - y -- C, wherein x comprises a cathepsin cleavage stie comprising KVSVR, y may comprise a linker or no linker, A comprises a first MHC II binding peptide, B comprises a second MHC II binding peptide, and C comprises a third MHC II binding peptide.

In certain embodiments, y if y is present, may comprise no linker, in which case A-x-B and C may be present in the inventive compositions as mixtures. Such a mixture approach can be used to easily combine a number of different MHC II binding peptides thus providing ease of use and/or synthesis simplification over, for instance, creating a single larger molecule that contains residues of the MHC II binding peptides. Mixtures may be formulated using traditional pharmaceutical mixing methods. These include liquid-liquid mixing in which two or more suspensions, each containing one or more sets of peptides, are directly combined or are brought together via one or more vessels containing diluent. As peptides may also be produced or stored in a powder form, dry powder-powder mixing could be performed as could the re-suspension of two or more powders in a common media. Depending on the properties of the peptides and their interaction potentials, there may be advantages conferred to one or another route of mixing.

The mixtures may be made using conventional pharmaceutical manufacturing and compounding techniques to arrive at useful dosage forms. Techniques suitable for use in practicing the present invention may be found in Handbook of Industrial Mixing: Science and Practice, Edited by Edward L. Paul, Victor A. Atiemo-Obeng, and Suzanne M. Kresta, 2004 John Wiley & Sons, Inc.; and Pharmaceutics: The Science of Dosage Form Design, 2nd Ed. Edited by M. E. Auten, 2001, Churchill Livingstone. In embodiments, typical inventive compositions that comprise the peptide mixtures may comprise inorganic or organic buffers (e.g., sodium or potassium salts of phosphate, carbonate, acetate, or citrate) and pH adjustment agents (e.g., hydrochloric acid, sodium or potassium hydroxide, salts of citrate or acetate, amino acids and their salts) antioxidants (e.g., ascorbic acid, alpha-tocopherol), surfactants (e.g., polysorbate 20, polysorbate 80, polyoxyethylene9-10 nonyl phenol, sodium desoxycholate), solution and/or cryo/lyo stabilizers (e.g., sucrose, lactose, mannitol, trehalose), osmotic adjustment agents (e.g., salts or sugars), antibacterial agents (e.g., benzoic acid, phenol, gentamicin), antifoaming agents (e.g., polydimethylsilozone), preservatives (e.g., thimerosal, 2-phenoxyethanol, EDTA), polymeric stabilizers and viscosity-adjustment agents (e.g., polyvinylpyrrolidone, poloxamer 488, carboxymethylcellulose) and co-solvents (e.g., glycerol, polyethylene glycol, ethanol).

In embodiments, y if it is present, may comprise a linker. In embodiments, a linker may directly connect amino acids - either natural or modified - that are part of the MHC II binding peptide, or a linker may add atoms, preferably multiple atoms, to link the MHC II binding peptides. Linkers may be useful for a number of reasons, including but not limited to ease of synthesis, facilitation of chemical cleavage, separation of MHC II binding peptides, insertion of a chemically reactive site (like a disulfide) and/or a protease cleavage site. Linkers may comprise cleavable linkers that are cleaved under certain physiological conditions and non-cleavable linkers that are poorly cleaved under typical physiological conditions encountered by the inventive compositions when administered to a subject.

In certain embodiments, y if it is present, may comprise a linker that comprises an amide linker, a disulfide linker, a sulfide linker, a 1,4-disubstituted 1,2,3-triazole linker, a thiol ester linker, or an imine linker. Additional linkers useful in the practice of the present invention comprise: thiol ester linkers formed from thiol and acid, hydrazide linkers formed from hydrazine and acid, imine linkers formed from amine and aldehyde or ketone, thiourea linkers formed from thiol and thioisocyante, amidine linkers formed from amine and imidate ester, and amine linkers formed from reductive amination of amine and aldehyde. In embodiments, x and/or y if it is present may comprise a linker that comprises a peptide sequence, preferably sequences that comprise a lysosome protease cleavage site (e.g. a cathepsin cleavage site), a biodegradable polymer, a substituted or unsubstituted alkane, alkene, aromatic or heterocyclic linker, a pH sensitive polymer, heterobifunctional linkers or an oligomeric glycol spacer.

Cleavable linkers include, but are not limited to peptide sequences, preferably peptide sequences that comprise a lysosomal protease cleavage site; a biodegradable polymer; a pH degradable polymer; or a disulfide bond. Lysosomal protease cleavage sites comprise peptide sequences specifically known to be cleaved by lysosomal proteases comprising serine proteases, threonine proteases, aspartate proteases, zinc proteases, metalloproteases glutamic acid proteases, cysteine proteases (AMSH/STAMBP Cathepsin F, Cathepsin 3, Cathepsin H, Cathepsin 6, Cathepsin L, Cathepsin 7/Cathepsin 1 Cathepsin O, Cathepsin A Cathepsin S, Cathepsin B, Cathepsin V, Cathepsin C/DPPI, Cathepsin X/Z/P, Cathepsin D, Legumain). Biodegradable polymers degrade under a variety of physiological conditions, while pH degradable polymers degrade at an accelerated rate under low (less than physiological pH) pH condition. In certain embodiments, the peptide sequence of the linker comprises an amino acid sequence as set forth in SEQ ID NO: 99 or 119.
pmglp (SEQ ID NO: 99)
skvsvr (SEQ ID NO: 119)

Additional information may be found in: A. Purcell et al., "More than one reason to rethink the use of peptides in vaccine design." J.Nat Rev Drug Discov. 2007; 5:404-14; R. Bei et al., "TAA polyepitope DNA-based vaccines: A potential tool for cancer therapy." J Biomed Biotech. 2010 ; 102785: 1-12; W. Wriggers et al., "Control of protein functional dynamics by peptide linkers." Biopolymers. 2005;80(6):736-46; J. Timmerman et al., "Carrier protein conjugate vaccines: the "missing link" to improved antibody and CTL responses?" Hum Vaccin. 2009 Mar;5(3):181-3' B. Law et al., "Proteolysis: a biological process adapted in drug delivery, therapy, and imaging." Bioconjug Chem. 2009 Sep;20(9):1683-95.

An amide linker is the linker formed between an amino group on one chemical component with the carboxyl group of a second chemical component. These linkers can be made using any of the conventional amide linker forming chemistries with suitably protected amino acids or polypeptides. In an embodiment, the recited amide linkers could be formed during overall synthesis of A and B (or B and C, etc.), thus simplifying the creation of x and/or y. This type of linking chemistry can be easily arranged to include a cleavable linking group.

A disulfide linker is a linker between two sulfur atoms of the form, for instance, of R₁-S-S-R₂. A disulfide linker can be formed by oxidative coupling of two same or dissimilar molecules such as peptides containing mercaptan substituents (-SH) or, preferably, by using a pre-formed linker of the form, for instance, of:

H₂N- R₁-S-S-R₂-CO₂H where the amino and or the carboxyl function are suitably protected. This type of linking chemistry is susceptible to reductive cleavage which would lead to the separation of the two individual memory peptides. This is significant because a reducing environment may be found in lysosomes, which is a target compartment of immunological interest.

Hydrazide and aldehyde/ketone chemistry may be used to form linkers. A first peptide containing a hydrazide or aldehyde/ketone function, terminal to the first peptide chain is prepared. A second peptide is prepared with either a hydrazide (if the first peptide contains an aldehyde/ketone) or an aldehyde/ketone (if the first peptide contains a hydrazide) terminal to the second peptide chain. The two peptides are then allowed to react which links the two peptides through a hydrazone function. In general, the hydrazone bond thus formed is cleavable under acidic conditions, such as those found in the lysozome. If greater stability of the linker is desired, the hydrazone can be reduced to form the corresponding stable (non-cleavable) alkylated hydrazide (similar to reductive amination of an amine with aldehyde or ketone to form the corresponding alkylamine).

Non-cleavable linkers can be formed using a variety of chemistries and can be formed using a number of different materials. Generally, a linker is considered non-cleavable when each such non-cleavable linker is stable for more than 12 hours under lysosomal pH conditions. Examples of non-cleavable linkers include but are not limited to groups containing amines, sulfides, triazoles, hydrazones, amide(ester)s, and substituted or unsubstituted alkanes, alkenes, aromatics or heterocycles; polymers; oligomeric glycol spacers; and/or non-natural or chemically modified amino acids. The following are examples of several common methodologies. The list is by no means complete and many other methods are possible.

A sulfide linker is of the form, for instance, of R₁-S-R₂. This linker can be made by either alkylation of a mercaptan or by Michael addition of a mercaptan on one molecule such as a peptide to an activated alkene on a second molecule such as a peptide, or by the radical addition of a mercaptan on one molecule such as a peptide to an alkene on a second molecule such as a peptide. The sulfide linker can also be pre-formed as, for instance: H₂N- R₁-S-R₂-CO₂H where the amino and or the carboxyl function are suitably protected. This type of linker is resistant to cleavage, but can be used to specifically link two suitably substituted and protected peptides.

A triazole linker may be specifically a 1,2,3-triazine of the form 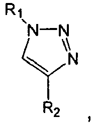 wherein R₁ and R₂ may be any chemical entities, and is made by the 1,3-dipolar addition of an azide attached to a first peptide to a terminal alkyne attached to a second peptide. This chemistry is described in detail by Sharpless et al., Angew. Chem. Int. Ed. 41(14), 2596, (2002), and is often referred to as "Sharpless click chemistry". A first peptide containing an azide or alkyne function, terminal to the first peptide chain is prepared. A second peptide is prepared with either an alkyne (if the first peptide contains an azide) or an azide (if the first peptide contains an alkyne) terminal to the second peptide chain. The two peptides are then allowed to react in a 3 + 2 cycloaddition with or without a catalyst which links the two peptides through a 1,2,3-triazine function.

Sulfur "click" chemistry may be used to form a linker. A first peptide containing a mercaptan or alkene function, terminal to the first peptide chain is prepared. A second peptide is prepared with either an alkene (if the first peptide contains a mercaptan) or a mercaptan (if the first peptide contains an alkene) terminal to the second peptide chain. The two peptides are allowed to react in the presence of light or a radical source which links the two peptides through a sulfide function.

Michael addition chemistry may be used to form a linker. Though a variety of Michael acceptor and donor pairs may be used for this purpose, a preferable example of thismethod is the use of mercaptans as the Michael donor and activated alkenes as the Michael acceptor. This chemistry differs from the sulfur click chemistry above in that the alkene needs to be electron deficient and radical catalysis is not necessary. A first peptide containing a mercaptan or alkene function, terminal to the first peptide chain is prepared. A second peptide is prepared with either an alkene (if the first peptide contains a mercaptan) or a mercaptan (if the first peptide contains an alkene) terminal to the second peptide chain. The two peptides are allowed to react in the presence of acid or base which links the two peptides through a sulfide function.

In embodiments, A and B; A and C, B and C, and A, B, and C each comprise peptides having different MHC II binding repertoires. DP, DQ and DR are proteins encoded by independent genes. In an outbred human population there are a large number of variants (alleles) of DP, DQ and DR, and each allele has a different characteristic peptide binding. For example a particular natural HLA-DP binding peptide may bind some DP alleles but not others. A peptide "binding repertoire" refers to the combination of alleles found in DP, DQ and/or DR to which an individual peptide will bind. Identification of peptides and/or combinations thereof that bind all DP, DQ and/or DR alleles, thus generating memory recall responses in a high percentage of people up to and including 100% of people, provides a means of improving vaccine efficiency.

In embodiments, A and B each comprise a sequence obtained or derived from a different infectious organism. In embodiments, A, B, and C each comprise a peptide sequence obtained or derived from a different infectious organism. In embodiments, preferred peptide sequences could be that of a peptide or protein epitope that can be recognized by a T-cell. Preferred peptide sequences comprise those MHC II binding peptides obtained or derived from Clostridium tetani, Hepatitis B virus, Human herpes virus, Influenza virus, Vaccinia virus, Epstein barr virus (EBV), Chicken pox virus, Measles virus, Rous sarcoma virus (RSV), Cytomegalovirus (CMV), Varicella zoster virus (VZV), Mumps virus, Corynebacterium diphtheria, Human adenoviridae, Small pox virus, and/or an infectious organism capable of infecting humans and generating human CD4+ memory cells specific to that infectious organism following the initiation of the infection. In embodiments the MHC II binding peptides comprise peptides having at least 70%, preferably at least 80%, more preferably at least 90%, even more preferably at least 95%, even more preferably at least 97%, or even more preferably at least 99% identity to a natural HLA-DP binding peptide, a natural HLA-DQ binding peptide, and/or a natural HLA-DR binding peptide obtained or derived from Clostridium tetani, Hepatitis B virus, Human herpes virus, Influenza virus, Vaccinia virus, Epstein barr virus (EBV), Chicken pox virus, Measles virus, Rous sarcoma virus (RSV), Cytomegalovirus (CMV), Varicella zoster virus (VZV), Mumps virus, Corynebacterium diphtheria, Human adenoviridae, Small pox virus, and/or an infectious organism capable of infecting humans and generating human CD4+ memory cells specific to that infectious organism following the initiation of the infection. In embodiments, A, B, and C are selected so as to provide an optimum immune response using the general strategies outlined in Examples 1-6 below.

In certain embodiments, for the purposes such as ease of processing, formulation, and/or for improved delivery within a biological system, it may be desirable to increase the aqueous solubility of the MHC II binding peptide. To this end, an increase in hydrophilicity may be achieved by adding hydrophilic N- and/or C-terminal amino acids, by adding or modifying amino acid sequences between binding sites, or by making substitutions to binding site amino acids. Increase in hydrophilicity may, for example, be measured by means of a lower GRAVY, Grand Average of Hydropathy, score. Where feasible, the design of prospective modifications may be influenced such as to avoid, or limit, potential negative effects on binding affinity.

One potential route of modification is the addition of non-binding site amino acids based on the amino acids adjacent to the binding site epitope, especially if those flanking amino acids would increase the average or local hydrophilicity of the peptide. That is, if a binding site epitope in its native extended sequence is flanked by hydrophilic amino acids to the N- and/or C-terminal side, then preserving some of those flanking hydrophilic amino acids in the peptide may increase its aqueous solubility. In the absence of flanking sequences that would likely increase solubility, or in the case that further increases in hydrophilicity are desired, non-native additions may be made, ideally based on similarity to the native sequence. Amino acid similarity may be judged by indices such as Blosum 45 or PAM 250 matrices or by other means known in the art. For example, if an epitope has a GRAVY score of - 1.0 and is preceded at the N-terminal end by a native amino acid sequence EASF (GRAVY = 0.075) then extension of the peptide to include EASF would lower the GRAVY score. Alternatively, one or more substitutions to said EASF lead sequence such as A with S, S with N, or F with Y (e.g., EASY, GRAVY = -0.95) or truncation and substitution (e.g., NY, GRAVY = -2.4) could also provide increased hydrophilicity.

In some cases it may be preferable to reduce the aqueous solubility of a peptide, for example to improve entrapment within a hydrophobic carrier matrix. In such cases, additions and substitutions similar to those described above, but reducing hydrophilicity, might be made.

It may further be advantageous to adjust net peptide charge at one or more pH values. For example, minimum solubility may be observed at the pl (isoelectric pH) of a peptide. In the case of where it would be desirable to have reduced solubility pH 7.4 and increased solubility at pH 3.0, then modifications or additions to the amino acid sequence could be made to achieve a pl of 7.4 and to achieve a significant net-positive charge at pH 3.0. In the case of a basic peptide, addition of acidic residues such as E or D or the substitution of a K with an E are example modifications that could reduce the pl.

The biological or chemical stability of a peptide may also be improved by the addition or substitution of amino acid or end-modification groups using techniques known in the art. Examples include, but are not limited to, amidation and acetylation, and may also include substitutions such as replacing a C-terminal Q (Gln) with an L or other amino acid less susceptible to rearrangement.

In embodiments, the invention is directed to compositions comprising A-x-B, wherein x comprises a cathepsin cleavage site comprising KVSVR and wherein A-x-B comprises an amino acid sequence that has at least 75% identity to any one of the amino acid sequences set forth as SEQ ID NOs: 12, 14, 17, 19, and 39-44, and preferably the polypeptide binding an MHC II molecule as described elsewhere herein. Also disclosed herein are the following polypeptides:
NNFTVSFWLRVPKVSASHLET (SEQ ID NO:1) (21, TT317557(950-969));
TLLYVLFEV (SEQ ID NO:2) (9, AdVhex64950(913-921));
ILMQYIKANSKFIGI (SEQ ID NO:3) (15, TT27213(830-841));
QSIALSSLMVAQAIPLVGEL (SEQ ID NO:4) (20, DT 52336(331-350));
TLLYVLFEVNNFTVSFWLRVPKVSASHLET (SEQ ID NO:5) (30, AdVTT950);
TLLYVLFEVILMQYIKANSKFIGI (SEQ ID NO:6) (24, AdVTT830);
ILMQYIKANSKFIGIQSIALSSLMVAQAIPLVGEL (SEQ ID NO:7) (35, TT830DT);
QSIALSSLMVAQAIPLVGELILMQYIKANSKFIGI (SEQ ID NO:8) (35, DTTT830);
ILMQYIKANSKFIGIQSIALSSLMVAQ (SEQ ID NO:9) (27, TT830DTtrunc);
QSIALSSLMVAQAIILMQYIKANSKFIGI (SEQ ID NO:10) (29, DTtruncTT830);
TLLYVLFEVPMGLPILMQYIKANSKFIGI (SEQ ID NO:11) (29, AdVpmglpiTT830);
TLLYVLFEVKVSVRILMQYIKANSKFIGI (SEQ ID NO:12) (29, AdVkvsvrTT830);
ILMQYIKANSKFIGIPMGLPQSIALSSLMVAQ (SEQ ID NO:13) (32, TT830pmglpDTTrunc or TT830pDTt);
ILMQYIKANSKFIGIKVSVRQSIALSSLMVAQ (SEQ ID NO:14) (32, TT830kvsvrDTTrunc1);
TLLYVLFEVQSIALSSLMVAQ (SEQ ID NO:15) (21, AdVDTt);
TLLYVLFEVpmglpQSIALSSLMVAQ (SEQ ID NO:16) (26, AdVpDTt);
TLLYVLFEVkvsvrQSIALSSLMVAQ (SEQ ID NO:17) (26, AdVkDTt);
TLLYVLFEVpmglp NNFTVSFWLRVPKVSASHLET (SEQ ID NO:18) (35, AdVpTT950);
TLLYVLFEVkvsvr NNFTVSFWLRVPKVSASHLET (SEQ ID NO:19) (35, AdVkTT950);
ILMQYIKANSKFIGI QSIALSSLMVAQTLLYVLFEV (SEQ ID NO:20) (36, TT830DTtAdV);
TLLYVLFEV ILMQYIKANSKFIGIQSIALSSLMVAQ (SEQ ID NO:21) (36, AdVTT830DTt);
QSIALSSLMVAQAIPLV (SEQ ID NO:22) (17, DTt-3);
IDKISDVSTIVPYIGPALNI (SEQ ID NO:23) (20, TT632)
QSIALSSLMVAQAIPLVIDKISDVSTIVPYIGPALNI (SEQ ID NO:24) (37, DTt-3TT632);
IDKISDVSTIVPYIGPALNIQSIALSSLMVAQAIPLV (SEQ ID NO:25) (37, TT632DTt-3);
QSIALSSLMVAQAIPLVpmglplDKISDVSTIVPYIGPALNI (SEQ ID NO:26) (43, DTt-3pTT632);
IDKISDVSTIVPYIGPALNIpmglpQSIALSSLMVAQAIPLV (SEQ ID NO:27) (43, TT632pDTt-3);
YVKQNTLKLAT (SEQ ID NO:28) (11, minX);
CYPYDVPDYASLRSLVASS (SEQ ID NO:29) (19, 7430);
NAELLVALENQHTI (SEQ ID NO:30) (14, 31201t);
TSLYVRASGRVTVSTK (SEQ ID NO:31) (16, 66325);
EKIVLLFAIVSLVKSDQICI (SEQ ID NO:32) (20, ABW1);
QILSIYSTVASSLALAIMVA (SEQ ID NO:33) (20, ABW2);
MVTGIVSLMLQIGNMISIWVSHSI (SEQ ID NO:34) (24, ABP);
EDLIFLARSALILRGSV (SEQ ID NO:35) (17, AAT);
CSQRSKFLLMDALKLSIED (SEQ ID NO:36) (19, AAW);
IRGFVYFVETLARSICE (SEQ ID NO:37) (14, IRG);
TFEFTSFFYRYGFVANFSMEL (SEQ ID NO:38) (21, TFE);
LIFLARSALILRkvsvrNAELLVALENQHTI (SEQ ID NO:39) (31, AATk3120t);
NAELLVALENQHTIkvsvrLIFLARSALILR (SEQ ID NO:40) (31, 3120tkAAT);
ILSIYSTVASSLALAIkvsvrLIFLARSALILR (SEQ ID NO:41) (33, ABW2kAAT);
UFLARSAULRkvsvrlLSIYSTVASSLALAI (SEQ ID NO:42) (33, AATkABW2);
LIFLARSALILRkvsvrCSQRSKFLLMDALKL (SEQ ID NO:43) (32, AATkAAW);
CSQRSKFLLMDALKLkvsvrLIFLARSALILR (SEQ ID NO:44) (32, AAWkAAT);
TFEFTSFFYRYGFVANFSMEL IRGFVYFVETLARSICE (SEQ ID NO:45) (38, TFEIRG) (SEQ ID NO:103); or
IRGFVYFVETLARSICE TFEFTSFFYRYGFVANFSMEL (SEQ ID NO:46) (38, IRGTFE) (SEQ ID NO:104).

Peptides for use in invention, particularly MHC II binding peptides, may be made using a variety of conventional techniques. In certain embodiments, the peptides can be made synthetically using standard methods such as synthesis on a solid support using Merrifield's or similar resins. This can be accomplished with or without a machine designed for such syntheses.

In alternative embodiments, in order to express peptides for use in the invention especially MHC II binding peptides, recombinant techniques may be used. In such embodiments, a nucleic acidencoding the entire peptide sequence (and linker sequence, if applicable) would be cloned into an expression vector that would be transcribed when transfected into a cell line. In embodiments, an expression vector may comprise a plasmid, retrovirus, or an adenovirus amongst others. The DNA for the peptide (and linking group, if present) can be isolated using standard molecular biology approaches, for example by using a polymerase chain reaction to produce the DNA fragment, which is then purified and cloned into an expression vector and transfected into a cell line. Additional techniques useful in the practice of this invention may be found in Current Protocols in Molecular Biology 2007 by John Wiley and Sons, Inc.; Molecular Cloning: A Laboratory Manual (Third Edition) Joseph Sambrook, Peter MacCallum Cancer Institute, Melbourne, Australia; David Russell, University of Texas Southwestern Medical Center, Dallas, Cold Spring Harbor.

Production of the recombinant peptides may be done in several ways using cells from different organisms, for example CHO cells, insect cells (e.g., for baculovirus expression), *E. coli* etc. Additionally, in order to get optimal protein translation the nucleic acid sequence can be modified to include codons that are commonly used in the organism from which the cells are derived. SEQ ID NOs 1-46 include examples of sequences obtained or derived from tetanus toxoid, diphtheria toxin, and adenovirus peptides, and SEQ ID NOs 47-68 include equivalent DNA sequence based on the preferred codon usage for humans and E. *coli.* Using DNA that is optimized for codon usage in a specific species may allow optimal recombinant protein production. Codon frequencies can be optimized for use in humans using frequency data such as that available from various codon usage records. One such record is the Codon Usage Database. Y. Nakamura et al., "Codon usage tabulated from the international DNA sequence databases: status for the year 2000." Nucl. Acids Res. 28, 292 (2000).

In embodiments, the inventive compositions comprise a nucleic acid that encodes a peptide comprising A-x-B wherein x comprises a cathepsin cleavage site. The nucleic acid may be DNA or RNA, such as mRNA. In embodiments, the inventive compositions comprise a complement, such as a full-length complement, or a degenerate (due to degeneracy of the genetic code) of any of the nucleic acids provided herein.

A comprises a first MHC II binding peptide, and B comprises a second MHC II binding peptide. Additionally, in embodiments, the nucleic acid encodes A - x - B - y - C, wherein x comprises a cathepsin cleavage site comprising KVSVR, yis an amide linker or a peptide linker, A comprises a first MHC II binding peptide, B comprises a second MHC II binding peptide, and C comprises a third MHC II binding peptide.

Certain sequences of interest are listed below. The native sequence is composition 1, (C1). The best human sequence based on the frequency of human codon use is composition 2, (C2). The conversions were performed using The Sequence Manipulation Suite: JavaScript programs for analyzing and formatting protein and DNA sequences. Biotechniques 28:1102-1104. http://www.bioinformatics.org/sms2/rev_trans.html
TT950: NNFTVSFWLRVPKVSASHLET (SEQ ID NO:1)
C1: aataattttaccgttagcttttggttgagggttcctaaagtatctgctagtcatttagaa (SEQ ID NO:47) AF154828 250-309
C2(human):
aacaacttcaccgtgagcttctggctgagagtgcccaaggtgagcgccagccacctggagacc (SEQ ID NO:48)
AdV: TLLYVLFEV (SEQ ID NO:2)
C1: acgcttctctatgttctgttcgaagt (SEQ ID NO:49)
FJ025931 20891-20917
C2(human):
accctgctgtacgtgctgttcgaggtg (SEQ ID NO:50)
TT830: ILMQYIKANSKFIGI (SEQ ID NO:3)
C1: attttaatgcagtatataaaagcaaattctaaatttataggtata (SEQ ID NO:51)
X06214 2800-2844
C2(human):
Atcctgatgcagtacatcaaggccaacagcaagttcatcggcatc (SEQ ID NO:52)
DT: QSIALSSLMVAQAIPLVGEL (SEQ ID NO:4)
C1: caatcgatagctttatcgtctttaatggttgctcaagctataccattggtaggagagcta (SEQ ID NO:53)
FJ858272 1066-1125
C2(human):
cagagcatcgccctgagcagcctgatggtggcccaggccatccccctggtgggcgagctg (SEQ ID NO:54)
Chimeric epitopes:
**AdVTT950**: TLLYVLFEVNNFTVSFWLRVPKVSASHLET (SEQ ID NO:5)
C2(human):
**AdVTT830**: TLLYVLFEVILMQYIKANSKFIGI (SEQ ID NO:6)
C2(human):
**TT830 DT**: ILMQYIKANSKFIGIQSIALSSLMVAQAIPLVGEL (SEQ ID NO:7)
C2(human): **DT TT830**: QSIALSSLMVAQAIPLVGELILMQYIKANSKFIGI (SEQ ID NO:8) C2(human): **TT830DTtrunc**: ILMQYIKANSKFIGIQSIALSSLMVAQ (SEQ ID NO:9) C2(human):
**DT trunc TT830**: QSIALSSLMVAQAIILMQYIKANSKFIGI (SEQ ID NO:10)
C2(human):

### Predicted chimeric cathepsin cleaved universal epitopes

**AdVpmglpTT830:** TLLYVLFEVPMG.LPILMQYIKANSKFIGI (SEQ ID NO:11)
C1 (Ecoli): C2(human):
**AdVkvsvrTT830**: TLLYVLFEVKVS.VRILMQYIKANSKFIGI (SEQ ID NO:12)
C1 (Ecoli): C2(human):
**TT830pmglpDTtrunc:** ILMQYIKANSKFIGIPMG.LPQSIALSSLMVAQ (SEQ ID NO:13)
C1 (Ecoli): C2(human):
**TT830kvsvrDTtrunc:** ILMQYIKANSKFIGIKVS.VRQSIALSSLMVAQ (SEQ ID NO:14)
C1 (Ecoli): C2(human):

In embodiments, the peptide linker comprises a lysosome protease cleavage site (e.g., a cathepsin cleavage site). In certain embodiments, the nucleic acid sequence that encodes a peptide linker comprises the nucleic acid sequence set forth as SEQ ID NO:69 or 70, a degenerate or a complement thereof.
ccgatgggcctacca (SEQ ID NO:69)
aaggtctcagtgagaac (SEQ ID NO:70)

In embodiments, A, B and/or C that are encoded by an inventive nucleic acid have at least 70% identity to a natural HLA-DP, HLA-DQ, or HLA-DR binding peptide. A, B and/or C encoded by a nucleic acid has, in certain embodiments, preferably at least 75%, more preferably at least 80%, still more preferably at least 85%, still more preferably at least 90%, still more preferably at least 95%, still more preferably at least 97%, or still even more preferably at least 99% identity to a natural HLA-DP, HLA-DQ, or HLA-DR binding peptide. Preferably, such nucleic acids encode a peptide that bind an MHC Class II molecule.

In embodiments, a nucleic acid, therefore, comprises a nucleic acid sequence that has at least 60% identity to a nucleic acid sequence that encodes a natural HLA-DP, HLA-DQ, or HLA-DR binding peptide. In certain embodiments, a nucleic acid has preferably at least 65%, more preferably at least 70%, still more preferably at least 75%, still more preferably at least 80%, still more preferably at least 85%, still more preferably at least 90%, still more preferably at least 95%, still more preferably at least 97%, or still even more preferably at least 99% identity to a nucleic acid sequence that encodes a natural HLA-DP, HLA-DQ, or HLA-DR binding peptide.

The percent identity can be calculated using various, publicly available software tools developed by NCBI (Bethesda, Maryland) that can be obtained through the internet (ftp:/ncbi.nlm.nih.gov/pub/). Exemplary tools include the BLAST system available at http://wwww.ncbi.nlm.nih.gov. Pairwise and ClustalW alignments (BLOSUM30 matrix setting) as well as Kyte-Doolittle hydropathic analysis can be obtained using the MacVector sequence analysis software (Oxford Molecular Group). Watson-Crick complements (including full-length complements) of the foregoing nucleic acids also are embraced by the invention.

Also disclosed herein are nucleic acids that hybridize to any of the nucleic acids provided herein. Standard nucleic acid hybridization procedures can be used to identify related nucleic acid sequences of selected percent identity. The term "stringent conditions" as used herein refers to parameters with which the art is familiar. Such parameters include salt, temperature, length of the probe, etc. The amount of resulting base mismatch upon hybridization can range from near 0% ("high stringency") to about 30% ("low stringency"). One example of high-stringency conditions is hybridization at 65°C in hybridization buffer (3.5X SSC, 0.02% Ficoll, 0.02% polyvinyl pyrrolidone, 0.02% Bovine Serum Albumin, 2.5mM NaH2PO4(pH7), 0.5% SDS, 2mM EDTA). SSC is 0.15M sodium chloride/0.015M sodium citrate, pH7; SDS is sodium dodecyl sulphate; and EDTA is ethylenediaminetetracetic acid. After hybridization, a membrane upon which the nucleic acid is transferred is washed, for example, in 2X SSC at room temperature and then at 0.1 - 0.5X SSC/0.1X SDS at temperatures up to 68°C.

In embodiments, the nucleic acid can be operably joined to a promoter. Expression in prokaryotic hosts can be accomplished using prokaryotic regulatory regions. Expression in eukaryotic hosts can be accomplished using eukaryotic regulatory regions. Such regions will, in general, include a promoter region sufficient to direct the initiation of RNA synthesis. In embodiments, the nucleic acid can further comprise transcriptional and translational regulatory sequences, depending upon the nature of the host. The transcriptional and translational regulatory signals may be obtained or derived from viral sources, such as a retrovirus, adenovirus, bovine papilloma virus, simian virus, or the like.

In embodiments, a nucleic acid is inserted into a vector capable of integrating the desired sequences into the host cell chromosome. Additional elements may also be needed for optimal synthesis of the mRNA. These elements may include splice signals, as well as transcription promoters, enhancers, and termination signals.

In embodiments, a nucleic acid is incorporated into a plasmid or viral vector capable of autonomous replication in the recipient host. Any of a wide variety of vectors may be employed for this purpose, such a prokaryotic and eukaryotic vectors. The eukaryotic vectors can be viral vectors. For example, and not by way of limitation, the vector can be a pox virus vector, herpes virus vector, adenovirus vector or any of a number of retrovirus vectors. The viral vectors include either DNA or RNA viruses to cause expression of the insert DNA or insert RNA.

The vector or other construct can be introduced into an appropriate host cell by any of a variety of suitable means, i.e., transformation, transfection, conjugation, protoplast fusion, electroporation, calcium phosphate-precipitation, direct microinjection, and the like. Additionally, DNA or RNA can be directly injected into cells or may be impelled through cell membranes after being adhered to microparticles or nanoparticles, such as the synthetic nanocarriers provided herein.

### D. USES OF THE INVENTIVE PEPTIDE: COMPOSITIONS AND METHODS

It is to be understood that the compositions of the invention can be made in any suitable manner, and the invention is in no way limited to compositions that can be produced using the methods described herein. Selection of an appropriate method may require attention to the properties of the particular moieties being associated.

The inventive compositions may be administered by a variety of routes of administration, including but not limited to parenteral (such as subcutaneous, intramuscular, intravenous, or intradermal); oral; transnasal, transmucosal, rectal; ophthalmic, or transdermal.

The compositions and methods described herein can be used to induce, enhance, suppress, direct, or redirect an immune response. The compositions and methods described herein can be used for the prophylaxis and/or treatment of conditions such as cancers, infectious diseases, metabolic diseases, degenerative diseases, autoimmune diseases, inflammatory diseases, immunological diseases, or other disorders and/or conditions. The compositions and methods described herein can also be used for the treatment of an addiction, such as an addiction to nicotine or a narcotic. The compositions and methods described herein can also be used for the prophylaxis and/or treatment of a condition resulting from the exposure to a toxin, hazardous substance, environmental toxin, or other harmful agent. The compositions and methods described herein can also be used to induce or enhance T-cell proliferation or cytokine production, for example, when the compositions provided herein are put in contact with T-cells *in vivo* or *in vitro.*

In an embodiment, the inventive compositions may be administered together with conjugate, or non-conjugate, vaccines. In embodiments, the inventive compositions may be bound covalently or non-covalently to a carrier peptide or protein, or to one or more antigens. Useful carriers comprises carrier proteins known to be useful in conjugate vaccines, including but not limited to tetanus toxoid (TT), diphtheria toxoid (DT), the nontoxic mutant of diphtheria toxin, CRM197, the outer membrane protein complex from group B N. meningitidis, and keyhole limpet hemocyanin (KLH). Other carriers can comprise the synthethic nanocarriers described elsewhere herein, and other carriers that might be known conventionally.

Conjugation may be performed using conventional covalent or non-covalent conjugation techniques. Useful techniques for utilizing the inventive compositions in such conjugated or conventional vaccines include but are not limited to those generally described in MD Lairmore et al., "Human T-lymphotropic virus type 1 peptides in chimeric and multivalent constructs with promiscuous T-cell epitopes enhance immunogenicity and overcome genetic restriction." J Virol. Oct;69(10):6077-89 (1995); CW Rittershause et al., "Vaccine-induced antibodies inhibit CETP activity in vivo and reduce aortic lesions in a rabbit model of atherosclerosis." Arterioscler Thromb Vasc Biol. Sep;20(9):2106-12 (2000); MV Chengalvala et al., "Enhanced immunogenicity of hepatitis B surface antigen by insertion of a helper T cell epitope from tetanus toxoid." Vaccine. Mar 5; 17(9-10):1035-41 (1999). NK Dakappagari et al., "A chimeric multi-human epidermal growth factor receptor-2 B cell epitope peptide vaccine mediates superior antitumor responses." J Immunol. Apr 15;170(8):4242-53 (2003); JT Garrett et al. "Novel engineered trastuzumab conformational epitopes demonstrate in vitro and in vivo antitumor properties against HER-2/neu." J Immunol. Jun 1;178(11):7120-31 (2007).

In other embodiments, the inventive compositions may be combined with antigen, or a conventional vaccine, in a vehicle to form an injectable mixture. The mixtures may be made using conventional pharmaceutical manufacturing and compounding techniques to arrive at useful dosage forms. Techniques suitable for use in practicing the present invention may be found in a variety of sources, including but not limited to M.F. Powell et al., Vaccine Design, 1995 Springer-Verlag publ.; or L. C. Paoletti et al. eds., Vaccines: from Concept to Clinic. A Guide to the Development and Clinical Testing of Vaccines for Human Use 1999 CRC Press publ.

In embodiments, the inventive compositions may be used in combination with synthetic nanocarriers. A wide variety of synthetic nanocarriers can be used according to the invention. In some embodiments, synthetic nanocarriers are spheres or spheroids. In some embodiments, synthetic nanocarriers are flat or plate-shaped. In some embodiments, synthetic nanocarriers are cubes or cuboidal. In some embodiments, synthetic nanocarriers are ovals or ellipses. In some embodiments, synthetic nanocarriers are cylinders, cones, or pyramids.

It is often desirable to use a population of synthetic nanocarriers that is relatively uniform in terms of size, shape, and/or composition so that each synthetic nanocarrier has similar properties. For example, at least 80%, at least 90%, or at least 95% of the synthetic nanocarriers, based on the total number of synthetic nanocarriers, may have a minimum dimension or maximum dimension that falls within 5%, 10%, or 20% of the average diameter or average dimension of the synthetic nanocarriers. In some embodiments, a population of synthetic nanocarriers may be heterogeneous with respect to size, shape, and/or composition.

Synthetic nanocarriers can be solid or hollow and can comprise one or more layers. In some embodiments, each layer has a unique composition and unique properties relative to the other layer(s). To give but one example, synthetic nanocarriers may have a core/shell structure, wherein the core is one layer (e.g. a polymeric core) and the shell is a second layer (e.g. a lipid bilayer or monolayer). Synthetic nanocarriers may comprise a plurality of different layers.

In some embodiments, synthetic nanocarriers may optionally comprise one or more lipids. In some embodiments, a synthetic nanocarrier may comprise a liposome. In some embodiments, a synthetic nanocarrier may comprise a lipid bilayer. In some embodiments, a synthetic nanocarrier may comprise a lipid monolayer. In some embodiments, a synthetic nanocarrier may comprise a micelle. In some embodiments, a synthetic nanocarrier may comprise a core comprising a polymeric matrix surrounded by a lipid layer (e.g., lipid bilayer, lipid monolayer, etc.). In some embodiments, a synthetic nanocarrier may comprise a non-polymeric core (e.g., metal particle, quantum dot, ceramic particle, bone particle, viral particle, proteins, nucleic acids, carbohydrates, etc.) surrounded by a lipid layer (e.g., lipid bilayer, lipid monolayer, etc.).

In some embodiments, synthetic nanocarriers can comprise one or more polymers. In some embodiments, such a polymer can be surrounded by a coating layer (e.g., liposome, lipid monolayer, micelle, etc.). In some embodiments, various elements of the synthetic nanocarriers can be coupled with the polymer.

In some embodiments, an immunofeature surface, targeting moiety, oligonucleotide and/or other element can be covalently associated with a polymeric matrix. In some embodiments, covalent association is mediated by a linker. In some embodiments, an immunofeature surface, targeting moiety, oligonucleotide and/or other element can be noncovalently associated with a polymeric matrix. For example, in some embodiments, an immunofeature surface, targeting moiety, oligonucleotide and/or other element can be encapsulated within, surrounded by, and/or dispersed throughout a polymeric matrix. Alternatively or additionally, an immunofeature surface, targeting moiety, oligonucleotide and/or other element can be associated with a polymeric matrix by hydrophobic interactions, charge interactions, van der Waals forces, etc.

A wide variety of polymers and methods for forming polymeric matrices therefrom are known conventionally. In general, a polymeric matrix comprises one or more polymers. Polymers may be natural or unnatural (synthetic) polymers. Polymers may be homopolymers or copolymers comprising two or more monomers. In terms of sequence, copolymers may be random, block, or comprise a combination of random and block sequences. Typically, polymers in accordance with the present invention are organic polymers.

Examples of polymers suitable for use in the present invention include, but are not limited to polyethylenes, polycarbonates (e.g. poly(1,3-dioxan-2one)), polyanhydrides (e.g. poly(sebacic anhydride)), polypropylfumerates, polyamides (e.g. polycaprolactam), polyacetals, polyethers, polyesters (e.g., polylactide, polyglycolide, polylactide-co-glycolide, polycaprolactone, polyhydroxyacid (e.g. poly(β-hydroxyalkanoate))), poly(orthoesters), polycyanoacrylates, polyvinyl alcohols, polyurethanes, polyphosphazenes, polyacrylates, polymethacrylates, polyureas, polystyrenes, and polyamines, polylysine, polylysine-PEG copolymers, and poly(ethyleneimine), poly(ethylene imine)-PEG copolymers.

In some embodiments, polymers in accordance with the present invention include polymers which have been approved for use in humans by the U.S. Food and Drug Administration (FDA) under 21 C.F.R. § 177.2600, including but not limited to polyesters (e.g., polylactic acid, poly(lactic-co-glycolic acid), polycaprolactone, polyvalerolactone, poly(1,3-dioxan-2one)); polyanhydrides (e.g., poly(sebacic anhydride)); polyethers (e.g., polyethylene glycol); polyurethanes; polymethacrylates; polyacrylates; and polycyanoacrylates.

In some embodiments, polymers can be hydrophilic. For example, polymers may comprise anionic groups (e.g., phosphate group, sulphate group, carboxylate group); cationic groups (e.g., quaternary amine group); or polar groups (e.g., hydroxyl group, thiol group, amine group). In some embodiments, a synthetic nanocarrier comprising a hydrophilic polymeric matrix generates a hydrophilic environment within the synthetic nanocarrier. In some embodiments, polymers can be hydrophobic. In some embodiments, a synthetic nanocarrier comprising a hydrophobic polymeric matrix generates a hydrophobic environment within the synthetic nanocarrier. Selection of the hydrophilicity or hydrophobicity of the polymer may have an impact on the nature of materials that are incorporated (e.g. coupled) within the synthetic nanocarrier.

In some embodiments, polymers may be modified with one or more moieties and/or functional groups. A variety of moieties or functional groups can be used in accordance with the present invention. In some embodiments, polymers may be modified with polyethylene glycol (PEG), with a carbohydrate, and/or with acyclic polyacetals derived from polysaccharides (Papisov, 2001, ACS Symposium Series, 786:301). Certain embodiments may be made using the general teachings of US Patent No. 5543158 to Gref et al., or WO publication WO2009/051837 by Von Andrian et al.

In some embodiments, polymers may be modified with a lipid or fatty acid group. In some embodiments, a fatty acid group may be one or more of butyric, caproic, caprylic, capric, lauric, myristic, palmitic, stearic, arachidic, behenic, or lignoceric acid. In some embodiments, a fatty acid group may be one or more of palmitoleic, oleic, vaccenic, linoleic, alpha-linoleic, gamma-linoleic, arachidonic, gadoleic, arachidonic, eicosapentaenoic, docosahexaenoic, or erucic acid.

In some embodiments, polymers may be polyesters, including copolymers comprising lactic acid and glycolic acid units, such as poly(lactic acid-co-glycolic acid) and poly(lactide-co-glycolide), collectively referred to herein as "PLGA"; and homopolymers comprising glycolic acid units, referred to herein as "PGA," and lactic acid units, such as poly-L-lactic acid, pofy-D-lactic acid, poly-D,L-lactic acid, poly-L-lactide, poly-D-lactide, and poly-D,L-lactide, collectively referred to herein as "PLA." In some embodiments, exemplary polyesters include, for example, polyhydroxyacids; PEG copolymers and copolymers of lactide and glycolide (e.g., PLA-PEG copolymers, PGA-PEG copolymers, PLGA-PEG copolymers, and derivatives thereof. In some embodiments, polyesters include, for example, poly(caprolactone), poly(caprolactone)-PEG copolymers, poly(L-lactide-co-L-lysine), poly(serine ester), poly(4-hydroxy-L-proline ester), poly[α-(4-aminobutyl)-L-glycolic acid], and derivatives thereof.

In some embodiments, a polymer may be PLGA. PLGA is a biocompatible and biodegradable co-polymer of lactic acid and glycolic acid, and various forms of PLGA are characterized by the ratio of lactic acid:glycolic acid. Lactic acid can be L-lactic acid, D-lactic acid, or D, L-lactic acid. The degradation rate of PLGA can be adjusted by altering the lactic acid:glycolic acid ratio. In some embodiments, PLGA to be used in accordance with the present invention is characterized by a lactic acid:glycolic acid ratio of approximately 85:15, approximately 75:25, approximately 60:40, approximately 50:50, approximately 40:60, approximately 25:75, or approximately 15:85.

In some embodiments, polymers may be one or more acrylic polymers. In certain embodiments, acrylic polymers include, for example, acrylic acid and methacrylic acid copolymers, methyl methacrylate copolymers, ethoxyethyl methacrylates, cyanoethyl methacrylate, aminoalkyl methacrylate copolymer, poly(acrylic acid), poly(methacrylic acid), methacrylic acid alkylamide copolymer, poly(methyl methacrylate), poly(methacrylic acid anhydride), methyl methacrylate, polymethacrylate, poly(methyl methacrylate) copolymer, polyacrylamide, aminoalkyl methacrylate copolymer, glycidyl methacrylate copolymers, polycyanoacrylates, and combinations comprising one or more of the foregoing polymers. The acrylic polymer may comprise fully-polymerized copolymers of acrylic and methacrylic acid esters with a low content of quaternary ammonium groups.

In some embodiments, polymers can be cationic polymers. In general, cationic polymers are able to condense and/or protect negatively charged strands of nucleic acids (e.g. DNA, or derivatives thereof). Amine-containing polymers such as poly(lysine) (Zauner et al., 1998, Adv. Drug Del. Rev., 30:97; and Kabanov et al., 1995, Bioconjugate Chem., 6:7), poly(ethylene imine) (PEI; Boussif et al., 1995, Proc. Natl. Acad. Sci., USA, 1995, 92:7297), and poly(amidoamine) dendrimers (Kukowska-Latallo et al., 1996, Proc. Natl. Acad. Sci., USA, 93:4897; Tang et al., 1996, Bioconjugate Chem., 7:703; and Haensler et al., 1993, Bioconjugate Chem., 4:372) are positively-charged at physiological pH, form ion pairs with nucleic acids, and mediate transfection in a variety of cell lines. In embodiments, the inventive synthetic nanocarriers may not comprise (or may exclude) cationic polymers.

In some embodiments, polymers can be degradable polyesters bearing cationic side chains (Putnam et al., 1999, Macromolecules, 32:3658; Barrera et al., 1993, J. Am. Chem. Soc., 115:11010; Kwon et al., 1989, Macromolecules, 22:3250; Lim et al., 1999, J. Am. Chem. Soc., 121:5633; and Zhou et al., 1990, Macromolecules, 23:3399). Examples of these polyesters include poly(L-lactide-co-L-lysine) (Barrera et al., 1993, J. Am. Chem. Soc., 115:11010), poly(serine ester) (Zhou et al., 1990, Macromolecules, 23:3399), poly(4-hydroxy-L-proline ester) (Putnam et al., 1999, Macromolecules, 32:3658; and Lim et al., 1999, J. Am. Chem. Soc., 121:5633), and poly(4-hydroxy-L-proline ester) (Putnam et al., 1999, Macromolecules, 32:3658; and Lim et al., 1999, J. Am. Chem. Soc., 121:5633).

The properties of these and other polymers and methods for preparing them are well known in the art (see, for example, U.S. Patents 6,123,727; 5,804,178; 5, 770, 417; 5, 736, 372; 5, 716, 404; 6,095,148; 5, 837, 752; 5, 902, 599; 5,696,175; 5,514,378; 5,512,600; 5,399,665; 5,019,379; 5,010,167; 4,806,621; 4,638,045; and 4,946,929; Wang et al., 2001, J. Am. Chem. Soc., 123:9480; Lim et al., 2001, J. Am. Chem. Soc., 123:2460; Langer, 2000, Acc. Chem. Res., 33:94; Langer, 1999, J. Control. Release, 62:7; and Uhrich et al., 1999, Chem. Rev., 99:3181). More generally, a variety of methods for synthesizing certain suitable polymers are described in Concise Encyclopedia of Polymer Science and Polymeric Amines and Ammonium Salts, Ed. by Goethals, Pergamon Press, 1980; Principles of Polymerization by Odian, John Wiley & Sons, Fourth Edition, 2004; Contemporary Polymer Chemistry by Allcock et al., Prentice-Hall, 1981; Deming et al., 1997, Nature, 390:386; and in U.S. Patents 6,506,577, 6,632,922, 6,686,446, and 6,818,732.

In some embodiments, polymers can be linear or branched polymers. In some embodiments, polymers can be dendrimers. In some embodiments, polymers can be substantially cross-linked to one another. In some embodiments, polymers can be substantially free of cross-links. In some embodiments, polymers can be used in accordance with the present invention without undergoing a cross-linking step. It is further to be understood that inventive synthetic nanocarriers may comprise block copolymers, graft copolymers, blends, mixtures, and/or adducts of any of the foregoing and other polymers. Those skilled in the art will recognize that the polymers listed herein represent an exemplary, not comprehensive, list of polymers that can be of use in accordance with the present invention.

In some embodiments, synthetic nanocarriers may not comprise a polymeric component. In some embodiments, synthetic nanocarriers may comprise metal particles, quantum dots, ceramic particles, etc. In some embodiments, a non-polymeric synthetic nanocarrier is an aggregate of non-polymeric components, such as an aggregate of metal atoms (e.g., gold atoms).

In some embodiments, synthetic nanocarriers may optionally comprise one or more amphiphilic entities. In some embodiments, an amphiphilic entity can promote the production of synthetic nanocarriers with increased stability, improved uniformity, or increased viscosity. In some embodiments, amphiphilic entities can be associated with the interior surface of a lipid membrane (e.g., lipid bilayer, lipid monolayer, etc.). Many amphiphilic entities known in the art are suitable for use in making synthetic nanocarriers in accordance with the present invention. Such amphiphilic entities include, but are not limited to, phosphoglycerides; phosphatidylcholines; dipalmitoyl phosphatidylcholine (DPPC); dioleylphosphatidyl ethanolamine (DOPE); dioleyloxypropyltriethylammonium (DOTMA); dioleoylphosphatidylcholine; cholesterol; cholesterol ester; diacylglycerol; diacylglycerolsuccinate; diphosphatidyl glycerol (DPPG); hexanedecanol; fatty alcohols such as polyethylene glycol (PEG); polyoxyethylene-9-lauryl ether; a surface active fatty acid, such as palmitic acid or oleic acid; fatty acids; fatty acid monoglycerides; fatty acid diglycerides; fatty acid amides; sorbitan trioleate (Span®85) glycocholate; sorbitan monolaurate (Span®20); polysorbate 20 (Tween®20); polysorbate 60 (Tween®60): polysorbate 65 (Tween®65); polysorbate 80 (Tween®80); polysorbate 85 (Tween®85); polyoxyethylene monostearate; surfactin; a poloxomer; a sorbitan fatty acid ester such as sorbitan trioleate; lecithin; lysolecithin; phosphatidylserine; phosphatidylinositol;sphingomyelin; phosphatidylethanolamine (cephalin); cardiolipin; phosphatidic acid; cerebrosides; dicetylphosphate; dipalmitoylphosphatidylglycerol; stearylamine; dodecylamine; hexadecyl-amine; acetyl palmitate; glycerol ricinoleate; hexadecyl sterate; isopropyl myristate; tyloxapol; poly(ethylene glycol)5000-phosphatidylethanolamine; poly(ethylene glycol)400-monostearate; phospholipids; synthetic and/or natural detergents having high surfactant properties; deoxycholates; cyclodextrins; chaotropic salts; ion pairing agents; and combinations thereof. An amphiphilic entity component may be a mixture of different amphiphilic entities. Those skilled in the art will recognize that this is an exemplary, not comprehensive, list of substances with surfactant activity. Any amphiphilic entity may be used in the production of synthetic nanocarriers to be used in accordance with the present invention.

In some embodiments, synthetic nanocarriers may optionally comprise one or more carbohydrates. Carbohydrates may be natural or synthetic. A carbohydrate may be a derivatized natural carbohydrate. In certain embodiments, a carbohydrate comprises monosaccharide or disaccharide, including but not limited to glucose, fructose, galactose, ribose, lactose, sucrose, maltose, trehalose, cellbiose, mannose, xylose, arabinose, glucoronic acid, galactoronic acid, mannuronic acid, glucosamine, galatosamine, and neuramic acid. In certain embodiments, a carbohydrate is a polysaccharide, including but not limited to pullulan, cellulose, microcrystalline cellulose, hydroxypropyl methylcellulose (HPMC), hydroxycellulose (HC), methylcellulose (MC), dextran, cyclodextran, glycogen, hydroxyethylstarch, carageenan, glycon, amylose, chitosan, N,O-carboxylmethylchitosan, algin and alginic acid, starch, chitin, inulin, konjac, glucommannan, pustulan, heparin, hyaluronic acid, curdlan, and xanthan. In embodiments, the inventive synthetic nanocarriers do not comprise (or specifically exclude) carbohydrates, such as a polysaccharide. In certain embodiments, the carbohydrate may comprise a carbohydrate derivative such as a sugar alcohol, including but not limited to mannitol, sorbitol, xylitol, erythritol, maltitol, and lactitol.

Compositions according to the invention may comprise inventive synthetic nanocarriers or vaccine constructs in combination with pharmaceutically acceptable excipients. The compositions may be made using conventional pharmaceutical manufacturing and compounding techniques to arrive at useful dosage forms. In an embodiment, inventive synthetic nanocarriers are suspended in sterile saline solution for injection together with a preservative. In embodiments, typical inventive compositions may comprise excipients that comprise inorganic or organic buffers (e.g., sodium or potassium salts of phosphate, carbonate, acetate, or citrate) and pH adjustment agents (e.g., hydrochloric acid, sodium or potassium hydroxide, salts of citrate or acetate, amino acids and their salts) antioxidants (e.g., ascorbic acid, alpha-tocopherol), surfactants (e.g., polysorbate 20, polysorbate 80, polyoxyethylene9-10 nonyl phenol, sodium desoxycholate), solution and/or cryo/lyo stabilizers (e.g., sucrose, lactose, mannitol, trehalose), osmotic adjustment agents (e.g., salts or sugars), antibacterial agents (e.g., benzoic acid, phenol, gentamicin), antifoaming agents (e.g., polydimethylsilozone), preservatives (e.g., thimerosal, 2-phenoxyethanol, EDTA), polymeric stabilizers and viscosity-adjustment agents (e.g., polyvinylpyrrolidone, poloxamer 488, carboxymethylcellulose) and co-solvents (e.g., glycerol, polyethylene glycol, ethanol).

MHC II binding peptides may be encapsulated into synthetic nanocarriers using a variety of methods including but not limited to C. Astete et al., "Synthesis and characterization of PLGA nanoparticles" J. Biomater. Sci. Polymer Edn, Vol. 17, No. 3, pp. 247-289 (2006); K. Avgoustakis "Pegylated Poly(Lactide) and Poly(Lactide-Co-Glycolide) Nanoparticles: Preparation, Properties and Possible Applications in Drug Delivery" Current Drug Delivery 1:321-333 (2004); C. Reis et al., "Nanoencapsulation I. Methods for preparation of drug-loaded polymeric nanoparticles" Nanomedicine 2:8- 21 (2006). Other methods suitable for encapsulating materials such as peptides into synthetic nanocarriers may be used, including without limitation methods disclosed in United States Patent 6,632,671 to Unger October 14, 2003. In another embodiment, the MHC II binding peptides may be adsorbed to a surface of the synthetic nanocarriers as described generally in M. Singh et al., "Anionic microparticles are a potent delivery system for recombinant antigens from Neisseria meningitidis serotype B." J Pharm Sci. Feb;93(2):273-82 (2004).

In embodiments, compositions according to the invention may comprise antigens and/or adjuvants. In embodiments, vaccines according to the invention may comprise inventive compositions together with antigens and/or adjuvants. Different types of antigens and/or adjuvants useful in the practice of the invention are noted elsewhere herein. As noted elsewhere herein, the MHC II binding peptides of the inventive compositions may be covalently or non-covalently coupled to the antigens and/or adjuvants, or they may be admixed with the antigens and/or adjuvants. General techniques for coupling or admixing materials have been noted elsewhere herein; such techniques may be adapted to coupling or admixing the MHC II binding peptides of the inventive compositions to or with the antigens and/or adjuvants. For detailed descriptions of available covalent conjugation methods, see Hermanson G T "Bioconjugate Techniques", 2nd Edition Published by Academic Press, Inc., 2008. In addition to covalent attachment, coupling may be accomplished by adsorbtion to a pre-formed carrier, such as synthetic nanocarrier, or by encapsulation during the formation of carriers, such as a synthetic nanocarrier. In a preferred embodiment, the MHC II binding peptides of the inventive compositions are coupled to synthetic nanocarriers that are also coupled to antigens and/or adjuvants.

Synthetic nanocarriers may be prepared using a wide variety of methods known in the art. For example, synthetic nanocarriers can be formed by methods as nanoprecipitation, flow focusing using fluidic channels, spray drying, single and double emulsion solvent evaporation, solvent extraction, phase separation, milling, microemulsion procedures, microfabrication, nanofabrication, sacrificial layers, simple and complex coacervation, and other methods well known to those of ordinary skill in the art. Alternatively or additionally, aqueous and organic solvent syntheses for monodisperse semiconductor, conductive, magnetic, organic, and other nanomaterials have been described (Pellegrino et al., 2005, Small, 1:48; Murray et al., 2000, Ann. Rev. Mat. Sci., 30:545; and Trindade et al., 2001, Chem. Mat., 13:3843). Additional methods have been described in the literature (see, e.g., Doubrow, Ed., "Microcapsules and Nanoparticles in Medicine and Pharmacy," CRC Press, Boca Raton, 1992; Mathiowitz et al., 1987, J. Control. Release, 5:13; Mathiowitz et al., 1987, Reactive Polymers, 6:275; and Mathiowitz et al., 1988, J. Appl. Polymer Sci., 35:755, and also US Patents 5578325 and 6007845).

Various materials may be coupled through encapsulation into synthetic nanocarriers as desirable using a variety of methods including but not limited to C. Astete et al., "Synthesis and characterization of PLGA nanoparticles" J. Biomater. Sci. Polymer Edn, Vol. 17, No. 3, pp. 247-289 (2006); K. Avgoustakis "Pegylated Poly(Lactide) and Poly(Lactide-Co-Glycolide) Nanoparticles: Preparation, Properties and Possible Applications in Drug Delivery" Current Drug Delivery 1:321-333 (2004); C. Reis et al., "Nanoencapsulation I. Methods for preparation of drug-loaded polymeric nanoparticles" Nanomedicine 2:8- 21 (2006). Other methods suitable for encapsulating materials, such as oligonucleotides, into synthetic nanocarriers may be used, including without limitation methods disclosed in United States Patent 6,632,671 to Unger (October 14, 2003).

In certain embodiments, synthetic nanocarriers are prepared by a nanoprecipitation process or spray drying. Conditions used in preparing synthetic nanocarriers may be altered to yield particles of a desired size or property (e.g., hydrophobicity, hydrophilicity, external morphology, "stickiness," shape, etc.). The method of preparing the synthetic nanocarriers and the conditions (e.g., solvent, temperature, concentration, air flow rate, etc.) used may depend on the materials to be coupled to the synthetic nanocarriers and/or the composition of the polymer matrix.

If particles prepared by any of the above methods have a size range outside of the desired range, particles can be sized, for example, using a sieve.

It is to be understood that the compositions of the invention can be made in any suitable manner, and the invention is in no way limited to compositions that can be produced using the methods described herein. Selection of an appropriate method may require attention to the properties of the particular moieties being associated.

In some embodiments, inventive synthetic nanocarriers are manufactured under sterile conditions or are terminally sterilized. This can ensure that resulting composition are sterile and non-infectious, thus improving safety when compared to non-sterile compositions. This provides a valuable safety measure, especially when subjects receiving synthetic nanocarriers have immune defects, are suffering from infection, and/or are susceptible to infection. In some embodiments, inventive synthetic nanocarriers may be lyophilized and stored in suspension or as lyophilized powder depending on the formulation strategy for extended periods without losing activity.

The inventive compositions may be administered by a variety of routes of administration, including but not limited to parenteral (such as subcutaneous, intramuscular, intravenous, or intradermal); oral; transnasal, transmucosal, rectal; ophthalmic, or transdermal.

### E. EXAMPLES

The invention will be more readily understood by reference to the following examples, which are included merely for purposes of illustration of certain aspects and embodiments of the present invention and not as limitations.

Those skilled in the art will appreciate that various adaptations and modifications of the just-described embodiments can be configured without departing from the scope of the invention. Other suitable techniques and methods known in the art can be applied in numerous specific modalities by one skilled in the art and in light of the description of the present invention described herein.

Therefore, it is to be understood that the invention can be practiced other than as specifically described herein. The above description is intended to be illustrative, and not restrictive. Many other embodiments will be apparent to those of skill in the art upon reviewing the above description. The scope of the invention should, therefore, be determined with reference to the appended claims.

### Example 1: Generation of Universal Memory Peptides

In order to generate chimeric peptides, Class II epitope prediction was performed using the Immune Epitope Database (IEDB) (http://www.immuneepitope.org/) T cell epitope prediction tools. For each peptide, the prediction tool produces a percentile rank for each of three methods (ARB, SMM_align and Sturniolo). The ranking is generated by comparing the peptide's score against the scores of five million random 15 mers selected from SWISSPROT database. The median of the percentile ranks for the three methods is then used to generate the rank for consensus method. Peptides to be evaluated using the consensus method may be generated using sequences derived or obtained from various sources, including infectious organisms capable of infecting humans and generating human CD4+ memory cells specific to that infectious organism following the initiation of the infection. Examples of such infectious organisms have been noted elsewhere herein.

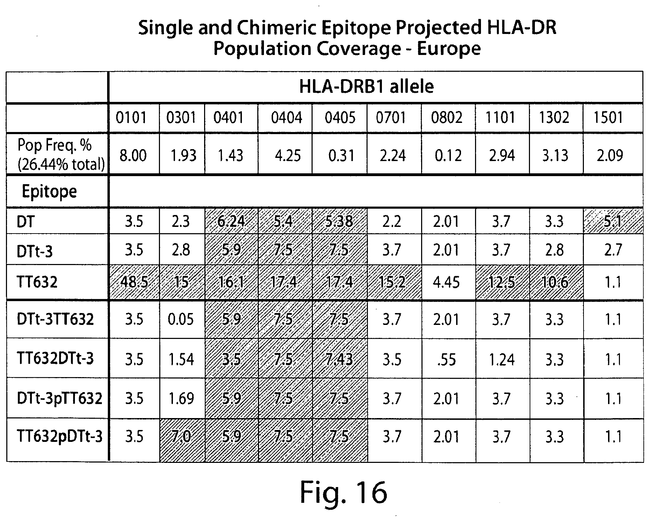

In a particular embodiment, individual protein and peptide epitopes were selected from tetanus toxin, diphtheria toxin or adenovirus, and were analyzed to in order to identify predicted HLA-DR and HLA-DP epitopes. For whole protein analysis, HLA-DR predicted epitopes were selected based on consensus ranking (predicted high affinity binders), and broad coverage across HLA-DR alleles. In addition, epitopes were selected that were predicted high affinity binders to HLA-DP0401 and DP0402. These 2 alleles for DP were selected because they are found in a high percentage of the population in North America (approximately 75%). Based on results from individual epitopes, in certain embodiments, chimeric peptides were generated that would give the predicted broadest coverage, and high affinity binding. See Figures 15-16. As shown in Figure 15, compositions can be generated having the form A-x-B that have broader predicted coverage and higher affinity binding than compositions having only A or B but not both.

In some cases cathepsin cleavage sites were inserted at the junction of the peptides. Chimeric peptides were synthesized (GenScript) and resuspended in water for use.

While the particular embodiment noted above was used to produce optimized compositions that comprised HLA-DR and HLA-DP binding peptides, the same techniques can be used to produce optimized compositions that comprise HLA-DQ binding peptides.

### Example 2: Core Amino Acid Sequence Evaluation

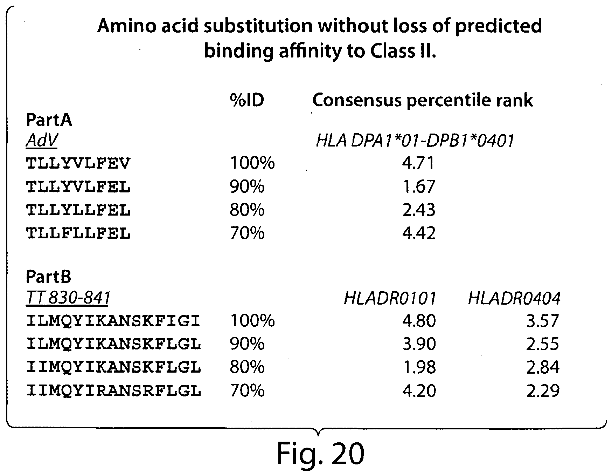

Both HLA-DP and HLA-DR specific epitopes have been evaluated for core binding epitopes by truncation analysis (1, 2). Core amino acid sequences selective for a specific HLA- class II protein have been found in common in several epitopes. An example of this are common core binding structures that have been identified which constitute a supertype of peptide binding specificity for HLA-DP4 (3). It is likely that these core amino acids maintain a structural configuration that allows high affinity binding. As a result it is possible to substitute non-core region amino acids with similar chemical properties without inhibiting the ability to bind to Class II (4). This can be shown experimentally using substitutional analysis and then epitope binding prediction programs. In order to perform the analysis individual amino acid substitutions were introduced, and the predicted affinity binding to Class II determined using the IEDB T-cell binding prediction tool (see Figure 20).

In this case two examples were shown, where in Part A, substitutions of up to 70% in an adenoviral epitope did not disrupt the affinity for binding to DP4. Part B illustrated substitutions of up to 70% in a tetanus toxoid epitope that did not inhibit its predicted binding to HLADR0101 or HLADR0404, which are representative of DR alleles. Accordingly, generation of a high affinity chimeric peptide with broad HLA coverage though modification of amino acid sequences did not disrupt the ability of the peptide to bind MHC II. In addition improved predicted affinity of the peptide may be achieved by substituting amino acids, as demonstrated in this Example.

While the particular embodiment noted above was used to exemplify optimized compositions that comprised HLA-DR or HLA-DP binding peptides, the same techniques can be used to produce optimized compositions that comprise HLA-DQ binding peptides.

### References:

1. Truncation analysis of several DR binding epitopes. O'Sullivan D, Sidney J, Del Guercio MF, Colón SM, Sette A. J Immunol. 1991 Feb 15;146(4):1240-6.
2. Adenovirus hexon T-cell epitope is recognized by most adults and is restricted by HLA DP4, the most common class II allele. Tang J, Olive M, Champagne K, Flomenberg N, Eisenlohr L, Hsu S, Flomenberg P. Gene Ther. 2004 Sep;11(18):1408-15.
3. HLA-DP4, the most frequent HLA II molecule, defines a new supertype of peptide-binding specificity.Castelli FA, Buhot C, Sanson A, Zarour H, Pouvelle- Moratille S, Nonn C, Gahery-Ségard H, Guillet JG, Ménez A, Georges B, Maillère B. J Immunol. 2002 Dec 15;169(12):6928-34.
4. Prediction of CD4(+) T cell epitopes restricted to HLA-DP4 molecules. Busson M, Castelli FA, Wang XF, Cohen WM, Charron D, Ménez A, Maillère B. J Immunol Methods. 2006 Dec 20;317(1-2):144-51

### Example 3: Peptide Evaluation

Inventive compositions comprising chimeric epitope peptides were evaluated for (1) potency of recall response; (2) the frequency of recall response against a random population sample population (N=20); and (3) the frequency of antigen-specific memory T-cells within individuals (N=20).

The potency of single epitopes and chimeric epitopes were evaluated by stimulating human PBMC with peptides in vitro for 24 hours and then analyzing the cells by flow cytometry. Activated CD4 central memory T-cells have the phenotype: CD4+ CD45RAlow CD62L+ IFN-γ +. To estimate the frequency in the population of specific recall responses to selected epitopes, 20 peripheral blood donors were evaluated for induction of cytokine expression.

Briefly, whole blood was obtained from Research Blood Components (Cambridge). Blood was diluted 1:1 in phosphate buffered saline (PBS) and then 35 mL overlaid on top of 12 mLs ficoll-paque premium(GE Healthcare) in a 50mL tube. Tubes were spun at 1400 RPM for 30 minutes, and the transition phase PBMCs collected, diluted in PBS with 10% fetal calf serum (FCS) and spun at 1200 rpm for 10 minutes. Cells were resuspended in cell freezing media (from Sigma) and immediately frozen at -80°C overnight. For long term storage, cells were transferred to liquid nitrogen. Cells were thawed (37°C waterbath) as needed and resuspended in PBS with 10% FCS, spun down and resuspended to 5 X 10^6 cells / mL in culture media (RPMI [cellgro]), supplemented with 5% heat inactivated human serum (Sigma) I-glutamine, penicillin and streptomycin).

For memory T-cell recall response assays, cells were cultured in 24-well plates with 4µM of a peptide according to the invention (obtained from GenScript) at 37°C 5%CO2 for 2 hours. One microlitre Brefeldin A (Golgiplug, BD) per mL of culture media was then added and cells returned to a 37°C incubator for 4-6 hours. Cells were then transferred to a lower temperature (27°C) incubator (5% CO2) overnight and then were processed for flow cytometry analysis. Detection of activated memory T-cells was performed by incubation of cells with CD4-FITC, CD45RA-PE, CD62L-Cy7PE (BD) followed by membrane permeabilization and fixing (BD). Intracellular expression of interferon-gamma was detected using an interferon-gamma-APC monoclonal (BioLegend). 200,000 - 500,000 cells were then analyzed using a FACSCalibre flow cytometer, and Cellquest software. Cells were scored positive if they were CD4+, CD45RAmedium, CD62Lhigh and IFN-gamma positive.

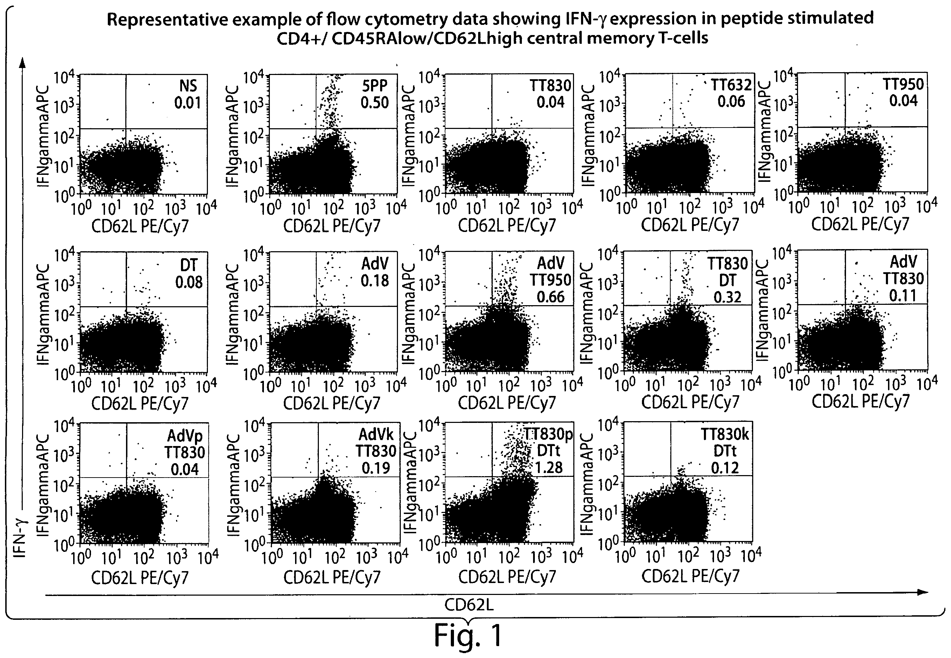

A representative example of flow cytometry data showing activation by chimeric peptides is shown in Figure 1, and the summary of all the data is shown in Figure 2.

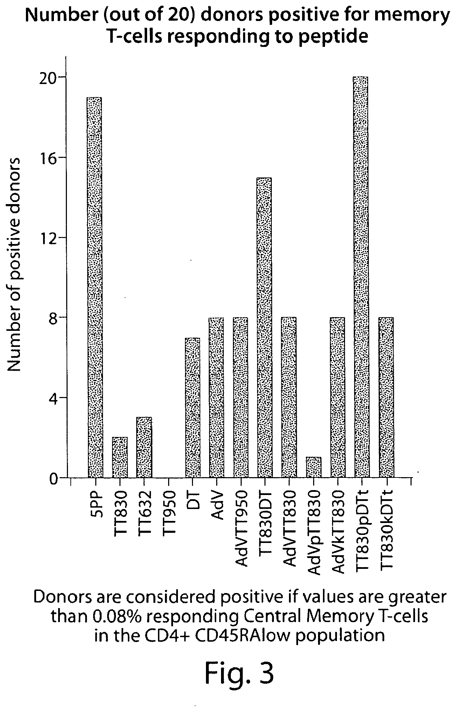

The data show: (1) Chimeric peptides according to the invention activate a higher number of central memory T-cells than individual peptides alone, and the chimeric peptide TT830pmglpDTt (which contains a cathepsin cleavage site) gave the highest response. (2) Inventive chimeric peptides get a recall response from more people than individual peptides, with TT830pmglpDTt being positive in 20/20 donors (Figure 3). (3) The chimeric peptide TT830pmglpDTt which contains a cathepsin cleavage site is more active than its individual components (TT830 and DT) alone, and better than a peptide identical except without a cleavage site (TT830DT), suggesting the addition of a cathepsin cleavage site into inventive chimeric peptides can provide an enhanced recall response. (4) The data confirms the T-cell epitope prediction analysis shown in Figure 15. The analysis predicted that chimeric peptides consisting of both TT830 and DT epitopes (TT830DTt) would provide the highest binding affinity across a broad range of HLA-DR alleles, and inclusion of a cathepsin cleavage site (TT830pmglpDTt) enhanced the response. Addition of TT830 or TT950 to the DP specific epitope AdV did not improve the number of positive responders compared to the AdV epitope alone. The high affinity and broad coverage of AdVTT830 was due to generation of a neoepitope at the junction of AdV and TT830. While they may generate predictions of high affinity, neo-epitopes will not induce a memory recall response in immunized individuals. Inclusion of a cathepsin cleavage site between the epitopes eliminates the neoepitope. In one case insertion of a cathepsin cleavage site eliminated activity of the AdV epitope (AdVpTT830), possibly due to an alteration in confirmation making the epitope unsuitable for Class II binding.

### Example 4: Testing of Peptide Activated Memory T-cells

Early central memory -cells express multiple cytokines (IL-2, TNF-α, IFN-γ) when re-activated with specific peptides, whereas committed effector memory T-cells are thought to selectively express IL-4 for TH2 committed effector memory, and IFN-y for TH1 committed effector memory. The status of peptide activated memory T-cells was tested using multi-color intracellular cytokine analysis of dendritic cell / CD4 cell co-cultures.

Human peripheral blood monocytes were isolated using negative-selection magnetic beads (Dynal) and cultured in the presence of GM-CSF and IL-4 for 1 week in order to induce differentiation into dendritic cells. Allogeneic CD4 T cells were isolated using magnetic bead separation (Dynal) and co-cultured in the presence of DCs in the presence or absence of peptide. The protocol for stimulation and analysis from that point is identical to that for PBMC described above in Example 2.

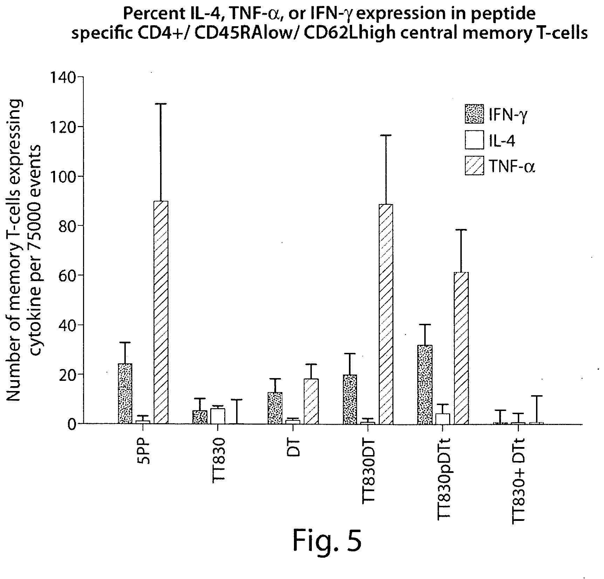

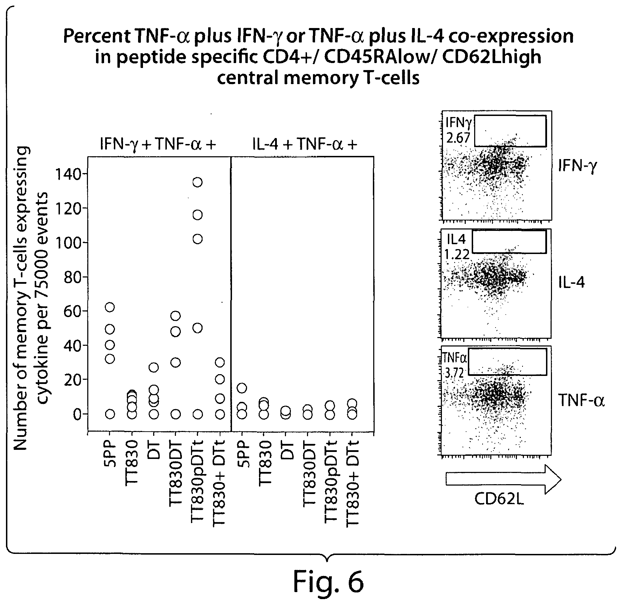

Stimulation with peptides TT830DT (SEQ. ID. No. 7) and TT830pDTt (TT830pmglpDTTrunc or SEQ. ID. No. 13) led to increased expression of TNF-α and IFN-γ, but not IL-4 (Figures 4, 5). Multiple color flow cytometry showed that both TT830DTt and TT830pDTt treated PBMC had peptide induced co-expression of TNF-α and IFN-γ, but not co-expression of TNF-α and IL-4 (Figure 6), suggesting that early central memory cells are activated.

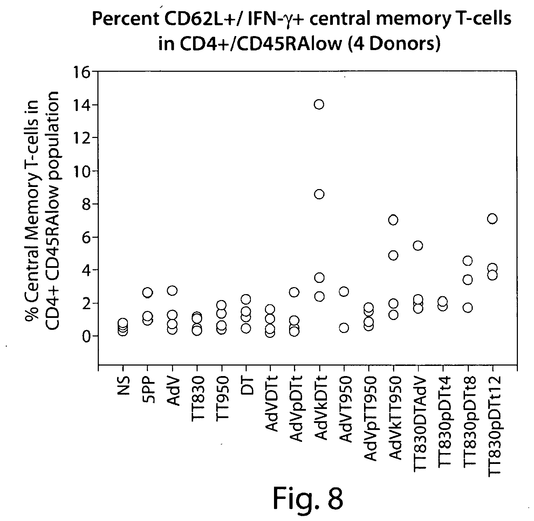

A series of chimeric peptides were constructed that contained a sequence from a DP4 specific adenoviral epitope, together with HLA-DR epitopes from TT and DT, with and without cathepsin linkers between the epitopes (Figure 8). As previously described, cells were cultured in 24-well plates with 4µM of a peptide according to the invention (obtained from GenScript) at 37°C and 5% CO2 for 2 hours. One microlitre Brefeldin A (Golgiplug, BD) per mL of culture media was then added and cells returned to a 37°C incubator for 4-6 hours. Cells were then transferred to a lower temperature (27°C) incubator (5% CO2) overnight and then were processed for flow cytometry analysis. Detection of activated memory T-cells was performed by incubation of cells with CD4-FITC, CD45RA-PE, CD62L-Cy7PE (BD) followed by membrane permeabilization and fixing (BD). Intracellular expression of interferon-gamma was detected using an interferon-gamma-APC monoclonal (BioLegend). 200,000 - 500,000 cells were then analyzed using a FACSCalibre flow cytometer, and Cellquest software. Cells were scored positive if they were CD4+, CD45RAmedium, CD62Lhigh and IFN-gamma positive. Analysis of 4 donors for memory T-cell recall response showed that individual peptides, and heterodimeric peptides lacking a cathepsin cleavage site produced a weaker response as compared to the donor response to heterotrimeric peptides (AdVkDTt, AdVkTT950) that contained the 'kvsvr' cathepsin cleavage site. In addition a heterotrimeric peptide (TT830DTAdV) containing AdV, DT, and TT epitopes also showed a recall response in all 4 donors.

### Example 5: Modifications of MHC II Binding Peptides to Adjust Physical Properties

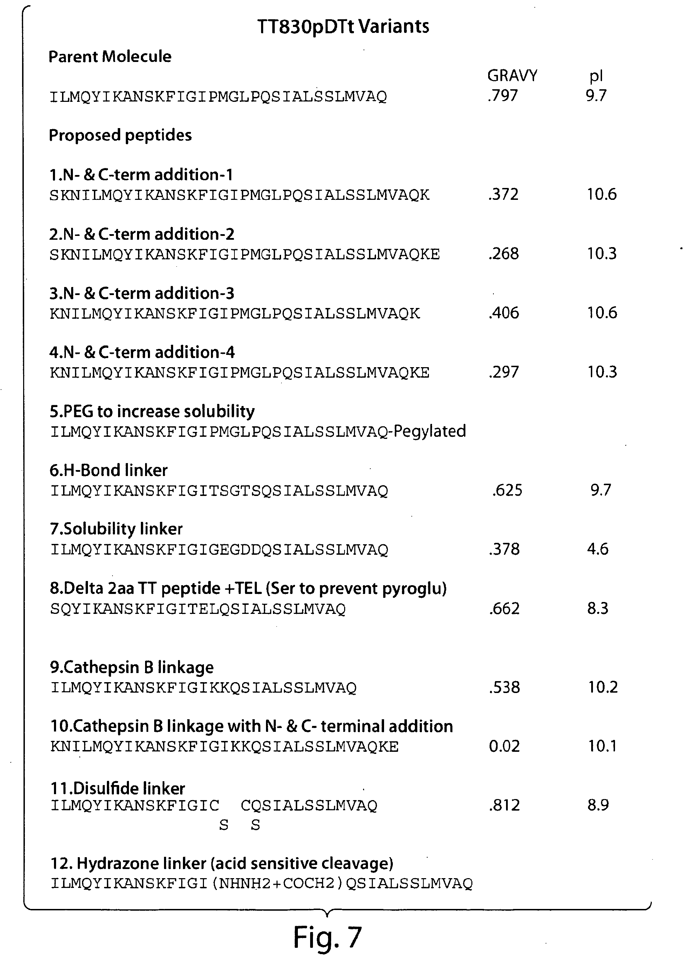

A series of modified TT830pDTt (SEQ ID NO. 13) sequences were generated in order to alter peptide properties as shown in Figure 7. The generical scope and nature of these types of modifications have been described elsewhere herein. Initial objectives of the modification of peptides were to: 1) improve aqueous solubility (lower GRAVY-Grand Average of Hydropathicity, 2) change the pl through modifications of the N- and/or C-terminal amino acids 3) modify the internal linkage (Cat S cleavage PMGLP), and to modify both external and internal linkage. 4) understand the importance of processing of the peptide in the endosomal compartment through modification of the Cat S binding site by changing to a Cathespin B cleavage or creating an alternative peptide breakdown process.

Additionally, variations of the AdVkDT sequence were generated in order to alter hydrophobicity of the peptide and to reduce the pl to near-neutral pH.

Sequence additions to the N-terminus were guided in part by similarity to the native amino acid sequence preceding the N-terminus of the AdV-derived epitope:
AdVkDTd1 EESTLLYVLFEVkvsvrQSIALSSLMVAQK (30), pl = 6.6-7.1 (seq 71)
AdVkDTd2 ESTLLYVLFEVkvsvrQSIALSSLMVAQKE (30), pl = 6.6-7.1 (seq 72)
AdVkDTd3 KESTLLYVLFEVkvsvrQSIALSSLMVAQE (30), pl = 6.6-7.1 (seq 73)

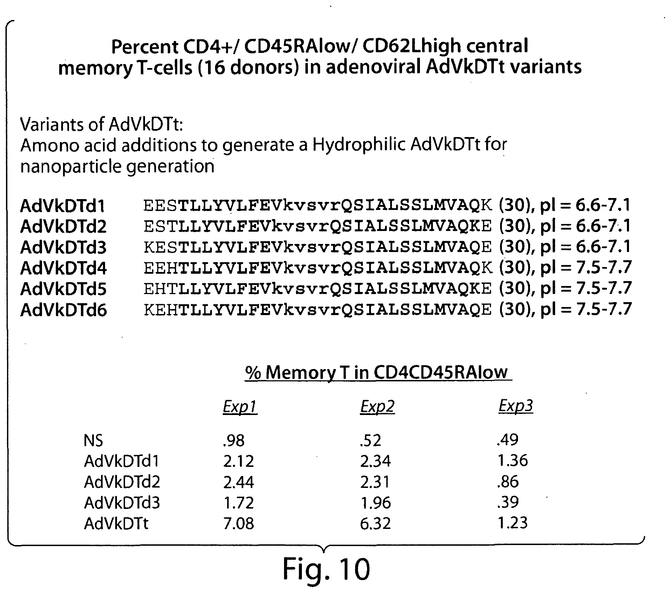

The results for variants of AdVkDT (SEQ ID NOS. 71-73) are shown (Figure 10). In all experiments from 3 different donors, the AdVkDT variants (SEQ ID NOS. 71-73) induced a robust recall response compared to a non-stimulated (NS) control.

### Example 6: Influenza Specific Memory Peptides

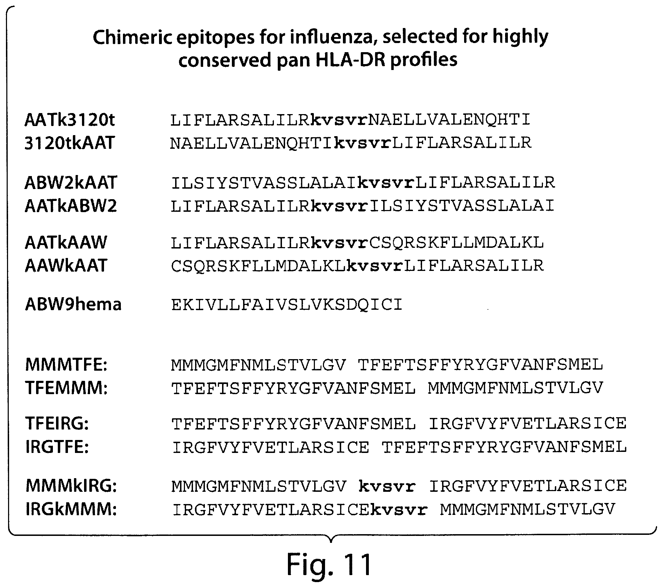

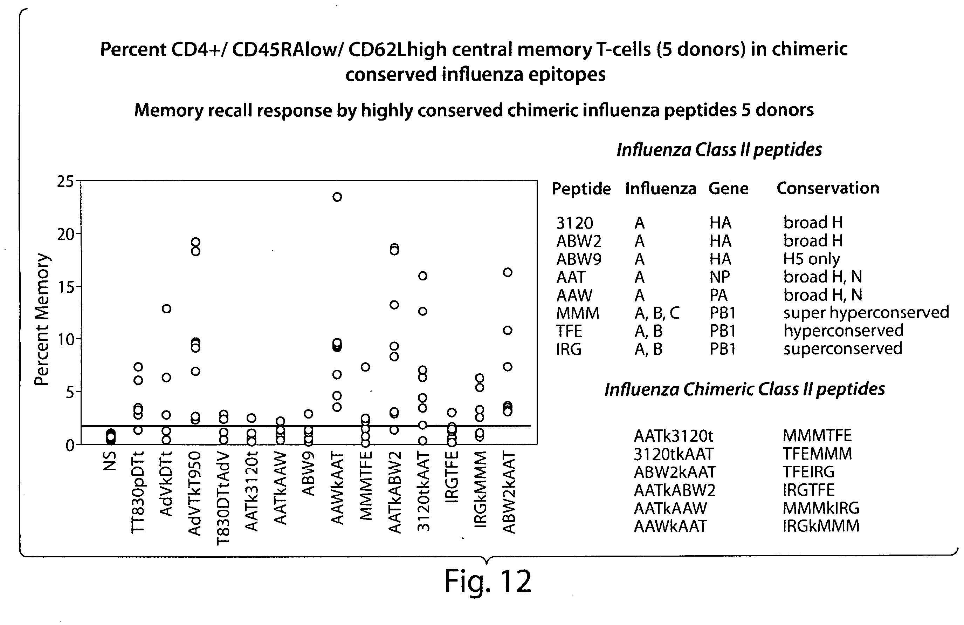

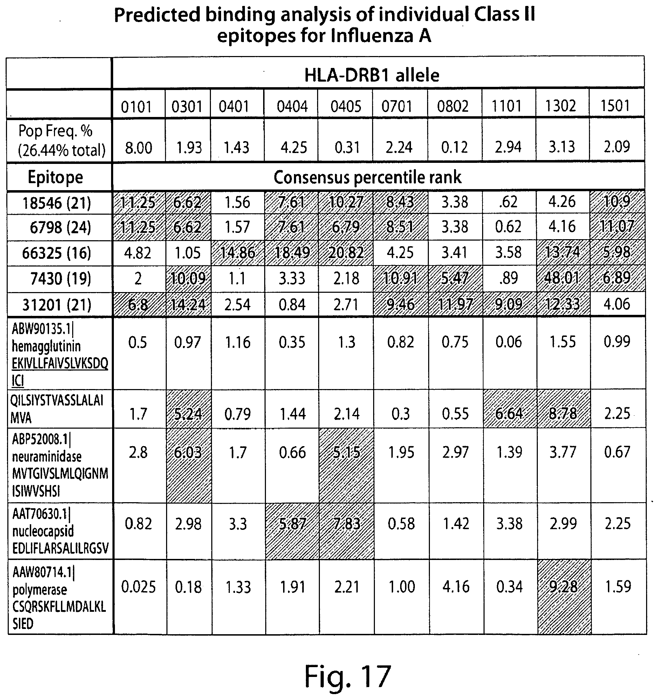

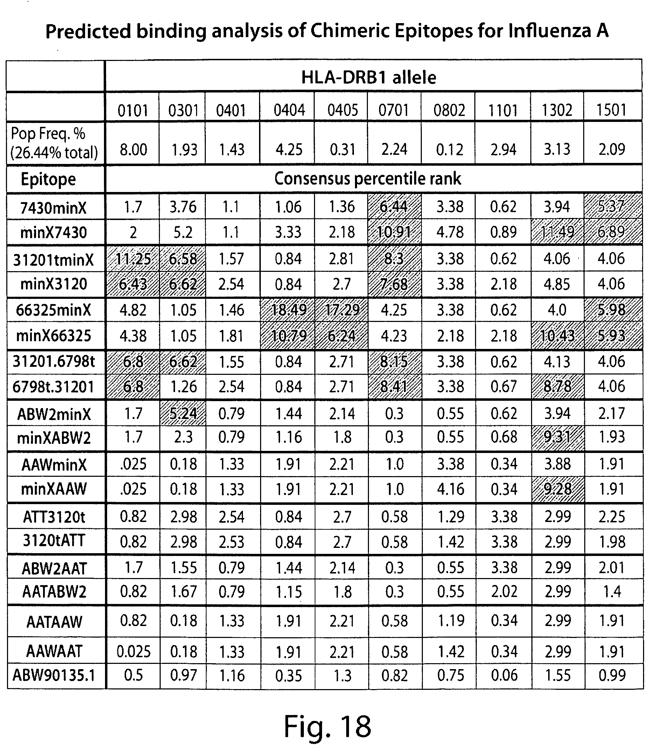

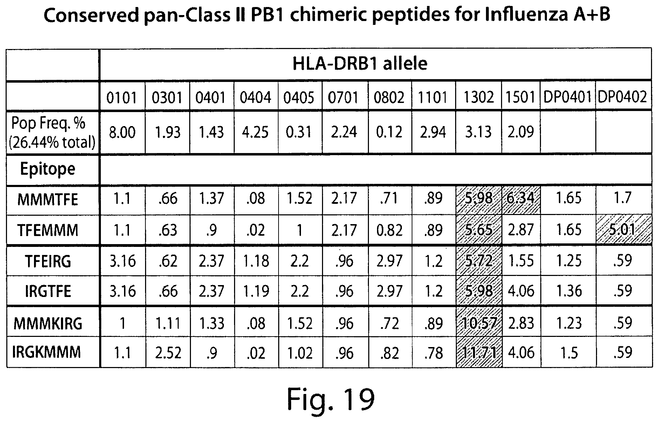

As an example of a specific single pathogen optimized composition according to the invention, pan HLA-DR epitopes were identified that were highly conserved within influenza type A, influenza type A and B, or influenza type A, B, and C (Figures 11 and 12) using the National Institute of Health's (NIH) Blast program and nucleotide database from the http://blast.ncbi.nlm.nih.gov/Blast.cgi in combination with Class II epitope prediction using the Immune Epitope Database (IEDB) (http://www.immuneepitope.org/) T cell epitope prediction tools. T cell epitope prediction results for individual epitopes and chimeric epitopes are shown in Figures 17-19, and chimeric epitopes with predicted high affinity were tested for the ability to generate a memory T-cell response. Briefly: PBMCs were cultured in 24-well plates with 4µM of peptide at 37oC 5%CO2 for 2 hours. Brefeldin A was then added and cells returned to a 37oC incubator for 4-6 hours. Cells were then transferred to a lower temperature (27oC) incubator (5% CO2) overnight and then were processed for flow cytometry analysis. Detection of activated memory T-cells was performed by incubation of cells with CD4-FITC, CD45RA-PE, CD62L-Cy7PE (BD). 200,000 - 500,000 cells were then analyzed using a FACSCalibre flow cytometer, and

Cellquest software. Cells were scored positive if they were CD4+, CD45RAmedium, CD62Lhigh and IFN-gamma positive.

**Individual epitopes:**

| | | |
|---|---|---|
| (minx) | YVKQNTLKLAT | (SEQ ID NO:74) |
| 7430) | CYPYDVPDYASLRSLVASS | (SEQ ID NO:75) |
| (31201t) | NAELLVALENQHTI | (SEQ ID NO:76) |
| (66325) | TSLYVRASGRVTVSTK | (SEQ ID NO:77) |
| (ABW1) | EKIVLLFAIVSLVKSDQICI | (SEQ ID NO:78) |
| (ABW2) | QILSIYSTVASSLALAIMVA | (SEQ ID NO:79) |
| (ABP) | MVTGIVSLMLQIGNMISIWVSHSI | (SEQ ID NO:80) |
| (AAT) | EDLIFLARSALILRGSV | (SEQ ID NO:81) |
| (AAW) | CSQRSKFLLMDALKLSIED | (SEQ ID NO:82) |
| (IRG) | IRGFVYFVETLARSICE | (SEQ ID NO:83) |
| (TFE) | TFEFTSFFYRYGFVANFSMEL | (SEQ ID NO:84) |
| (MMM) | MMMGMFNMLSTVLGV | (SEQ ID NO:85) |

**Chimeric epitopes:**

| | | |
|---|---|---|
| AATk3120t | LIFLARSALILRkvsvrNAELLVALENQHTI | (SEQ ID NO:86) |
| 3120tkAAT | NAELLVALENQHTIkvsvrLIFLARSALILR | (SEQ ID NO:87) |
| ABW2kAAT | ILSIYSTVASSLALAIkvsvrLIFLARSALILR | (SEQ ID NO:88) |
| AATkABW2 | LIFLARSALILRkvsvrILSIYSTVASSLALAI | (SEQ ID NO:89) |
| AATkAAW | LIFLARSALILRkvsvrCSQRSKFLLMDALKL | (SEQ ID NO:90) |
| AAWkAAT | CSQRSKFLLMDALKLkvsvrLIFLARSALILR | (SEQ ID NO:91) |
| ABW9hema | EKIVLLFAIVSLVKSDQICI | (SEQ ID NO:92) |
| MMMTFE | MMMGMFNMLSTVLGV TFEFTSFFYRYGFVANFSMEL | (SEQ ID NO:93) |
| TFEMMM | TFEFTSFFYRYGFVANFSMEL MMMGMFNMLSTVLGV | (SEQ ID NO:94) |
| TFEIRG | TFEFTSFFYRYGFVANFSMEL IRGFVYFVETLARSICE | (SEQ ID NO:95) |
| IRGTFE | IRGFVYFVETLARSICE TFEFTSFFYRYGFVANFSMEL | (SEQ ID NO:96) |
| MMMkIRG | MMMGMFNMLSTVLGV kvsvr IRGFVYFVETLARSICE | (SEQ ID NO:97) |
| IRGkMMM | IRGFVYFVETLARSICEkvsvr MMMGMFNMLSTVLGV | (SEQ ID NO:98) |

Chimeric Influenza peptide sequences are shown in Figure 11. T-cell memory recall response from 5 PBMC donors is shown in Figure 12. A memory T-cell recall response was positive for chimeric epitopes AAWkAAT, AATkABW2, 3120tkAAT, and ABW2kAAT, but not in the non-chimeric H5 restricted pan HLA-DR epitope ABW9. These data show that four inventive chimeric conserved epitope containing peptides specific for influenza are active in inducing a memory recall response.

### Example 7: Synthetic Nanocarrier Formulations (Prophetic)

Resiquimod (aka R848) is synthesized according to the synthesis provided in Example 99 of US Patent 5,389,640 to Gerster et al. A PLA-PEG-nicotine conjugate is prepared at Selecta Biosciences using a conventional conjugation strategy. PLA is prepared by a ring opening polymerization using D,L-lactide (MW = approximately 15 KD - 18 KD). The PLA structure is confirmed by NMR. The polyvinyl alcohol (Mw = 11 KD - 31 KD, 85% hydrolyzed) is purchased from VWR scientific. These are used to prepare the following solutions:
1. Resiquimod in methylene chloride @ 7.5 mg/mL
2. PLA-PEG-nicotine in methylene chloride @ 100 mg/mL
3. PLA in methylene chloride @ 100 mg/mL
4. Peptide in water @ 10 mg/mL, the peptide having the sequence: ILMQYIKANSKFIGIPMGLPQSIALSSLMVAQ (SEQ ID NO. 13)
5. Polyvinyl alcohol in water @50 mg/mL.

Solution #1 (0.4 mL), solution #2 (0.4 mL), solution #3 (0.4 mL) and solution #4 (0.1 mL) are combined in a small vial and the mixture is sonicated at 50% amplitude for 40 seconds using a Branson Digital Sonifier 250. To this emulsion is added solution #5 (2.0 mL) and sonication at 35% amplitude for 40 seconds using the Branson Digital Sonifier 250 forms the second emulsion. This is added to a beaker containing water (30 mL) and this mixture is stirred at room temperature for 2 hours to form the nanoparticles. A portion of the nanocarrier dispersion (1.0 mL) is diluted with water (14 mL) and this is concentrated by centrifugation in an Amicon Ultra centrifugal filtration device with a membrane cutoff of 100 KD. When the volume is about 250µL, water (15 mL) is added and the particles are again concentrated to about 250µL using the Amicon device. A second washing with phosphate buffered saline (pH = 7.5, 15 mL) is done in the same manner and the final concentrate is diluted to a total volume of 1.0 mL with phosphate buffered saline. This is expected to provide a final nanocarrier dispersion of about 2.7 mg/mL in concentration.

### Example 8: Synthetic Nanocarrier Formulations (Prophetic)

Resiquimod (aka R848) is synthesized according to the synthesis provided in Example 99 of US Patent 5,389,640 to Gerster et al. PLA-PEG-nicotine conjugate is prepared at Selecta Biosciences. PLA is prepared by a ring opening polymerization using D,L-lactide (MW = approximately 15 KD - 18 KD). The PLA structure is confirmed by NMR. The polyvinyl alcohol (Mw = 11 KD - 31 KD, 85% hydrolyzed) is purchased from VWR scientific. These are used to prepare the following solutions:
1. PLA-R848 conjugate @ 100 mg/mL in methylene chloride
2. PLA-PEG-nicotine in methylene chloride @ 100 mg/mL
3. PLA in methylene chloride @ 100 mg/mL
4. Peptide in water @ 12 mg/mL, the peptide having the sequence: TLLYVLFEVNNFTVSFWLRVPKVSASHLET (SEQ ID NO. 5)
5. Polyvinyl alcohol in water @50 mg/mL.

Solution #1 (0.25 to 0.75 mL), solution #2 (0.25 mL), solution #3 (0.25 to 0.5 mL) and solution #4 (0.1 mL) are combined in a small vial and the mixture is sonicated at 50% amplitude for 40 seconds using a Branson Digital Sonifier 250. To this emulsion is added solution #5 (2.0 mL) and sonication at 35% amplitude for 40 seconds using the Branson Digital Sonifier 250 forms the second emulsion. This is added to a beaker containing phosphate buffer solution (30 mL) and this mixture is stirred at room temperature for 2 hours to form the nanoparticles. To wash the particles a portion of the nanoparticle dispersion (7.0 mL) is transferred to a centrifuge tube and spun at 5,300g for one hour, supernatant is removed, and the pellet is re-suspended in 7.0 mL of phosphate buffered saline. The centrifuge procedure is repeated and the pellet is re-suspended in 2.2 mL of phosphate buffered saline for an expected final nanoparticle dispersion of about 10 mg/mL.

### Example 9: Conjugation of Inventive Compositions to Carrier Protein (Prophetic)

A peptide (Seq ID No.5) is modified with an additional Gly-Cys at the C-terminal for conjugation to a carrier protein, CRM197 via the thiol group on the C-terminal Cys. CRM₁₉₇, is a non-toxic mutant of diphtheria toxin with one amino acid change in its primary sequence. The glycine present at the amino acid position 52 of the molecule is replaced with a glutamic acid via a single nucleic acid codon change. Due to this change, the protein lacks ADP-ribosyl transferase activity and becomes non-toxic. It has a molecular weight of 58,408 Da.

Free amino groups of CRM₁₉₇ are bromoacetylated by reaction with an excess of bromoacetic acid N-hydroxysuccinimide ester (Sigma Chemical Co., St. Louis, Mo.) CRM₁₉₇ (15 mg) is dissolved in 1.0 M NaHCO₃ (pH 8.4) and cooled with ice. A solution of bromoacetic acid N-hydroxysuccinimide ester (15 mg in 200 µL dimethylformamide (DMF), is added slowly to the CRM₁₉₇ solution, and the solution is gently mixed at room temperature in the dark for 2 hour. The resulting bromoacetylated (activated) protein is then purified by diafiltration via a dialysis with a 10 K MWCO membrane. The degree of bromoacetylation was determined by reacting the activated CRM₁₉₇ with cysteine, followed by amino acid analysis and quantitation of the resulting carboxymethylcysteine (CMC).

The bromoacetylated CRM₁₉₇ is dissolved in 1 M sodium carbonate/bicarbonate buffer at pH 9.0 and maintained at 2-8 C under argon. A solution of peptide (TLLYVLFEVNNFTVSFWLRVPKVSASHLET-G-C (SEQ ID NO:107; modified SEQ ID NO. 5)) (10 mg) in 1 M sodium carbonate/bicarbonate buffer at pH 9.0 is added to the bromoacetylated CRM₁₉₇ solution, and the mixture is stirred at 2-8° C for 15-20 hours. The remaining bromoacetyl groups are then capped with a 20-fold molar excess of N-acetylcysteamine for 4-8 hours at 2-8° C. The resulting peptide-CRM197 conjugate is then purified at room temperature by diafiltration on a 10K MWCO membrane by diafiltering against 0.01 M sodium phosphate buffer/0.9% NaCl, pH 7.0. The retentate, peptide-CRM197 conjugate, is collected and analyzed for protein content (Lowry or Micro-BCA colorimetric assay), by SDS-PAGE, by amino acid analysis, and for immunogenicity in mice.

### Example 10: Mixture of Inventive Compositions with Conventional Vaccine Comprising an Antigen (Prophetic)

PLA is prepared by a ring opening polymerization using D,L-lactide (MW = approximately 15 KD - 18 KD). The PLA structure is confirmed by NMR. The polyvinyl alcohol (Mw = 11 KD - 31 KD, 87-89% hydrolyzed) is purchased from VWR scientific. These are used to prepare the following solutions:
1. PLA in methylene chloride @ 100 mg/mL
2. PLA-PEG in methylene chloride @ 100 mg/mL
3. Peptide in aqueous solution @ 10 mg/mL, the peptide having the sequence of SEQ ID NO. 91
4. Polyvinyl alcohol in water or phosphate buffer @ 50 mg/mL.

Solution #1 (0.5 to 1.0 mL), solution # 2 (0.25 to 0.5 mL), and solution #3 (0.05 to 0.3 mL) are combined in a glass pressure tube and the mixture is sonicated at 50% amplitude for 40 seconds using a Branson Digital Sonifier 250. To this emulsion is added solution #4 (2.0 to 3.0 mL) and sonication at 30% amplitude for 40 to 60 seconds using the Branson Digital Sonifier 250 forms the second emulsion. This is added to a beaker containing phosphate buffer solution (30 mL) and this mixture is stirred at room temperature for 2 hours to form the nanocarriers. To wash the particles a portion of the nanocarrier dispersion (27.0 to 30.0 mL) is transferred to a centrifuge tube and spun at 21,000g for 45 minutes, supernatant is removed, and the pellet is re-suspended in 30.0 mL of phosphate buffered saline. The centrifuge procedure is repeated and the pellet is re-suspended in 8.1 - 9.3 mL of phosphate buffered saline.

A 4 mL aliquot of the suspended synthetic nanocarriers is centrifuged to settle the synthetic nanocarriers. The supernatant is discarded and a 0.5-mL suspension of Fluarix® trivalent influenza virus vaccine is added. The combination vaccine is agitated to re-suspend the nanocarriers and the resulting suspension is stored at-20°C prior to use.

### Example 11: Coupling of Inventive Compositions to Gold Nanocarriers (Prophetic)

Step-1. Formation of Gold Nanocarriers (AuNCs): A aq. solution of 500 mL of 1 mM HAuCl4 is heated to reflux for 10 min with vigorous stirring in a 1 L roundbottom flask equipped with a condenser. A solution of 50 mL of 40 mM of trisodium citrate is then rapidly added to the stirring solution. The resulting deep wine red solution is kept at reflux for 25-30 min and the heat is withdrawn and the solution is cooled to room temperature. The solution is then filtered through a 0.8 µm membrane filter to give the AuNCs solution. The AuNCs are characterized using . visible spectroscopy and transmission electron microscopy. The AuNCs are ca. 20 nm diameter capped by citrate with peak absorption at 520 nm.
Step-2. Direct peptide conjugation to AuNCs: The C-terminal peptide of Example 9 (a peptide of SEQ ID NO. 5 containing a C-terminal cysteine) is coupled to the AuNCs as follows: A solution of 145 µl of the peptide (10 µM in 10 mM pH 9.0 carbonate buffer) is added to 1 mL of 20 nm diameter citrate-capped gold nanoparticles (1.16 nM) to produce a molar ratio of thiol to gold of 2500:1. The mixture is stirred at room temperature under argon for 1 hour to allow complete exchange of thiol with citrate on the gold nanoparticles. The peptide-AuNCs conjugate is then purified by centrifuge at 12,000g for 30 minutes. The supernatant is decanted and the pellet containing peptide-AuNCs is resuspended 1 mL WFI water for further analysis and bioassay.

### Example 12: Synthetic Nanocarriers Using Modified Compositions of Example 5

Resiquimod (aka R848) was synthesized according to the synthesis provided in Example 99 of US Patent 5,389,640 to Gerster et al. and was conjugated to PLGA, forming PLGA-R848, using an amide linker. PLGA (IV 0.10 dL/g) and PLA (IV 0.21 dUg) were purchased from Lakeshore Biomaterials. A PLA-PEG-nicotine conjugate was prepared at Selecta Biosciences using a conventional conjugation strategy. Polyvinyl alcohol (Mw = 11 KD - 31 KD, 87-89% hydrolyzed) was purchased from JT Baker.

These were used to prepare the following solutions:
1. PLGA-R848 in methylene chloride @ 100 mg/mL
2. PLA-PEG-nicotine in methylene chloride @ 100 mg/mL
3. PLA in methylene chloride @ 100 mg/mL
4. Peptide @ 10 mg/mL in a solution comprised of 10% DMSO, 50% lactic acid USP, and 40% water, the peptide having the sequence:

### EESTLLYVLFEVKVSVRQSIALSSLMVAQK (SEQ ID NO:71)

### 5. Polyvinyl alcohol in pH 8 phosphate buffer @ 50mg/mL

Solution #1 (0.5 mL), solution #2 (0.25 mL), and solution #3 (0.25mL) were combined and solution #4 (0.25mL) was added in a small vessel and the mixture was sonicated at 50% amplitude for 40 seconds using a Branson Digital Sonifier 250. To this emulsion was added solution #5 (2.0 mL). The mixture was sonicated at 30% amplitude for 40 seconds using the Branson Digital Sonifier 250 to form the second emulsion. This emulsion was then added to a stirring 50mL beaker containing a 70mM pH 8 phosphate buffer solution (30 mL) and was then stirred at room temperature for 2 hours to form the synthetic nanocarriers.

To wash the synthetic nanocarriers, a portion of the synthetic nanocarrier dispersion (27.5mL) was transferred to a 50mL centrifuge tube and spun at 9500 rpm (13,800 g) for one hour at 4°C, supernatant was removed, and the pellet was re-suspended in 27.5 mL of PBS (phosphate buffered saline). The centrifuge-based wash procedure was repeated and the pellet was re-suspended in 8.5 g of phosphate buffered saline for a nominal synthetic nanocarrier dispersion concentration of 10 mg/mL. Gravimetric determination of actual concentration was made, and the concentration subsequently adjusted in PBS to 5 mg/mL.

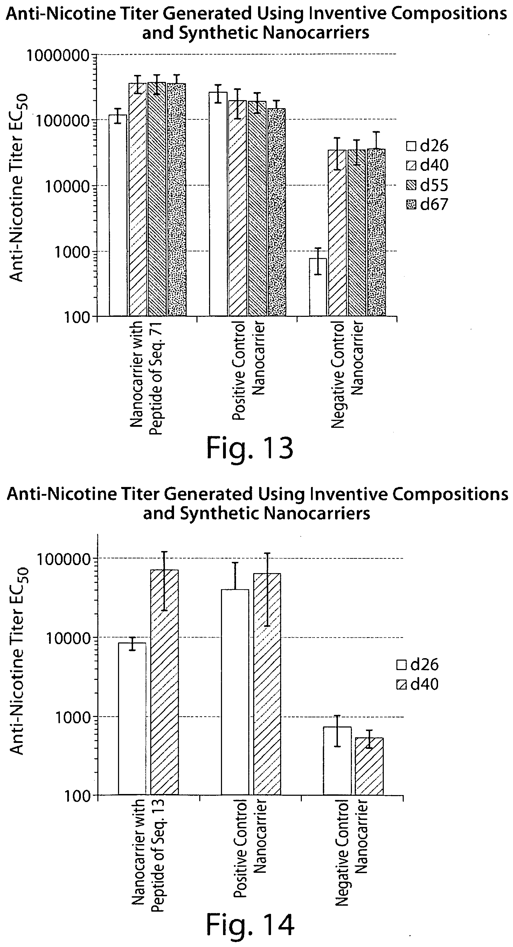

Immunogenicity of the synthetic nanocarrier formulation was determined by an inoculation study in C57BL6 mice. Inoculations were made subcutaneously into the hind pads of naïve C57BL6 mice (5 mice per group) according to a schedule of a prime on day 0 followed by boosts on days 14 and 28. For each inoculation a total of 100 µg nanocarriers was injected, 50 µg per hind limb. Sera were collected at days 26, 40, 55, and 67. Anti-nicotine antibody titers were determined for the sera as EC50 values. Control groups were inoculated in like fashion utilizing synthetic nanocarrier of same polymeric formulation, incorporating a known murine MHC II binding peptide (ovalbumin 323-339 amide) as a positive control, or without any MHC II binding peptide. Data is shown in Figure 13.

### Example 13: Synthetic Nanocarriers Using Inventive Compositions

PLGA (5050 DLG 2.5A, IV 0.25 dL/g) was purchased from Lakeshore Biomaterials. A PLA-PEG-nicotine conjugate was prepared at Selecta Biosciences. Polyvinyl alcohol (Mw = 11 KD - 31 KD, 87-89% hydrolyzed) was purchased from JT Baker.

These were used to prepare the following solutions:
1. PLGA in methylene chloride @ 100 mg/mL
2. PLA-PEG-nicotine in methylene chloride @ 100 mg/mL
3. Peptide @ 4 mg/mL in a solvent comprised of of 10% DMSO in water, the peptide having the sequence: ILMQYIKANSKFIGIPMGLPQSIALSSLMVAQ (SEQ ID NO: 13)
4. Polyvinyl alcohol in pH 8 phosphate buffer @ 50 mg/mL

Solution #1 (0.375 mL), and solution #3 (0.125 mL) were combined and diluted with 0.50 mL methylene chloride before solution #3 (0.25 mL) was added in a small vessel and the mixture was sonicated at 50% amplitude for 40 seconds using a Branson Digital Sonifier 250. To this emulsion was added solution #4 (3.0 mL). The mixture was sonicated at 30% amplitude for 60 seconds using the Branson Digital Sonifier 250 to form the second emulsion. This emulsion was then added to a stirring 50mL beaker containing a 70mM pH 8 phosphate buffer solution (30 mL) and was then stirred at room temperature for 2 hours to form the synthetic nanocarriers.

To wash the particles a portion of the synthetic nanocarriers dispersion (29mL) was transferred to a 50mL centrifuge tube and spun at 21,000 rcf for 45 minutes at 4°C, supernatant was removed, and the pellet was re-suspended in 29 mL of PBS (phosphate buffered saline). The centrifuge-based wash procedure was repeated and the pellet was then re-suspended in 4.4 g of PBS for a nominal synthetic nanocarriers dispersion concentration of 10 mg/mL. Gravimetric determination of actual concentration was made, and the concentration subsequently adjusted in PBS to 5 mg/mL.

Immunogenicity of the synthetic nanocarriers formulation was determined by an inoculation study in BALB/c mice. Synthetic nanocarriers were mixed with a solution of murine-active CpG adjuvant, PS-1826 immediately prior to injection. Inoculations were made subcutaneously into the hind pads of naïve BALB/c mice (5 mice per group) according to a schedule of a prime on day 0 followed by boosts on days 14 and 28. For each inoculation a total of 100 µg synthetic nanocarriers and 20 µg PS-1826 was injected, divided equally between the hind limbs. Sera were collected at days 26, and 40. Anti-nicotine antibody titers were determined for the sera as EC50 values. Control groups were inoculated in like fashion utilizing synthetic nanocarriers of similar polymeric formulation, with the positive control synthetic nanocarriers incorporating a known murine MHC II binding peptide (ovalbumin 323-339 amide), and the negative control nanocarrier lacking an MHC II binding peptide. Results are shown in Figure 14.

## Claims

1. A composition comprising synthetic nanocarriers comprising:
A - x - B; and
a pharmaceutically acceptable excipient;
wherein x comprises a cathepsin cleavage site comprising KVSVR;
wherein A comprises a first MHC II binding peptide, and the first MHC II binding peptide comprising a peptide having at least 70%, 80% or 90% identity to: a natural HLA-DP binding peptide, a natural HLA-DQ binding peptide, or a natural HLA-DR binding peptide;
wherein B comprises a second MHC II binding peptide, and the second MHC II binding peptide comprising a peptide having at least 70%, 80% or 90% identity to: a natural HLA-DP binding peptide, a natural HLA-DQ binding peptide, or a natural HLA-DR binding peptide, and
wherein A and B do not have 100% identity to one another.

2. The composition of claim 1, further comprising one or more antigens.

3. The composition of claim 2, wherein at least one of the antigens is not conjugated to the A-x-B.

4. The composition of claim 1, wherein the composition comprises:
A-x-B-y-C;and
a pharmaceutically acceptable excipient;
wherein y may comprise a linker or no linker;
wherein C comprises a third MHC II binding peptide, and the third MHC II binding peptide comprising a peptide having at least 70%, 80% or 90% identity to: a natural HLA-DP binding peptide, a natural HLA-DQ binding peptide, or a natural HLA-DR binding peptide; and
wherein A, B, and C do not have 100% identity to one another.

5. The composition of claim 4 wherein:
(a) y comprise a linker that comprises an amide linker, a disulfide linker, a sulfide linker, a 1,4-disubstituted 1,2,3-triazole linker, a thiol ester linker, a hydrazide linker, an imine linker, a thiourea linker, an amidine linker, or an amine linker; or
(b) y comprise a linker comprising a peptide sequence, a lysosome protease cleavage site, a biodegradable polymer, a substituted or unsubstituted alkane, alkene, aromatic or heterocyclic linker, a pH sensitive polymer, heterobifunctional linkers or an oligomeric glycol spacer; or
(c) y comprise no linker, and A-x-B and C comprise a mixture present in the composition.

6. A composition comprising:
one or more isolated nucleic acids, for example DNA or RNA, that encode A - x - B as defined in claim 1 or A - x - B - y - C as defined in claim 4, wherein when there is more than one isolated nucleic acid, the isolated nucleic acids together encode the A - x - B or A - x - B - y - C, and
wherein, when present, y comprises an amide linker, no linker, or a peptide linker.

7. The composition of claim 6 as dependent on claim 4, wherein: (a) y is a peptide linker that comprises a lysosome protease cleavage site, optionally, wherein A - x - B and C are encoded for by separate isolated nucleic acids in the composition or by the same isolated nucleic acid in the composition; (b) y is no linker, and A - x - B and C are encoded for by two or more separate isolated nucleic acids in the composition; or (c) y is no linker, and A - x - B and C are encoded for by the same isolated nucleic acid in the composition.

8. A composition comprising one or more isolated nucleic acids that are fulllength complements of the one or more isolated nucleic acids of claim 6 or claim 7.

9. A composition of any one of claims 1 to 5, wherein: (a) at least a portion of the composition of any one of the preceding claims is present on a surface of the synthetic nanocarrier; or (b) at least a portion of the composition of any one of the preceding claims is encapsulated by the synthetic nanocarrier.

10. A composition comprising A-x-B, wherein x comprises a cathepsin cleavage site comprising KVSVR and wherein A-x-B comprises an amino acid sequence that has at least 75% identity to any one of the amino acid sequences set forth as SEQ ID NOs: 12, 14, 17, 19, and 39-44; optionally wherein:
(a) the amino acid sequence has at least 85% or 95% identity to any one of the amino acid sequences set forth as SEQ ID NOs: 12, 14, 17, 19, and 39-44; or
(b) the amino acid sequence has any one of the amino acid sequences set forth as SEQ ID NOs: 12, 14, 17, 19, and 39-44.

11. A dosage form comprising: (a) the composition of any one of the preceding claims; or (b) a vaccine comprising the composition of any one of the preceding claims, optionally further comprising: (i) an adjuvant or (ii) a carrier conjugated to said composition.

12. The composition or dosage form of any of the preceding claims for use in a method comprising administering the composition or dosage form to a subject.

13. The composition of any one of claims 1-10 and 12 or dosage form of claim 11 or 12, wherein the natural HLA-DP, DQ or DR binding peptide comprises a peptide sequence obtained or derived from Clostridium tetani, Hepatitis B virus, Human herpes virus, Influenza virus, Vaccinia virus, Epstein-Barr virus, Chicken pox virus, Measles virus, Rous sarcoma virus, Cytomegalovirus, Varicella zoster virus, Mumps virus, Corynebacterium diphtheria, Human adenoviridae, Small pox virus, or an infectious organism capable of infecting humans and generating human CD4+ memory cells specific to the infectious organism following the initiation of infection.

14. The composition of any one of claims 1-10 and 12-13 or dosage form of any one of claims 11-13, wherein A and B or A and B and C each comprise: (a) peptide sequences obtained or derived from different infectious organisms; or (b) peptide sequences obtained or derived from identical infectious organisms; or (c) peptides having different MHC II binding repertoires.

15. The composition of any one of claims 1-10 and 12-14 or dosage form of any one of claims 11-14, wherein: (a) A, x, or B; or (b) A, x, B, y, or C, comprise sequence or chemical modifications that increase aqueous solubility of A - x - B or A - x - B - y - C, respectively , wherein the sequence or chemical modifications comprise addition of hydrophilic N- and/or C-terminal amino acids, hydrophobic N- and/or C-terminal amino acids, substitution of amino acids to achieve a pl of about 7.4 and to achieve a net-positive charge at about pH 3.0, and substitution of amino acids susceptible to rearrangement.

16. The composition of any one of claims 1-10 and 12-15 or dosage form of any one of claims 11-15, wherein the first and/or second MHC II and/or third MHC II binding peptide has a length ranging from: (a) 5-mer to 50-mer; (b) 5-mer to 30-mer; (c) 6-mer to 25-mer.

## Patentansprüche

1. Eine Zusammensetzung, umfassend synthetische Nanoträger [Nanocarrier], umfassend:
A - x - B;
und
einen pharmazeutisch annehmbaren Hilfsstoff;
wobei x eine Spaltstelle des Kathepsin umfasst, die KVSVR umfasst;
wobei A ein erstes MHC II bindendes Peptid umfasst und das erste MHC II bindende Peptid ein Peptid mit mindestens 70 %, 80 % oder 90 % Identität umfasst zu: einem natürlichen HLA-DP bindenden Peptid, einem natürlichen HLA-DQ bindenden Peptid oder einem natürlichen HLA-DR bindenden Peptid;
wobei B ein zweites MHC II bindendes Peptid umfasst und das zweite MHC II bindende Peptid ein Peptid mit mindestens 70 %, 80 % oder 90 % Identität umfasst zu: einem natürlichen HLA-DP bindenden Peptid, einem natürlichen HLA-DQ bindenden Peptid oder einem natürlichen HLA-DR bindenden Peptid; und
wobei A und B keine Identität von 100 % zueinander aufweisen.

2. Zusammensetzung nach Anspruch 1, die ferner ein oder mehrere Antigene umfasst.

3. Zusammensetzung nach Anspruch 2, wobei mindestens eines der Antigene nicht an das A-x-B konjugiert ist.

4. Zusammensetzung nach Anspruch 1, wobei die Zusammensetzung umfasst:
A-x-B-y-C;
und
einen pharmazeutisch annehmbaren Hilfsstoff;
wobei y einen Linker oder keinen Linker umfassen kann;
wobei C ein drittes MHC II bindendes Peptid umfasst und das dritte MHC II bindende Peptid ein Peptid mit mindestens 70 %, 80 % oder 90 % Identität umfasst zu: einem natürlichen HLA-DP bindenden Peptid, einem natürlichen HLA-DQ bindenden Peptid oder einem natürlichen HLA-DR bindenden Peptid; und
wobei A, B und C keine Identität von 100 % zueinander aufweisen.

5. Zusammensetzung nach Anspruch 4, wobei
(a) y einen Linker umfasst, der einen Amid-Linker, einen Disulfid-Linker, einen Sulfid-Linker, einen 1,4-disubstituierten 1,2,3-Triazol-Linker, einen Thiol-Ester-Linker, einen Hydrazid-Linker, einen Imin-Linker, einen Thioharnstoff-Linker, einen Amidin-Linker oder einen Amin-Linker umfasst; oder
(b) y einen Linker umfasst, der eine Peptidsequenz, eine Spaltstelle der Lysosom-Protease, ein biologisch abbaubares Polymer, einen substituierten oder nicht substituierten Alkan-, Alken-, aromatischen oder heterocyclischen Linker, ein pHempfindliches Polymer, heterobifunktionelle Linker oder einen oligomeren Glykol-Spacer umfasst; oder
(c) y keinen Linker umfasst, und A-x-B und C eine in der Zusammensetzung vorhandene Mischung umfassen.

6. Eine Zusammensetzung, umfassend:
eine oder mehrere isolierte Nukleinsäuren, beispielsweise DNA oder RNA, die für A - x - B wie in Anspruch 1 definiert kodieren oder A - x - B - y - C wie in Anspruch 4 definiert kodieren, wobei, wenn mehr als eine isolierte Nukleinsäure vorliegt, die isolierten Nukleinsäuren zusammen das A - x - B oder A - x - B - y - C kodieren, und
wobei, falls vorhanden, y einen Amid-Linker, keinen Linker oder einen Peptid-Linker umfasst.

7. Zusammensetzung nach Anspruch 6, wie von Anspruch 4 abhängig, wobei: (a) y ein Peptid-Linker ist, der eine Spaltstelle der Lysosom-Protease umfasst, wahlweise, wobei A - x - B und C durch getrennte isolierte Nukleinsäuren in der Zusammensetzung oder durch die gleiche isolierte Nukleinsäure in der Zusammensetzung kodiert sind; (b) y kein Linker ist und A - x - B und C durch zwei oder mehr getrennte isolierte Nukleinsäuren in der Zusammensetzung kodiert sind; oder (c) y kein Linker ist und A - x - B und C durch die gleiche isolierte Nukleinsäure in der Zusammensetzung kodiert sind.

8. Zusammensetzung, umfassend eine oder mehrere isolierte Nukleinsäuren, die Komplemente gesamter Länge der einen oder mehreren isolierten Nukleinsäuren nach Anspruch 6 oder Anspruch 7 sind.

9. Zusammensetzung nach einem der Ansprüche 1 bis 5, wobei: (a) wenigstens ein Teil der Zusammensetzung nach einem der vorhergehenden Ansprüche auf einer Oberfläche des synthetischen Nanoträgers vorliegt; (b) wenigstens ein Teil der Zusammensetzung nach einem der vorhergehenden Ansprüche durch den synthetischen Nanoträger eingekapselt ist.

10. Zusammensetzung, umfassend A-x-B, wobei x eine Spaltstelle des Kathepsin umfasst, die KVSVR umfasst, und worin A-x-B eine Aminosäuresequenz umfasst, die mindestens 75 % Identität zu irgendeiner der Aminosäuresequenzen aufweist, die als SEQ ID NO: 12, 14, 17, 19 und 39-44 angegeben sind; wobei wahlweise:
(a) die Aminosäuresequenz mindestens 85 % oder 95 % Identität zu irgendeiner der Aminosäuresequenzen aufweist, die als SEQ ID NO: 12, 14, 17, 19 und 39-44 angegeben sind; oder
(b) die Aminosäuresequenz irgendeine der Aminosäuresequenzen aufweist, die als SEQ ID NO: 12, 14, 17,19 und 39-44 angegeben sind.

11. Eine Dosierungsform, umfassend: (a) die Zusammensetzung nach einem der vorhergehenden Ansprüche; oder (b) einen Impfstoff, der die Zusammensetzung nach einem der vorhergehenden Ansprüche umfasst, wahlweise weiterhin umfassend: (i) ein Adjuvans oder (ii) einen Träger, der mit der Zusammensetzung konjugiert ist.

12. Zusammensetzung oder Dosierungsform nach einem der vorstehenden Ansprüche zur Verwendung in einem Verfahren, das die Verabreichung der Zusammensetzung oder Dosierungsform an ein Subjekt umfasst.

13. Zusammensetzung nach einem der Ansprüche 1 bis 10 und 12 oder Dosierungsform nach Anspruch 11 oder 12, wobei das natürliche HLA-DP, -DQ oder - DR bindende Peptid eine Peptidsequenz umfasst, die gewonnen wurde aus oder abgeleitet ist von Clostridium tetani, Hepatitis B-Virus, humanem Herpes-Virus, Influenza-Virus, Vaccinia-Virus, Epstein-Barr-Virus, Windpocken-Virus, Measles-Virus, Rous-Sarcoma-Virus, Cytomegalovirus, Varicella-Zoster-Virus, Mumps-Virus, Corynebacterium diphtheria, humane Adenoviridae, Pocken-Virus oder einem infektiösen Organismus, der fähig ist, Menschen zu infizieren und humane CD4+ Gedächtniszellen zu generieren, die nach der Initiation der Infektion für den infektiösen Organismus spezifisch sind.

14. Zusammensetzung nach einem der Ansprüche 1 bis 10 und 12-13 oder Dosierungsform nach einem der Ansprüche 11 bis 13, wobei A und B oder A und B und C jeweils umfassen: (a) Peptidsequenzen, die gewonnen wurden aus oder abgeleitet sind von verschiedenen infektiösen Organismen; oder (b) Peptidsequenzen, die gewonnen wurden aus oder abgeleitet sind von identischen infektiösen Organismen; oder (c) Peptide, die unterschiedliche MHC II bindende Repertoire aufweisen.

15. Zusammensetzung nach einem der Ansprüche 1 bis 10 und 12-14 oder Dosierungsform nach einem der Ansprüche 11 bis 14, wobei: (a) A, x oder B; oder (b) A, x, B, y oder C Sequenz- oder chemische Modifikationen umfassen, die die wässrige Löslichkeit von A - x - B oder respektive A - x - B - y - C erhöhen, wobei die Sequenz- oder chemischen Modifikationen die Addition von hydrophilen N- und/oder C-terminalen Aminosäuren, hydrophoben N- und/oder C-terminalen Aminosäuren, die Substitution von Aminosäuren zur Erzielung eines pl von etwa 7,4 und zur Erzielung einer netto-positiven Ladung bei etwa pH 3,0, und die Substitution von Aminosäuren, die für eine Umlagerung anfällig sind, umfassen.

16. Zusammensetzung nach einem der Ansprüche 1 bis 10 und 12-15 oder Dosierungsform nach einem der Ansprüche 11 bis 15, wobei das erste und/oder zweite MHC II und/oder dritte MHC II bindende Peptid eine Länge aufweist, die sich erstreckt von: (a) 5-mer bis 50-mer; (b) 5-mer bis 30-mer; (c) 6-mer bis 25-mer.

## Revendications

1. Composition comprenant des nanosupports synthétiques comprenant :
A-x-B ;
et
un excipient pharmaceutiquement acceptable ;
dans laquelle x comprend un site de clivage de cathepsine comprenant KVSVR ;
dans laquelle A comprend un premier peptide se liant au CMH II, et le premier peptide se liant au CMH II comprenant un peptide présentant au moins 70 %, 80 % ou 90 % d'identité avec : un peptide se liant au HLA-DP naturel, un peptide se liant au HLA-DQ naturel, ou un peptide se liant au HLA-DR naturel ;
dans laquelle B comprend un second peptide se liant au CMH II, et le second peptide se liant au CMH II comprenant un peptide présentant au moins 70 %, 80 % ou 90 % d'identité avec : un peptide se liant au HLA-DP naturel, un peptide se liant au HLA-DQ naturel, ou un peptide se liant au HLA-DR naturel, et
dans laquelle A et B ne présentent pas 100 % d'identité l'un avec l'autre.

2. Composition selon la revendication 1, comprenant en outre un ou plusieurs antigènes.

3. Composition selon la revendication 2, dans laquelle au moins l'un des antigènes n'est pas conjugué à A-x-B.

4. Composition selon la revendication 1, la composition comprenant :
A-x-B-y-C ;
et
un excipient pharmaceutiquement acceptable ;
dans laquelle y peut comprendre un lieur ou aucun lieur ;
dans laquelle C comprend un troisième peptide se liant au CMH II, et le troisième peptide se liant au CMH II comprenant un peptide présentant au moins 70 %, 80 % ou 90 % d'identité avec : un peptide se liant au HLA-DP naturel, un peptide se liant au HLA-DQ naturel, ou un peptide se liant au HLA-DR naturel ; et
dans laquelle A, B, et C ne présentent pas 100 % d'identité les uns avec les autres.

5. Composition selon la revendication 4 dans laquelle :
(a) y comprend un lieur qui comprend un lieur amide, un lieur disulfure, un lieur sulfure, un lieur 1,2,3-triazole 1,4-disubstitué, un lieur ester de thiol, un lieur hydrazide, un lieur imine, un lieur thiourée, un lieur amidine, ou un lieur amine ; ou
(b) y comprend un lieur comprenant une séquence peptidique, un site de clivage de protéase lysosomale, un polymère biodégradable, un lieur alcane, alcène, aromatique ou hétérocyclique substitué ou non substitué, un polymère sensible au pH, des lieurs hétérobifonctionnels ou un espaceur glycol oligomère ; ou
(c) y ne comprend aucun lieur, et A-x-B et C composent un mélange présent dans la composition.

6. Composition comprenant :
un ou plusieurs acides nucléiques isolés, par exemple de l'ADN ou de l'ARN, qui codent pour A-x-B tel que défini dans la revendication 1 ou A-x-B-y-C tel que défini dans la revendication 4, dans laquelle lorsqu'il y a plus d'un acide nucléique isolé, les acides nucléiques isolés ensemble codent pour A-x-B ou A-x-B-y-C, et
dans laquelle, lorsqu'il est présent, y comprend un lieur amide, aucun lieur, ou un lieur peptidique.

7. Composition selon la revendication 6 telle que dépendante de la revendication 4, dans laquelle : (a) y est un lieur peptidique qui comprend un site de clivage de protéase lysosomale, éventuellement, dans laquelle A-x-B et C sont codés par des acides nucléiques isolés séparés dans la composition ou par le même acide nucléique isolé dans la composition ; (b) y ne représente aucun lieur, et A-x-B et C sont codés par deux acides nucléiques isolés séparés ou plus dans la composition ; ou (c) y ne représente aucun lieur, et A-x-B et C sont codés par le même acide nucléique isolé dans la composition.

8. Composition comprenant un ou plusieurs acides nucléiques isolés qui sont des complémentaires pleine longueur des un ou plusieurs acides nucléiques isolés selon la revendication 6 ou la revendication 7.

9. Composition selon l'une quelconque des revendications 1 à 5, dans laquelle : (a) au moins une partie de la composition selon l'une quelconque des revendications précédentes est présente sur une surface du nanosupport synthétique ; ou (b) au moins une partie de la composition selon l'une quelconque des revendications précédentes est encapsulée par le nanosupport synthétique.

10. Composition comprenant A-x-B, dans laquelle x comprend un site de clivage de cathepsine comprenant KVSVR et dans laquelle A-x-B comprend une séquence d'acides aminés qui présente au moins 75 % d'identité avec l'une quelconque des séquences d'acides aminés représentées par SEQ ID NO : 12, 14, 17, 19, et 39 à 44 ; éventuellement dans laquelle :
(a) la séquence d'acides aminés présente au moins 85 % ou 95 % d'identité avec l'une quelconque des séquences d'acides aminés représentées par SEQ ID NO : 12, 14, 17, 19, et 39 à 44 ; ou
(b) la séquence d'acides aminés a l'une quelconque des séquences d'acides aminés représentées par SEQ ID NO : 12, 14, 17, 19, et 39 à 44.

11. Forme galénique comprenant : (a) la composition selon l'une quelconque des revendications précédentes ; ou (b) un vaccin comprenant la composition selon l'une quelconque des revendications précédentes, comprenant éventuellement en outre : (i) un adjuvant ou (ii) un support conjugué à ladite composition.

12. Composition ou forme galénique selon l'une quelconque des revendications précédentes pour une utilisation dans un procédé comprenant l'administration de la composition ou de la forme galénique à un sujet.

13. Composition selon l'une quelconque des revendications 1 à 10 et 12 ou forme galénique selon la revendication 11 ou 12, dans laquelle le peptide se liant au HLA-DP, DQ ou DR naturel comprend une séquence peptidique obtenue ou dérivée à partir de Clostridium tetani, du virus de l'hépatite B, d'un herpèsvirus humain, du virus de la grippe, du virus de la vaccine, du virus d'Epstein-Barr, du virus de la varicelle, du virus de la rougeole, du virus du sarcome de Rous, du cytomégalovirus, du virus de la varicelle zona, du virus des oreillons, de Corynebacterium diphtheriae, d'adénoviridés humains, du virus de la variole, ou d'un organisme infectieux capable d'infecter des êtres humains et de produire des cellules à mémoire CD4+ humaines spécifiques de l'organisme infectieux à la suite de l'initiation de l'infection.

14. Composition selon l'une quelconque des revendications 1 à 10 et 12 et 13 ou forme galénique selon l'une quelconque des revendications 11 à 13, dans laquelle A et B ou A et B et C comprennent chacun : (a) des séquences peptidiques obtenues ou dérivées à partir de différents organismes infectieux ; ou (b) des séquences peptidiques obtenues ou dérivées à partir d'organismes infectieux identiques ; ou (c) des peptides ayant des répertoires de liaison au CMH II différents.

15. Composition selon l'une quelconque des revendications 1 à 10 et 12 à 14 ou forme galénique selon l'une quelconque des revendications 11 à 14, dans laquelle : (a) A, x, ou B ; ou (b) A, x, B, y, ou C, comprennent des modifications de séquence ou chimiques qui augmentent la solubilité aqueuse de A-x-B ou A-x-B-y-C, respectivement, dans laquelle les modifications de séquence ou chimiques comprennent l'addition d'acides aminés N- et/ou C-terminaux hydrophiles, d'acides aminés N- et/ou C-terminaux hydrophobes, la substitution d'acides aminés pour obtenir un pI d'environ 7,4 et pour obtenir une charge nette positive à environ pH 3,0, et la substitution d'acides aminés susceptibles d'un réarrangement.

16. Composition selon l'une quelconque des revendications 1 à 10 et 12 à 15 ou forme galénique selon l'une quelconque des revendications 11 à 15, dans laquelle le premier et/ou deuxième peptide se liant au CMH II et/ou troisième peptide se liant au CMH II a une longueur située dans la plage de : (a) 5-mer à 50-mer ; (b) 5-mer à 30-mer ; (c) 6-mer à 25-mer.
